# EUROPÄISCHE PATENTSCHRIFT

(11) **EP 3 227 286 B1**
(45) Veröffentlichungstag und Bekanntmachung des Hinweises auf die Patenterteilung: **26.12.2018**
(21) Anmeldenummer: 15801454.8
(22) Anmeldetag: 30.11.2015
(51) Int. Cl.: C07D 471/04, C07D 487/04, A61K 31/437, A61P 9/00

(54) **SUBSTITUIERTE PYRAZOLO[1,5-A]PYRIDINE UND IMIDAZO[1,2-A]PYRAZINE UND IHRE VERWENDUNG**
SUBSTITUTED PYRAZOLO[1,5-A]PYRIDINES AND IMIDAZO[1,2-A]PYRAZINES AND THEIR USE
PYRAZOLO[1,5-A]PYRIDINES ET IMIDAZO[1,2-A]PYRAZINES SUBSTITUÉES ET LEUR UTILISATION

(30) Priorität: 02.12.2014 EP 14195899
(43) Veröffentlichungstag der Anmeldung: 11.10.2017
(73) Patentinhaber: Bayer Pharma Aktiengesellschaft, 13353 Berlin (DE)
(72) Erfinder: VAKALOPOULOS, Alexandros, 40721 Hilden (DE); BROCKSCHNIEDER, Damian, 42781 Haan (DE); WUNDER, Frank, 42117 Wuppertal (DE); STASCH, Johannes-Peter, 00046 Grottaferrata (RM) (IT); MARQUARDT, Tobias, 42115 Wuppertal (DE); DIETZ, Lisa, 42111 Wuppertal (DE); LI, Volkhart Min-Jian, 42553 Velbert (DE)
(74) Vertreter: BIP Patents
(86) Internationale Anmeldenummer: PCT/EP2015/078008
(87) Internationale Veröffentlichungsnummer: WO 2016/087342

(56) Entgegenhaltungen:
- US-A1- 2013 210 824
- US-A1- 2014 128 425
- STRAUB A ET AL: "NO-Independent stimulators of soluble guanylate cyclase", BIOORGANIC & MEDICINAL CHEMISTRY LETTERS, PERGAMON, AMSTERDAM, NL, Bd. 11, Nr. 6, 26. März 2001 (2001-03-26), Seiten 781-784, XP004230931, ISSN: 0960-894X, DOI: 10.1016/S0960-894X(01)00073-7

## Beschreibung

Die vorliegende Anmeldung betrifft neue substituierte Pyrazolo[1,5-a]pyridine und Imidazo[1,2-a]pyrazine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von kardiovaskulären Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorphosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223). US 2014/128425 beschreibt Imidazo[1,2-a]pyridincarboxamide als Stimulatoren der löslichen Guanylatcyclase.

Unter anderem in WO 2012/072512-A1 und WO 2010/117787 sind verschiedene Pyrazolo[1,5-a]pyridin-Derivate beschrieben, die zur Behandlung von Erkrankungen verwendet werden können. WO 89/03833-A1 und WO 96/34866-A1 beschreiben verschiedene Imidazo[1,2-a]pyrazin-Derivate, die zur Behandlung von Erkrankungen verwendet werden können.

Aufgabe der vorliegenden Erfindung war die Bereitstellung neuer Substanzen, die als Stimulatoren der löslichen Guanylatcyclase wirken, und als solche zur Behandlung und/oder Prophylaxe von Krankheiten geeignet sind.

Gegenstand der vorliegenden Erfindung sind Verbindungen der allgemeinen Formel (I-A) und (I-B), 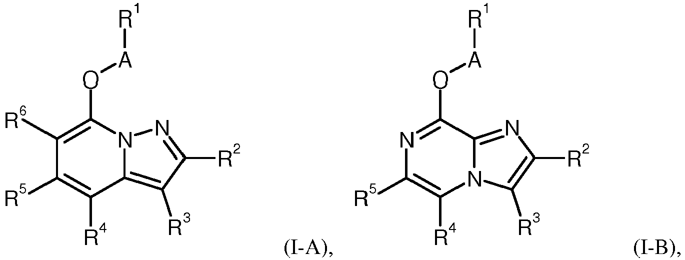 in welchen
- A: für CH₂, CD₂ oder CH(CH₃) steht,
- R¹: für (C₄-C₆)-Alkyl, (C₃-C₇)-Cycloalkyl, Pyridyl oder Phenyl steht, w
obei (C₄-C₆)-Alkyl bis zu sechsfach mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
wobei Pyridyl mit 1 oder 2 Substituenten unabhängig voneinander ausgewählt aus der Gruppe Halogen, Cyano und (C₁-C₄)-Alkyl substituiert ist,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl und (C₁-C₄)-Alkoxy substituiert sein kann,
- R²: für Wasserstoff, (C₁-C₄)-Alkyl, (C₁-C₄)-Alkoxymethyl, Cyclopropyl, Cyclobutyl, Monofluormethyl, Difluormethyl oder Trifluormethyl steht,
- R³: für Phenyl oder 5- bis 10-gliedriges Heteroaryl steht,
wobei Phenyl mit 1 bis 3 Substituenten unabhängig voneinander ausgewählt aus der Gruppe Halogen, Cyano, Trifluormethyl, Difluormethyl, (C₁-C₆)-Alkyl, (C₁-C₄)-Alkoxy-carbonyl, (C₁-C₄)-Alkylcarbonyl, Hydroxycarbonyl, -(C=O)NR⁷R⁸, (C₁-C₄)-Alkylsulfonyl, (C₃-C₆)-Cycloalkylsulfonyl, (C₁-C₄)-Alkylthio, (C₁-C₄)-Alkoxy, Trifluormethoxy, Difluormethoxy, Phenoxy, Hydroxy, Amino, Morpholinyl, Piperidinyl, Pyrrolidinyl, Piperazinyl und (C₃-C₆)-Cycloalkyl substituiert sein kann,
worin Amino mit 1 oder 2 Substituenten unabhängig voneinander ausgewählt aus (C₁-C₆)-Alkyl, (C₁-C₄)-Alkylcarbonyl, (C₃-C₆)-Cycloalkylsulfonyl, (C₁-C₄)-Alkylsulfonyl und Methoxy-(C₁-C₄)-alkyl substituiert sein kann,
   worin (C₁-C₆)-Alkyl mit Amin substituiert sein kann,
worin (C₁-C₆)-Alkyl mit 1 oder 2 Substituenten ausgewählt aus der Gruppe Fluor, Trifluormethoxy, -(C=O)NR⁷R⁸, (C₁-C₄)-Alkoxy, (C₃-C₆)-Cycloalkyl, Morpholinyl, Piperidinyl, Pyrrolidinyl, Piperazinyl, Hydroxy und Amino substituiert sein kann,
   worin Amino mit 1 oder 2 Substituenten unabhängig voneinander ausgewählt aus (C₁-C₆)-Alkyl, (C₁-C₄)-Alkylcarbonyl, (C₁-C₄)-Alkoxy-carbonyl, (C₃-C₆)-Cycloalkylsulfonyl, (C₁-C₄)-Alkylsulfonyl und Methoxy-(C₁-C₄)-alkyl substituiert sein kann,
und worin
   R⁷ und R⁸ jeweils unabhängig voneinander für Wasserstoff, (C₁-C₄)-Alkyl oder (C₃-C₇)-Cycloalkyl stehen,
wobei 5- bis 10-gliedriges Heteroaryl mit 1 bis 3 Substituenten unabhängig voneinander ausgewählt aus der Gruppe Fluor, Chlor, Cyano, (C₁-C₆)-Alkyl, (C₁-C₄)-Alkoxy, Amino, (C₁-C₄)-Alkoxycarbonyl, Hydroxycarbonyl, -(C=O)NR⁷R⁸, Phenyl, Pyridyl, Pyrimidyl, 1,3-Thiazol-5-yl, Pyrrolidinyl, Piperidinyl, Morpholinyl, Piperazinyl und (C₃-C₇)-Cycloalkyl substituiert sein kann,
worin (C₁-C₆)-Alkyl mit 1 bis 3 Substituenten ausgewählt aus der Gruppe Fluor, Cyano, Hydroxy, Amino, Trifluormethyl, Difluormethyl, (C₁-C₄)-Alkylsulfonyl, (C₁-C₄)-Alkylcarbonyl, (C₁-C₄)-Alkoxycarbonyl, Hydroxycarbonyl, -(C=O)NR⁷R⁸ (C₁-C₄)-Alkoxy, Phenoxy, Phenyl, Pyridyl, Pyrimidyl, 5-gliedriges Heteroaryl, (C₃-C₇)-Cycloalkyl, Morpholinyl, Piperidinyl, Pyrrolidinyl, Piperazinyl, 1,1-Dioxidothiomorpholin-4-yl und Azetidin substituiert sein kann,
   worin 5-gliedriges Heteroaryl mit 1 bis 3 Substituenten ausgewählt aus der Gruppe Halogen, (C₁-C₄)-Alkyl und (C₁-C₄)-Alkoxy substituiert sein kann,
   worin
   R⁷ und R⁸ jeweils unabhängig voneinander für Wasserstoff, (C₁-C₄)-Alkyl oder (C₃-C₇)-Cycloalkyl stehen, worin Piperidinyl mit 1 bis 4 Substituenten Fluor substituiert sein kann,
   worin Phenyl mit 1 bis 3 Substituenten ausgewählt aus der Gruppe Halogen, (C₁-C₄)-Alkyl und (C₁-C₄)-Alkoxy substituiert sein kann,
   worin Azetidin mit Hydroxy substituiert sein kann,
   worin Amino mit 1 oder 2 Substituenten (C₁-C₄)-Alkyl substituiert sein kann,
   und
   worin Piperazinyl mit 1 bis 3 Substituenten unabhängig voneinander ausgewählt aus der Gruppe (C₁-C₄)-Alkyl, (C₃-C₇)-Cycloalkyl und Trifluormethyl substituiert sein kann,
worin (C₃-C₇)-Cycloalkyl mit 1 oder 2 Substituenten unabhängig ausgewählt aus der Gruppe Halogen, (C₁-C₆)-Alkyl, (C₁-C₄)-Alkoxycarbonyl und Hydroxy-carbonyl substituiert sein kann,
worin (C₁-C₄)-Alkoxy mit Amino substituiert sein kann,
worin Amino mit 1 oder 2 Substituenten unabhängig voneinander ausgewählt aus (C₁-C₆)-Alkyl, (C₁-C₄)-Alkylcarbonyl, (C₃-C₆)-Cycloalkylsulfonyl und(C₁-C₄)-Alkylsulfonyl substituiert sein kann,
   worin (C₁-C₆)-Alkyl mit Amino substituiert sein kann,
   und
   worin (C₁-C₆)-Alkyl bis zu fünffach mit Fluor substituiert sein kann,
worin Phenyl, Pyridyl und Pyrimidyl mit 1 oder 2 Substituenten ausgewählt aus der Gruppe Methyl, Ethyl und Fluor substituiert sein können,
worin
   R⁷ und R⁸ jeweils unabhängig voneinander für Wasserstoff, (C₁-C₄)-Alkyl oder (C₃-C₇)-Cycloalkyl stehen,
oder
- R³: für eine Gruppe der Formel 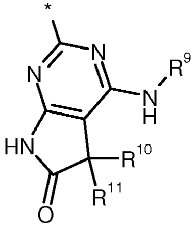 steht, wobei
* für die Anknüpfstelle an das Pyrazolopyridin bzw. das Imidazopyrazin steht,
R⁹ für Wasserstoff oder (C₁-C₆)-Alkyl steht,
wobei (C₁-C₆)-Alkyl mit Amino substituiert sein kann,
und
worin (C₁-C₆)-Alkyl bis zu fünffach mit Fluor substituiert sein kann,
R¹⁰ für Wasserstoff, Methyl oder Ethyl steht,
R¹¹ für Wasserstoff, Methyl, Ethyl, Trifluormethyl oder Cyclopropyl steht, oder
R¹⁰ und R¹¹ zusammen mit dem Kohlenstoffatom, an das sie gebunden sind, einen 3- bis 6-gliedrigen Carbocyclus bilden,
- R⁴: für Wasserstoff steht,
- R⁵: für Wasserstoff, Halogen, Cyano, Difluormethyl, Trifluormethyl, (C₁-C₄)-Alkyl, (C₃-C₇)-Cycloalkyl, (C₂-C₄)-Alkinyl, (C₁-C₄)-Alkylamino, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Erfindungsgemäße Verbindungen sind die Verbindungen der Formeln (I-A) und (I-B) und deren Salze, Solvate und Solvate der Salze, die von Formeln (I-A) und (I-B) umfassten Verbindungen der nachfolgend genannten Formeln und deren Salze, Solvate und Solvate der Salze sowie die von Formeln (I-A) und (I-B) umfassten, nachfolgend als Ausführungsbeispiele genannten Verbindungen und deren Salze, Solvate und Solvate der Salze, soweit es sich bei den von Formeln (I-A) und (I-B) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem werden auch Prodrugs der erfindungsgemäßen Verbindungen beschrieben. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl, 2-Ethylbutyl.
Cycloalkyl bzw. Carbocyclus bzw. Carbocyclyl steht in Rahmen der Erfindung für einen monocyclischen, bicyclischen oder tricyclischen gesättigten Alkylrest mit der jeweils angegebenen Anzahl an Ring-Kohlenstoffatomen. Beispielhaft und vorzugsweise seien genannt: Cyclopropyl, Cyclobutyl, Cyclopentyl, Cyclohexyl, Cycloheptyl und Adamantyl.
Alkenyl steht im Rahmen der Erfindung für einen linearen oder verzweigten Alkenylrest mit 2 bis 6 Kohlenstoffatomen und einer oder zwei Doppelbindungen. Bevorzugt ist ein linearer oder verzweigter Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.
Alkinyl steht im Rahmen der Erfindung für einen linearen oder verzweigten Alkinylrest mit 2 bis 6 Kohlenstoffatomen und einer Dreifachbindung. Beispielhaft und vorzugsweise seien genannt: Ethinyl, n-Prop-1-in-1-yl, n-Prop-2-in-1-yl, n-But-2-in-1-yl und n-But-3-in-1-yl.
Alkandiyl steht im Rahmen der Erfindung für einen linearen oder verzweigten divalenten Alkylrest mit 1 bis 4 Kohlenstoffatomen. Beispielhaft und vorzugsweise seien genannt: Methylen, 1,2-Ethylen, Ethan-1,1-diyl, 1,3-Propylen, Propan-1,1-diyl, Propan-1,2-diyl, Propan-2,2-diyl, 1,4-Butylen, Butan-1,2-diyl, Butan-1,3-diyl und Butan-2,3-diyl.
Alkoxy steht im Rahmen der Erfindung für einen linearen oder verzweigten Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy und tert.-Butoxy.
Alkoxycarbonyl stehen im Rahmen der Erfindung für einen linearen oder verzweigten Alkoxyrest mit 1 bis 4 Kohlenstoffatomen und einer am Sauerstoffatom angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Alkylsulfonyl steht in Rahmen der Erfindung für einen linearen oder verzweigten Alkylrest mit 1 bis 4 Kohlenstoffatomen, der über eine Sulfonylgruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.
Heterocyclus bzw Heterocyclyl steht im Rahmen der Erfindung für einen monocyclischen, gesättigten Heterocyclus mit insgesamt 4 bis 7 Ringatomen bzw. 5 bis 10 Ringatomen, der ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Beispielhaft seien genannt: Azetidinyl, Oxetanyl, Pyrrolidinyl, Pyrazolidinyl, Tetrahydrofuranyl, Thiolanyl, Piperidinyl, Piperazinyl, Tetrahydropyranyl, Tetrahydrothiopyranyl, Morpholinyl, Thiomorpholinyl, Hexahydroazepinyl und Hexahydro-1,4-diazepinyl. Bevorzugt sind Azetidinyl, Oxetanyl, Pyrrolidinyl, Tetrahydrofuranyl, Piperidinyl, Piperazinyl, Tetrahydropyranyl und Morpholinyl.
Heteroaryl steht im Rahmen der Erfindung für einen monocyclischen oder bicyclischen aromatischen Heterocyclus (Heteroaromaten) der bis zu vier gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, Chinolinyl, Thiazolyl, Oxazolyl, Isoxazolyl, Isothiazolyl, Triazolyl, Oxadiazolyl, Thiadiazolyl, Tetrazolyl, Pyridyl, Pyrimidinyl, Pyridazinyl, Pyrazinyl und Triazinyl.
Halogen schließt im Rahmen der Erfindung Fluor, Chlor, Brom und Iod ein. Bevorzugt sind Chlor oder Fluor.

In der Formel der Gruppe, für die R³ bzw. R¹ stehen kann, steht der Endpunkt der Linie, an dem das Zeichen * und # steht, nicht für ein Kohlenstoffatom beziehungsweise eine CH₂-Gruppe, sondern ist Bestandteil der Bindung zu dem jeweils bezeichneten Atom, an das R³ bzw. R¹ gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Bevorzugt sind im Rahmen der vorliegenden Erfindung Verbindungen der Formel (I-A) und (I-B), in welcher
- A: für CH₂ oder CD₂ steht,
- R¹: für Cyclohexyl, Pyridyl oder Phenyl steht,
wobei Cyclohexyl bis zu vierfach mit Fluor substituiert sein kann,
wobei Pyridyl mit 1 oder 2 Substituenten Fluor substituiert ist,
und
wobei Phenyl mit 1 bis 4 Substituenten unabhängig voneinander ausgewählt aus der Gruppe Fluor, Chlor, Cyano, Methyl und Methoxy substituiert sein kann,
- R²: für Wasserstoff, (C₁-C₄)-Alkyl, Cyclopropyl, Difluormethyl oder Trifluormethyl steht,
- R³: für Phenyl oder 5- oder 6-gliedriges Heteroaryl steht,
wobei Phenyl mit 1 bis 3 Substituenten unabhängig voneinander ausgewählt aus der Gruppe Fluor, Chlor, Cyano, Trifluormethyl, Difluormethyl, (C₁-C₆)-Alkyl, (C₁-C₄)-Alkoxycarbonyl, (C₁-C₄)-Alkylcarbonyl, Hydroxycarbonyl, -(C=O)NR⁷R⁸, (C₁-C₄)-Alkylsulfonyl, (C₃-C₆)-Cycloalkylsulfonyl, (C₁-C₄)-Alkoxy, Trifluormethoxy, Difluormethoxy, Hydroxy, Amino, Morpholinyl, Piperidinyl, Pyrrolidinyl, Piperazinyl und Cyclopropyl substituiert sein kann,
worin Amino mit 1 oder 2 Substituenten unabhängig voneinander ausgewählt aus (C₁-C₆)-Alkyl, (C₁-C₄)-Alkylcarbonyl und (C₁-C₄)-Alkylsulfonyl substituiert sein kann,
worin (C₁-C₆)-Alkyl mit 1 oder 2 Substituenten ausgewählt aus der Gruppe Fluor, Trifluormethoxy, -(C=O)NR⁷R⁸, (C₁-C₄)-Alkoxy, (C₃-C₆)-Cycloalkyl, Morpholinyl, Piperidinyl, Pyrrolidinyl, Piperazinyl, Hydroxy und Amino substituiert sein kann,
   worin Amino mit 1 oder 2 Substituenten unabhängig voneinander ausgewählt aus (C₁-C₆)-Alkyl, (C₁-C₄)-Alkylcarbonyl und (C₁-C₄)-Alkylsulfonyl substituiert sein kann,
und worin
   R⁷ und R⁸ jeweils unabhängig voneinander für Wasserstoff, (C₁-C₄)-Alkyl oder Cyclopropyl stehen,
wobei 5- oder 6-gliedriges Heteroaryl mit 1 bis 3 Substituenten unabhängig voneinander ausgewählt aus der Gruppe Fluor, Chlor, Cyano, (C₁-C₆)-Alkyl, (C₁-C₄)-Alkoxy, Amino, (C₁-C₄)-Alkoxycarbonyl, Hydroxycarbonyl, -(C=O)NR⁷R⁸, Phenyl, Pyridyl, Pyrimidyl, Pyrrolidinyl, Piperidinyl, Morpholinyl, Piperazinyl und Cyclopropyl substituiert sein kann,
worin (C₁-C₆)-Alkyl mit 1 bis 3 Substituenten ausgewählt aus der Gruppe Fluor, Cyano, Hydroxy, Amino, Trifluormethyl, Difluormethyl, (C₁-C₄)-Alkylsulfonyl, (C₁-C₄)-Alkoxycarbonyl, Hydroxycarbonyl, -(C=O)NR⁷R⁸, (C₁-C₄)-Alkoxy, Phenyl, Pyridyl, Pyrimidyl, 5-gliedriges Heteroaryl, (C₃-C₇)-Cycloalkyl, Morpholinyl, Piperidinyl, Pyrrolidinyl, Piperazinyl, 1,1-Dioxidothiomorpholin-4-yl und Azetidin substituiert sein kann,
worin 5-gliedriges Heteroaryl mit 1 bis 3 Substituenten ausgewählt aus der Gruppe Fluor, Chlor, Methyl, Ethyl und Methoxy substituiert sein kann,
worin
   R⁷ und R⁸ jeweils unabhängig voneinander für Wasserstoff, Methyl, Ethyl oder Cyclopropyl stehen,
worin Piperidinyl mit 1 bis 4 Substituenten Fluor substituiert sein kann,
worin Phenyl mit 1 bis 3 Substituenten ausgewählt aus der Gruppe Fluor, Methyl, Ethyl und Methoxy substituiert sein kann,
worin Amino mit 1 oder 2 Substituenten unabhängig voneinander ausgewählt aus (C₁-C₆)-Alkyl oder (C₁-C₄)-Alkylcarbonyl substituiert sein kann,
worin (C₁-C₆)-Alkyl mit Amino substituiert sein kann,
worin Phenyl, Pyridyl und Pyrimidyl mit 1 oder 2 Substituenten ausgewählt aus der Gruppe Methyl, Ethyl und Fluor substituiert sein können,
und worin
R⁷ und R⁸ jeweils unabhängig voneinander für Wasserstoff, Methyl, Ethyl oder Cyclopropyl stehen,
oder
- R³: für eine Gruppe der Formel 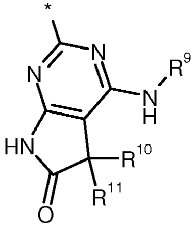 steht, wobei
* für die Anknüpfstelle an das Pyrazolopyridin bzw. das Imidazopyrazin steht,
R⁹ für Wasserstoff oder (C₁-C₆)-Alkyl steht,
wobei (C₁-C₆)-Alkyl mit Amino substituiert sein kann,
R¹⁰ für Methyl oder Ethyl steht,
R¹¹ für Methyl, Ethyl, Trifluormethyl oder Cyclopropyl steht,
- R⁴: für Wasserstoff steht,
- R⁵: für Wasserstoff, Fluor, Chlor, Cyano, Difluormethyl, Trifluormethyl, (C₁-C₄)-Alkyl, (C₃-C₅)-Cycloalkyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I-A), in welcher
- A: für CH₂ oder CD₂ steht,
- R¹: für Cyclohexyl, Pyridyl oder Phenyl steht,
wobei Cyclohexyl bis zu vierfach mit Fluor substituiert sein kann,
wobei Pyridyl mit 1 oder 2 Substituenten Fluor substituiert ist,
und
wobei Phenyl mit 1 bis 4 Substituenten unabhängig voneinander ausgewählt aus der Gruppe Fluor, Chlor, Cyano, Methyl und Methoxy substituiert sein kann,
- R²: für Wasserstoff, (C₁-C₄)-Alkyl, Cyclopropyl, Difluormethyl oder Trifluormethyl steht,
- R³: für Phenyl oder 5- oder 6-gliedriges Heteroaryl steht,
wobei Phenyl mit 1 bis 3 Substituenten unabhängig voneinander ausgewählt aus der Gruppe Fluor, Chlor, Cyano, Trifluormethyl, Difluormethyl, (C₁-C₆)-Alkyl, (C₁-C₄)-Alkoxycarbonyl, (C₁-C₄)-Alkylcarbonyl, Hydroxycarbonyl, -(C=O)NR⁷R⁸, (C₁-C₄)-Alkylsulfonyl, (C₃-C₆)-Cycloalkylsulfonyl, (C₁-C₄)-Alkoxy, Trifluormethoxy, Difluormethoxy, Hydroxy, Amino, Morpholinyl, Piperidinyl, Pyrrolidinyl, Piperazinyl und Cyclopropyl substituiert sein kann,
worin Amino mit 1 oder 2 Substituenten unabhängig voneinander ausgewählt aus (C₁-C₆)-Alkyl, (C₁-C₄)-Alkylcarbonyl und (C₁-C₄)-Alkylsulfonyl substituiert sein kann,
worin (C₁-C₆)-Alkyl mit 1 oder 2 Substituenten ausgewählt aus der Gruppe Fluor, Trifluormethoxy, -(C=O)NR⁷R⁸, (C₁-C₄)-Alkoxy, (C₃-C₆)-Cycloalkyl, Morpholinyl, Piperidinyl, Pyrrolidinyl, Piperazinyl, Hydroxy und Amino substituiert sein kann,
   worin Amino mit 1 oder 2 Substituenten unabhängig voneinander ausgewählt aus (C₁-C₆)-Alkyl, (C₁-C₄)-Alkylcarbonyl und (C₁-C₄)-Alkylsulfonyl substituiert sein kann,
und worin
   R⁷ und R⁸ jeweils unabhängig voneinander für Wasserstoff, (C₁-C₄)-Alkyl oder Cyclopropyl stehen,
wobei 5- oder 6-gliedriges Heteroaryl mit 1 bis 3 Substituenten unabhängig voneinander ausgewählt aus der Gruppe Fluor, Chlor, Cyano, (C₁-C₆)-Alkyl, (C₁-C₄)-Alkoxy, Amino, (C₁-C₄)-Alkoxycarbonyl, Hydroxycarbonyl, -(C=O)NR⁷R⁸, Phenyl, Pyridyl, Pyrimidyl, Pyrrolidinyl, Piperidinyl, Morpholinyl, Piperazinyl und Cyclopropyl substituiert sein kann,
worin (C₁-C₆)-Alkyl mit 1 bis 3 Substituenten ausgewählt aus der Gruppe Fluor, Cyano, Hydroxy, Amino, Trifluormethyl, Difluormethyl, (C₁-C₄)-Alkylsulfonyl, (C₁-C₄)-Alkoxycarbonyl, Hydroxycarbonyl, -(C=O)NR⁷R⁸, (C₁-C₄)-Alkoxy, Phenyl, Pyridyl, Pyrimidyl, 5-gliedriges Heteroaryl, (C₃-C₇)-Cycloalkyl, Morpholinyl, Piperidinyl, Pyrrolidinyl, Piperazinyl, 1,1-Dioxidothiomorpholin-4-yl und Azetidin substituiert sein kann,
   worin 5-gliedriges Heteroaryl mit 1 bis 3 Substituenten ausgewählt aus der Gruppe Fluor, Chlor, Methyl, Ethyl und Methoxy substituiert sein kann,
   worin
   R⁷ und R⁸ jeweils unabhängig voneinander für Wasserstoff, Methyl, Ethyl oder Cyclopropyl stehen, worin Piperidinyl mit 1 bis 4 Substituenten Fluor substituiert sein kann,
   worin Phenyl mit 1 bis 3 Substituenten ausgewählt aus der Gruppe Fluor, Methyl, Ethyl und Methoxy substituiert sein kann,
worin Amino mit 1 oder 2 Substituenten unabhängig voneinander ausgewählt aus (C₁-C₆)-Alkyl oder (C₁-C₄)-Alkylcarbonyl, substituiert sein kann,
   worin (C₁-C₆)-Alkyl mit Amino substituiert sein kann,
worin Phenyl, Pyridyl und Pyrimidyl mit 1 oder 2 Substituenten ausgewählt aus der Gruppe Methyl, Ethyl und Fluor substituiert sein können,
und worin
   R⁷ und R⁸ jeweils unabhängig voneinander für Wasserstoff, Methyl, Ethyl oder Cyclopropyl stehen,
- R⁴: für Wasserstoff steht,
- R⁵: für Wasserstoff, Fluor, Chlor, Cyano, Difluormethyl, Trifluormethyl, (C₁-C₄)-Alkyl, (C₃-C₅)-Cycloalkyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I-A), in welcher
- A: für CH₂ steht,
- R¹: für Phenyl steht,
wobei Phenyl bis zu dreifach mit Fluor substituiert ist,
- R²: für Methyl steht,
- R³: für Phenyl, Pyridyl, Pyrimidyl oder 4-Pyrazolyl steht,
wobei Phenyl mit 1 bis 3 Substituenten unabhängig voneinander ausgewählt aus der Gruppe Fluor, (C₁-C₆)-Alkyl, Hydroxycarbonyl, -(C=O)NR⁷R⁸, Methylsulfonyl, Ethylsulfonyl, Amino, Morpholinyl, Piperidinyl, Pyrrolidinyl und Piperazinyl substituiert sein kann,
worin Amino mit 1 oder 2 Substituenten unabhängig voneinander ausgewählt aus (C₁-C₆)-Alkyl, Methylcarbonyl, Ethylcarbonyl, Methylsulfonyl und Ethylsulfonyl substituiert sein kann,
worin (C₁-C₆)-Alkyl mit 1 oder 2 Substituenten ausgewählt aus der Gruppe Fluor, Morpholinyl, Piperidinyl, Pyrrolidinyl, Piperazinyl und Amino substituiert sein kann,
   worin Amino mit 1 oder 2 Substituenten unabhängig voneinander ausgewählt aus (C₁-C₄)-Alkyl, Methylcarbonyl, Ethylcarbonyl, Methylsulfonyl und Ethylsulfonyl substituiert sein kann,
und worin
   R⁷ und R⁸ jeweils unabhängig voneinander für Wasserstoff oder Cyclopropyl stehen,
wobei Pyridyl und 4-Pyrazolyl mit 1 oder 2 Substituenten unabhängig voneinander ausgewählt aus der Gruppe Fluor, Chlor, (C₁-C₆)-Alkyl, Methoxy, Amino, Hydroxy-carbonyl, -(C=O)NR⁷R⁸, Phenyl, Pyridyl, Pyrrolidinyl, Piperidinyl, Morpholinyl und Piperazinyl substituiert sein können,
worin (C₁-C₆)-Alkyl mit 1 bis 3 Substituenten ausgewählt aus der Gruppe Fluor, Hydroxy, Amino, Hydroxycarbonyl, -(C=O)NR⁷R⁸, Methoxy, Phenyl, Pyridyl, Pyrazolyl, (C₃-C₇)-Cycloalkyl, Morpholinyl, Piperidinyl, Pyrrolidinyl und Piperazinyl substituiert sein kann,
   worin Pyrazolyl mit 1 oder 2 Substituenten Methyl substituiert sein kann,
   und worin
   R⁷ und R⁸ jeweils unabhängig voneinander für Wasserstoff, Methyl, Ethyl oder Cyclopropyl stehen,
worin Amino mit 1 oder 2 Substituenten (C₁-C₆)-Alkyl substituiert sein kann,
   worin (C₁-C₆)-Alkyl mit Amino substituiert sein kann,
worin Phenyl, Pyridyl und Pyrimidyl mit 1 oder 2 Substituenten ausgewählt aus der Gruppe Methyl, Ethyl und Fluor substituiert sein können,
und worin
   R⁷ und R⁸ jeweils unabhängig voneinander für Wasserstoff, Methyl oder Cyclopropyl stehen,
- R⁴: für Wasserstoff steht,
- R⁵: für Wasserstoff oder Methyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I-A), in welcher
- A: für CH₂ steht,
- R¹: für eine Phenyl-Gruppe der Formel 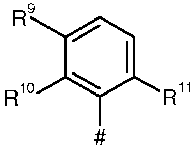 steht, wobei
# für die Anknüpfstelle an A steht, und
R⁹ für Wasserstoff oder Fluor steht,
R¹⁰ für Fluor steht,
R¹¹ für Fluor steht,
- R²: für Methyl steht,
- R³: für Phenyl, 3-Pyridyl, 4-Pyridyl oder 4-Pyrazolyl steht,
wobei Phenyl in 3-Position mit Fluor, (C₁-C₆)-Alkyl, Hydroxycarbonyl, -(C=O)NR⁷R⁸, Methylsulfonyl, Ethylsulfonyl, Amino, Morpholinyl, Piperidinyl, Pyrrolidinyl und Piperazinyl substituiert sein kann,
worin Amino mit 1 oder 2 Substituenten unabhängig voneinander ausgewählt aus (C₁-C₆)-Alkyl, Methylcarbonyl, Ethylcarbonyl, Methylsulfonyl und Ethylsulfonyl substituiert sein kann,
worin (C₁-C₆)-Alkyl mit 1 oder 2 Substituenten ausgewählt aus der Gruppe Fluor, Morpholinyl, Piperidinyl, Pyrrolidinyl, Piperazinyl und Amino substituiert sein kann,
   worin Amino mit 1 oder 2 Substituenten unabhängig voneinander ausgewählt aus (C₁-C₄)-Alkyl, Methylcarbonyl, Ethylcarbonyl, Methylsulfonyl und Ethylsulfonyl substituiert sein kann,
und worin
R⁷ und R⁸ jeweils unabhängig voneinander für Wasserstoff oder Cyclopropyl stehen,
wobei 3-Pyridyl und 4-Pyridyl jeweils mit 1 oder 2 Substituenten unabhängig voneinander ausgewählt aus der Gruppe Fluor, Chlor, (C₁-C₆)-Alkyl, Methoxy, Amino, Hydroxy-carbonyl, -(C=O)NR⁷R⁸, Phenyl, Pyridyl, Pyrrolidinyl, Piperidinyl, Morpholinyl und Piperazinyl substituiert sein können,
worin Amino mit 1 oder 2 Substituenten (C₁-C₆)-Alkyl substituiert sein kann,
   worin (C₁-C₆)-Alkyl mit Amino substituiert sein kann,
worin Phenyl, Pyridyl und Pyrimidyl mit 1 oder 2 Substituenten ausgewählt aus der Gruppe Methyl, Ethyl und Fluor substituiert sein können,
und worin
   R⁷ und R⁸ jeweils unabhängig voneinander für Wasserstoff, Methyl oder Cyclopropyl stehen,
wobei 4-Pyrazolyl an 1-Position mit (C₁-C₆)-Alkyl, Phenyl oder Pyridyl substituiert sein kann,
worin (C₁-C₆)-Alkyl mit 1 bis 3 Substituenten ausgewählt aus der Gruppe Fluor, Hydroxy, Amino, Methoxycarbonyl, Ethoxycarbonyl, Hydroxycarbonyl,-(C=O)NR⁷R⁸, Methoxy, Phenyl, Pyridyl, Pyrazolyl, (C₃-C₇)-Cycloalkyl, Morpholinyl, Piperidinyl, Pyrrolidinyl und Piperazinyl substituiert sein kann,
   worin Pyrazolyl mit 1 bis 3 Substituenten Methyl substituiert sein kann,
   worin
   R⁷ und R⁸ jeweils unabhängig voneinander für Wasserstoff, Methyl, Ethyl oder Cyclopropyl stehen,
worin Phenyl und Pyridyl mit 1 oder 2 Substituenten ausgewählt aus der Gruppe Methyl, Ethyl und Fluor substituiert sein können,
- R⁴: für Wasserstoff steht,
- R⁵: für Wasserstoff oder Methyl steht,
- R⁶: für Wasserstoff steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung Verbindungen der Formel (I-A), in welcher
- A: für CH₂ steht,
- R¹: für eine Phenyl-Gruppe der Formel 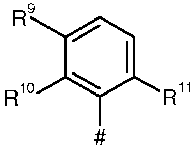 steht, wobei
# für die Anknüpfstelle an A steht, und
R⁹ für Wasserstoff oder Fluor steht,
R¹⁰ für Fluor steht,
R¹¹ für Fluor steht,
- R²: für Methyl steht,
- R³: für Phenyl, 3-Pyridyl, 4-Pyridyl oder 4-Pyrazolyl steht,
wobei Phenyl in 3-Position mit (C₁-C₆)-Alkyl, Hydroxycarbonyl, -(C=O)NR⁷R⁸, Methylsulfonyl, Ethylsulfonyl, Amino und Pyrrolidinyl substituiert sein kann,
worin Amino mit 1 oder 2 Substituenten unabhängig voneinander ausgewählt aus (C₁-C₆)-Alkyl, Methylcarbonyl und Methylsulfonyl substituiert sein kann,
worin (C₁-C₆)-Alkyl mit 1 oder 2 Substituenten ausgewählt aus der Gruppe Fluor, Pyrrolidinyl und Amino substituiert sein kann,
   worin Amino mit 1 oder 2 Substituenten unabhängig voneinander ausgewählt aus (C₁-C₄)-Alkyl, Methylcarbonyl und Methylsulfonyl substituiert sein kann,
und worin
   R⁷ und R⁸ jeweils unabhängig voneinander für Wasserstoff oder Cyclopropyl stehen,
wobei 3-Pyridyl und 4-Pyridyl jeweils mit 1 oder 2 Substituenten unabhängig voneinander ausgewählt aus der Gruppe Fluor, Chlor, (C₁-C₆)-Alkyl, Amino, Hydroxycarbonyl,-(C=O)NR⁷R⁸, Phenyl und Piperazinyl substituiert sein können,
worin Amino mit 1 oder 2 Substituenten (C₁-C₆)-Alkyl substituiert sein kann,
   worin (C₁-C₆)-Alkyl mit Amino substituiert sein kann,
worin Phenyl mit 1 oder 2 Substituenten ausgewählt aus der Gruppe Methyl, Ethyl und Fluor substituiert sein kann,
und worin
R⁷ und R⁸ jeweils für Wasserstoff steht,
wobei 4-Pyrazolyl an 1-Position mit (C₁-C₆)-Alkyl oder Phenyl substituiert sein kann,
worin (C₁-C₆)-Alkyl mit 1 bis 3 Substituenten ausgewählt aus der Gruppe Hydroxy, Amino, Methoxycarbonyl, Hydroxycarbonyl, -(C=O)NR⁷R⁸, Phenyl, Pyrazolyl, (C₃-C₇)-Cycloalkyl und Morpholinyl substituiert sein kann,
   worin Pyrazolyl mit 1 oder 3 Substituenten Methyl substituiert sein kann,
   worin
   R⁷ für Wasserstoff steht,
   R⁸ für Cyclopropyl steht,
worin Phenyl und Pyridyl mit 1 oder 2 Substituenten Fluor substituiert sein können,
- R⁴: für Wasserstoff steht,
- R⁵: für Wasserstoff oder Methyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I-B), in welcher
- A: für CH₂ oder CD₂ steht,
- R¹: für oder Phenyl steht,
wobei Phenyl mit 1 bis 3 Substituenten Fluor substituiert ist,
- R²: für Methyl steht,
- R³: für 5- oder 6-gliedriges Heteroaryl steht,
wobei 5- oder 6-gliedriges Heteroaryl mit 1 bis 3 Substituenten unabhängig voneinander ausgewählt aus der Gruppe (C₁-C₄)-Alkyl und Amino substituiert sein kann,
worin (C₁-C₄)-Alkyl mit 1 bis 3 Substituenten ausgewählt aus der Gruppe Hydroxy, Amino, (C₁-C₄)-Alkoxycarbonyl, Hydroxycarbonyl, Phenyl, 5-gliedriges Heteroaryl, (C₃-C₇)-Cycloalkyl, Morpholinyl und 1,1-Dioxidothiomorpholin-4-yl substituiert sein kann,
worin Amino mit (C₁-C₆)-Alkyl substituiert sein kann,
   worin (C₁-C₆)-Alkyl mit Amino substituiert sein kann,
oder
- R³: für eine Gruppe der Formel 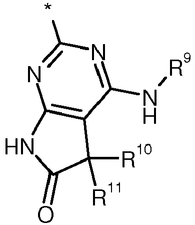 steht, wobei
* für die Anknüpfstelle an das Imidazopyrazin steht,
R⁹ für Wasserstoff oder (C₁-C₆)-Alkyl steht,
wobei (C₁-C₆)-Alkyl mit Amino substituiert sein kann,
R¹⁰ für Methyl oder Ethyl steht,
R¹¹ für Methyl, Ethyl oder Trifluormethyl steht,
- R⁴: für Wasserstoff steht,
- R⁵: für Wasserstoff oder Methyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I-B), in welcher
- A: für CH₂ steht,
- R¹: für eine Phenyl-Gruppe der Formel 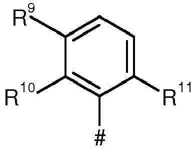 steht, wobei
# für die Anknüpfstelle an A steht, und
R⁹ für Wasserstoff steht,
R¹⁰ für Fluor steht,
R¹¹ für Fluor steht,
- R²: für Methyl steht,
- R³: für 4-Pyrazolyl oder 1,3,5-Triazinyl steht,
wobei 1,3,5-Triazinyl zweifach mit Amino substituiert ist,
oder
- R³: für eine Gruppe der Formel 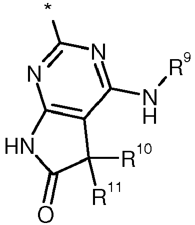 steht, wobei
* für die Anknüpfstelle an das Imidazopyrazin steht,
R⁹ für Wasserstoff steht,
R¹⁰ für Methyl steht,
R¹¹ für Methyl steht,
- R⁴: für Wasserstoff steht,
- R⁵: für Methyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I-A), 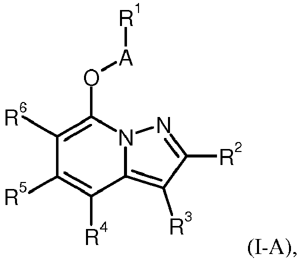 sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I-B), 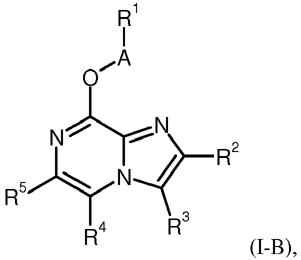 sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I-A), in
welcher
- R¹: für eine Phenyl-Gruppe der Formel 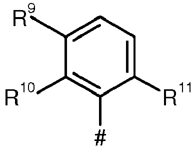 steht, wobei
# für die Anknüpfstelle an A steht, und
R⁹ für Wasserstoff oder Fluor steht,
R¹⁰ für Fluor steht,
R¹¹ für Fluor steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I-A), in welcher
- R²: für Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I-A), in welcher
- R³: für Phenyl, 3-Pyridyl, 4-Pyridyl oder 4-Pyrazolyl steht,
wobei Phenyl in 3-Position mit (C₁-C₆)-Alkyl, Hydroxycarbonyl, -(C=O)NR⁷R⁸, Methylsulfonyl, Ethylsulfonyl, Amino und Pyrrolidinyl substituiert sein kann,
worin Amino mit 1 oder 2 Substituenten unabhängig voneinander ausgewählt aus (C₁-C₆)-Alkyl, Methylcarbonyl und Methylsulfonyl substituiert sein kann,
worin (C₁-C₆)-Alkyl mit 1 oder 2 Substituenten ausgewählt aus der Gruppe Fluor, Pyrrolidinyl und Amino substituiert sein kann,
   worin Amino mit 1 oder 2 Substituenten unabhängig voneinander ausgewählt aus (C₁-C₄)-Alkyl, Methylcarbonyl und Methylsulfonyl substituiert sein kann,
und worin
   R⁷ und R⁸ jeweils unabhängig voneinander für Wasserstoff oder Cyclopropyl stehen,
wobei 3-Pyridyl und 4-Pyridyl jeweils mit 1 oder 2 Substituenten unabhängig voneinander ausgewählt aus der Gruppe Fluor, Chlor, (C₁-C₆)-Alkyl, Amino, Hydroxycarbonyl,-(C=O)NR⁷R⁸ und Piperazinyl substituiert sein können,
worin Amino mit 1 oder 2 Substituenten (C₁-C₆)-Alkyl substituiert sein kann,
   worin (C₁-C₆)-Alkyl mit Amino substituiert sein kann,
und worin
   R⁷ und R⁸ jeweils für Wasserstoff steht,
wobei 4-Pyrazolyl an 1-Position mit (C₁-C₆)-Alkyl oder Phenyl substituiert sein kann,
worin (C₁-C₆)-Alkyl mit 1 bis 3 Substituenten ausgewählt aus der Gruppe Hydroxy, Amino, Methoxycarbonyl, Hydroxycarbonyl, -(C=O)NR⁷R⁸, Phenyl, Pyrazolyl, (C₃-C₇)-Cycloalkyl und Morpholinyl substituiert sein kann,
   worin Pyrazolyl mit 1 oder 3 Substituenten Methyl substituiert sein kann,
   worin
   R⁷ für Wasserstoff steht,
   R⁸ für Cyclopropyl steht,
worin Phenyl und Pyridyl mit 1 oder 2 Substituenten Fluor substituiert sein können,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I-A), in
welcher
- R³: für Phenyl steht,
wobei Phenyl in 3-Position mit (C₁-C₆)-Alkyl, Hydroxycarbonyl, -(C=O)NR⁷R⁸, Methylsulfonyl, Ethylsulfonyl, Amino und Pyrrolidinyl substituiert sein kann,
worin Amino mit 1 oder 2 Substituenten unabhängig voneinander ausgewählt aus (C₁-C₆)-Alkyl, Methylcarbonyl und Methylsulfonyl substituiert sein kann,
worin (C₁-C₆)-Alkyl mit 1 oder 2 Substituenten ausgewählt aus der Gruppe Fluor, Pyrrolidinyl und Amino substituiert sein kann,
   worin Amino mit 1 oder 2 Substituenten unabhängig voneinander ausgewählt aus (C₁-C₄)-Alkyl, Methylcarbonyl und Methylsulfonyl substituiert sein kann,
und worin
   R⁷ und R⁸ jeweils unabhängig voneinander für Wasserstoff oder Cyclopropyl stehen,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I-A), in
welcher
- R³: für 3-Pyridyl oder 4-Pyridyl steht,
wobei 3-Pyridyl und 4-Pyridyl jeweils mit 1 oder 2 Substituenten unabhängig voneinander ausgewählt aus der Gruppe Fluor, Chlor, (C₁-C₆)-Alkyl, Amino, Hydroxycarbonyl,-(C=O)NR⁷R⁸ und Piperazinyl substituiert sein können,
worin Amino mit 1 oder 2 Substituenten (C₁-C₆)-Alkyl substituiert sein kann,
   worin (C₁-C₆)-Alkyl mit Amino substituiert sein kann,
und worin
   R⁷ und R⁸ jeweils für Wasserstoff steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung auch Verbindungen der Formel (I-A), in
welcher
- R³: für 3-Pyridyl steht,
wobei 3-Pyridyl mit 1 oder 2 Substituenten unabhängig voneinander ausgewählt aus der Gruppe Chlor, Methyl, Hydroxycarbonyl und -(C=O)NR⁷R⁸ substituiert sein kann,
worin
R⁷ und R⁸ jeweils für Wasserstoff steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung auch Verbindungen der Formel (I-A), in
welcher
- R³: für 4-Pyridyl steht,
wobei 4-Pyridyl mit 1 oder 2 Substituenten unabhängig voneinander ausgewählt aus der Gruppe Fluor, Amino und Piperazinyl substituiert sein können,
worin Amino mit 1 oder 2 Substituenten (C₁-C₆)-Alkyl substituiert sein kann,
worin (C₁-C₆)-Alkyl mit Amino substituiert sein kann,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I-A), in
welcher
- R³: für 4-Pyrazolyl steht,
wobei 4-Pyrazolyl an 1-Position mit (C₁-C₆)-Alkyl oder Phenyl substituiert sein kann,
worin (C₁-C₆)-Alkyl mit 1 bis 3 Substituenten ausgewählt aus der Gruppe Hydroxy, Amino, Methoxycarbonyl, Hydroxycarbonyl, -(C=O)NR⁷R⁸, Phenyl, Pyrazolyl, (C₃-C₇)-Cycloalkyl und Morpholinyl substituiert sein kann,
   worin Pyrazolyl mit 1 bis 3 Substituenten Methyl substituiert sein kann,
   worin
   R⁷ für Wasserstoff steht,
   R⁸ für Cyclopropyl steht,
worin Phenyl und Pyridyl mit 1 oder 2 Substituenten Fluor substituiert sein können,
sowie ihre *N*-Oxide, Salze, Solvate, Salze der *N*-Oxide und Solvate der *N*-Oxide und Salze.

Besonders bevorzugt sind im Rahmen der vorliegenden Erfindung auch Verbindungen der Formel (I-A), in
welcher
- R³: für 4-Pyrazolyl steht,
wobei 4-Pyrazolyl an 1-Position mit (C₁-C₆)-Alkyl oder Phenyl substituiert sein kann,
worin (C₁-C₆)-Alkyl mit Hydroxy, Amino, Methoxycarbonyl, Hydroxycarbonyl,-(C=O)NR⁷R⁸, Phenyl, Pyrazolyl, (C₃-C₇)-Cycloalkyl oder Morpholinyl substituiert sein kann,
   worin Pyrazolyl mit 1 oder 2 Substituenten Methyl substituiert sein kann,
   worin
   R⁷ für Wasserstoff steht,
   R⁸ für Cyclopropyl steht,
worin Phenyl mit Fluor substituiert sein kann,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I-A), in welcher
- R⁵: für Wasserstoff oder Methyl steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung auch Verbindungen der Formel (I-A), in
welcher
- R⁵: für Wasserstoff steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung auch Verbindungen der Formel (I-A), in
welcher
- R⁵: für Methyl steht,
sowie ihre *N*-Oxide, Salze, Solvate, Salze der *N*-Oxide und Solvate der *N*-Oxide und Salze.

Die in den jeweiligen Kombinationen bzw. bevorzugten Kombinationen von Resten im Einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I-A) dadurch gekennzeichnet, dass man
eine Verbindung der Formel (II) 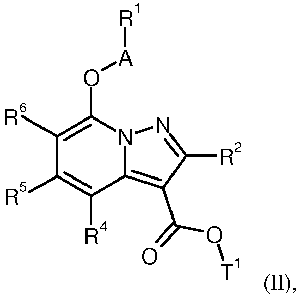 in welcher A, R¹, R², R⁴, R⁵ und R⁶ jeweils die oben angegebenen Bedeutungen haben und
- T¹: für (C₁-C₄)-Alkyl oder Benzyl steht,
in einem inerten Lösungsmittel in Gegenwart einer geeigneten Base oder Säure zu einer Carbonsäure der Formel (III) 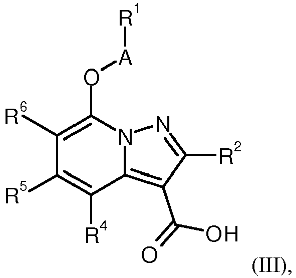 in welcher A, R¹, R², R⁴, R⁵ und R⁶ jeweils die oben angegebenen Bedeutungen haben,
umsetzt und diese dann mit einem Halogen-Äquivalent in Anwesenheit einer geeigneten Base in eine Verbindung der Formel (IV) 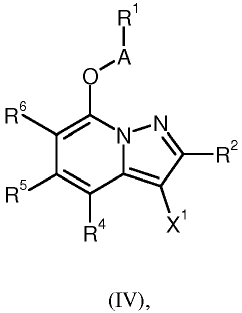 in welcher A, R¹, R², R⁴, R⁵ und R⁶ jeweils die oben angegebenen Bedeutungen haben und
- X¹: für Chlor, Brom oder Iod steht,
überführt, und diese im Anschluß in einem inerten Lösungsmittel in Gegenwart eines geeigneten Übergangsmetallkatalysators mit einer Verbindung der Formel (V), 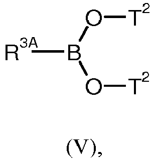 in welcher
- R^{3A}: die oben für R³ angegebenen Bedeutungen hat und
- T²: für Wasserstoff oder (C₁-C₄)-Alkyl steht, oder beide Reste T² zusammen eine -C(CH₃)₂-C(CH₃)₂-Brücke bilden,
zu einer Verbindung der Formel (I-A1) 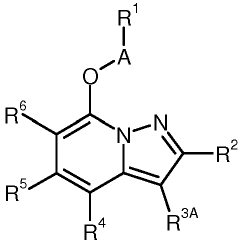 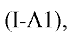 umsetzt, in welcher A, R¹, R², R⁴, R⁵ und R⁶ jeweils die oben angegebenen Bedeutungen haben,
und anschließend für den Fall, dass R^{3A} für 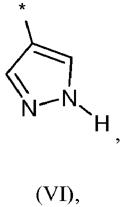 steht, diese Verbindungen in einem inerten Lösungsmittel in Gegenwart einer geeigneten Base mit einer Verbindung der Formel (VII) 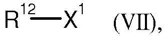 in welcher
- X¹: für eine geeignete Abgangsgruppe, insbesondere Chlor, Brom, Iod, Mesylat, Triflat oder Tosylat steht,
und
- R¹²: für (C₁-C₆)-Alkyl steht,
worin (C₁-C₆)-Alkyl mit 1 bis 3 Substituenten ausgewählt aus der Gruppe Fluor, Cyano, Hydroxy, Amino, Trifluormethyl, Difluormethyl, (C₁-C₄)-Alkylsulfonyl, (C₁-C₄)-Alkylcarbonyl, (C₁-C₄)-Alkoxycarbonyl, Hydroxycarbonyl, -(C=O)NR⁷R⁸ (C₁-C₄)-Alkoxy, Phenoxy, Phenyl, Pyridyl, Pyrimidyl, 5-gliedriges Heteroaryl, (C₃-C₇)-Cycloalkyl, Morpholinyl, Piperidinyl, Pyrrolidinyl, Piperazinyl, 1,1-Dioxidothiomorpholin-4-yl und Azetidin substituiert sein kann,
worin 5-gliedriges Heteroaryl mit 1 bis 3 Substituenten ausgewählt aus der Gruppe Halogen, (C₁-C₄)-Alkyl und (C₁-C₄)-Alkoxy substituiert sein kann,
worin
   R⁷ und R⁸ jeweils unabhängig voneinander für Wasserstoff, (C₁-C₄)-Alkyl oder (C₃-C₇)-Cycloalkyl stehen,
worin Piperidinyl mit 1 bis 4 Substituenten Fluor substituiert sein kann,
worin Phenyl mit 1 bis 3 Substituenten ausgewählt aus der Gruppe Halogen, (C₁-C₄)-Alkyl und (C₁-C₄)-Alkoxy substituiert sein kann,
worin Azetidin mit Hydroxy substituiert sein kann,
worin Amino mit loder 2 Substituenten (C₁-C₄)-Alkyl substituiert sein kann,
   und
worin Piperazinyl mit 1 bis 3 Substituenten unabhängig voneinander ausgewählt aus der Gruppe (C₁-C₄)-Alkyl, (C₃-C₇)-Cycloalkyl und Trifluormethyl substituiert sein kann,
zu einer Verbindung der Formel (I-A2) 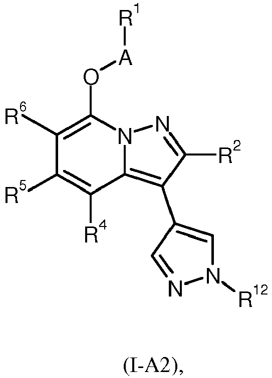 umsetzt,
in welcher A, R¹, R², R⁴, R⁵, R⁶ und R¹² jeweils die oben angegebenen Bedeutungen haben und
anschliessend gegebenenfalls vorhandene Schutzgruppen abspaltet, und die resultierenden Verbindungen der Formel (I-A) gegebenenfalls mit den entsprechenden (i) Lösungsmitteln und/oder (ii) Säuren oder Basen in ihre Solvate, Salze und/oder Solvate der Salze überführt,
Das beschriebene Herstellverfahren kann durch das folgende Syntheseschema (Schema 1) beispielhaft verdeutlicht werden: 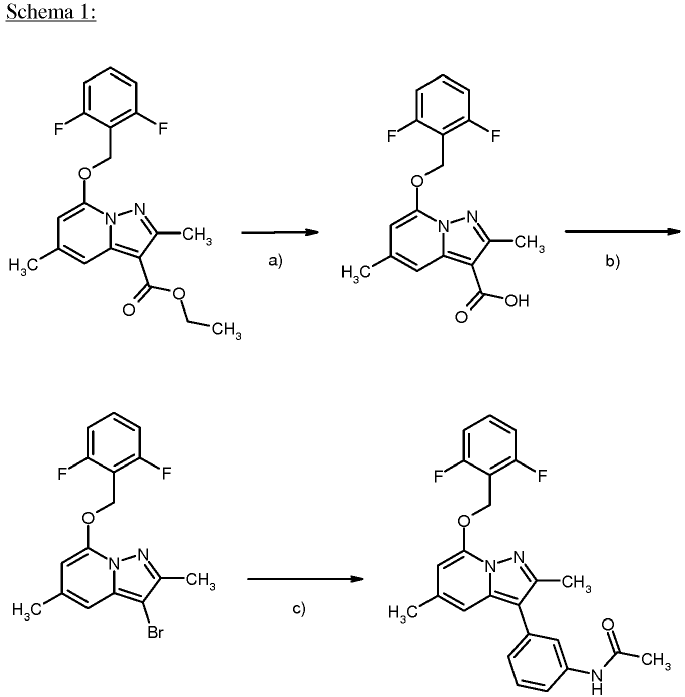 [a): Natriumhydroxid, 1,4-Dioxan, 90°C; b): *N*-Bromsuccinimid, DMF, Natriumhydrogencarbonat, Raumtemperatur; c): (3-Acetamidophenyl)borsäure, Chloro(2-dicyclohexylphosphino-2',4',6'-triisopropyl-1,1'-biphenyl)[2-(2'-amino-1,1'-biphenyl)palladium (II) [CAS: 1310584-14-5], K₃PO₄, Acetonitril, 60°C oder 100°C].

Weiterer Gegenstand der Erfindung ist ein Verfahren zur Herstellung der erfindungsgemäßen Verbindungen der Formel (I-B) dadurch gekennzeichnet, dass man
eine Verbindung der Formel (VIII) 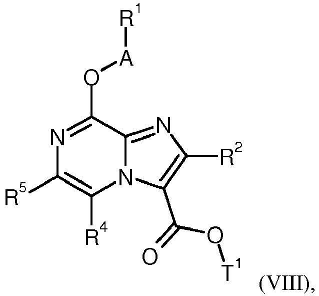 in welcher A, R¹, R², R⁴ und R⁵ jeweils die oben angegebenen Bedeutungen haben und
- T¹: für (C₁-C₄)-Alkyl oder Benzyl steht,
in einem inerten Lösungsmittel in Gegenwart einer geeigneten Base oder Säure zu einer Carbonsäure der Formel (IX) 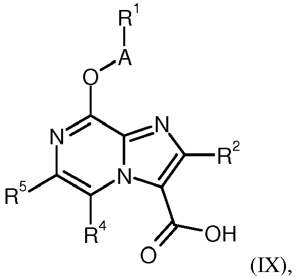 in welcher A, R¹, R², R⁴ und R⁵ jeweils die oben angegebenen Bedeutungen haben,
umsetzt und diese dann mit einem Halogen-Äquivalent in Anwesenheit einer Base in eine Verbindung der Formel (X) 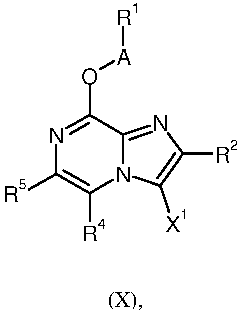 in welcher A, R¹, R², R⁴ und R⁵ jeweils die oben angegebenen Bedeutungen haben und
- X¹: für Chlor, Brom oder Iod steht,
überführt, und diese im Anschluß in einem inerten Lösungsmittel in Gegenwart eines geeigneten Übergangsmetallkatalysators mit einer Verbindung der Formel (V), 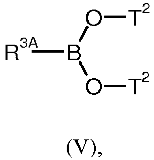 in welcher
- R^{3A}: die oben für R³ angegebenen Bedeutungen hat und
- T²: für Wasserstoff oder (C₁-C₄)-Alkyl steht, oder beide Reste T² zusammen eine -C(CH₃)₂-C(CH₃)₂-Brücke bilden,
umsetzt, gegebenenfalls vorhandene Schutzgruppen abspaltet, und die resultierenden Verbindungen der Formel (I-B) gegebenenfalls mit den entsprechenden (i) Lösungsmitteln und/oder (ii) Säuren oder Basen in ihre Solvate, Salze und/oder Solvate der Salze überführt.

Die Verbindungen der Formeln (V) und (VII) sind kommerziell erhältlich, literaturbekannt oder können in Analogie zu literaturbekannten Verfahren hergestellt werden.

Die Hydrolyse der Ester-Gruppe T¹ der Verbindungen der Formel (II) bzw. (VIII) erfolgt nach üblichen Methoden, indem man die Ester in inerten Lösungsmitteln mit Säuren oder Basen behandelt, wobei bei letzterem die zunächst entstehenden Salze durch Behandeln mit Säure in die freien Carbonsäuren überführt werden. Im Falle der tert.-Butylester erfolgt die Esterspaltung bevorzugt mit Säuren. Im Falle der Benzylester erfolgt die Esterspaltung bevorzugt hydrogenolytisch mit Palladium auf Aktivkohle oder Raney-Nickel. Als inerte Lösungsmittel eignen sich für diese Reaktion Wasser oder die für eine Esterspaltung üblichen organischen Lösungsmittel. Hierzu gehören bevorzugt Alkohole wie Methanol, Ethanol, n-Propanol, Isopropanol, n-Butanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol eingesetzt.

Als Basen für die Ester-Hydrolyse sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium- oder Lithiumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der tert.-Butylester und Salzsäure im Falle der Methylester.

Die Esterspaltung erfolgt im Allgemeinen in einem Temperaturbereich von 0°C bis +100°C, bevorzugt bei +0°C bis +50°C.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Als Lösungsmittel eignen sich für den Verfahrensschritt (III) → (IV) bzw. (IX) → (X) Alkohole wie Methanol, Ethanol, n-Propanol, Isopropanol, n-Butanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Acetonitril, N-Methylpyrrolidin (NMP), Dichlormethan, Dimethylformamid oder Dimethylsulfoxid. Ebenso ist es möglich, Gemische der genannten Lösungsmittel einzusetzen. Bevorzugt werden Dimethylformamid oder Methylpyrrolidin eingesetzt.

Die Umsetzung (III) → (IV) bzw. (IX) → (X) erfolgt gegenbenenfalls in Gegenwart einer geeigneten Base. Geeignete Basen für diese Umsetzung sind die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Natriumhydrogencarbonat, Kaliumhydrogencarbonat, Alkali-Alkoholate wie Natrium- oder Kaliummethanolat oder Natrium- oder Kalium-tert.-butylat, Amide wie Natriumamid, Lithium-, Natrium- oder Kalium-bis(trimethylsilyl)amid oder Lithiumdiisopropylamid, oder organische Amine wie Triethylamin, *N*-Methylmorpholin, *N*-Methylpiperidin, *N*,*N*-Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO®) oder Kaliumphosphat. Bevorzugt wird Natriumhydrogencarbonat oder Kaliumhydrogencarbonat verwendet.

Als Halogen-Quelle bei der Umsetzung (III) → (IV) bzw. (IX) → (X) eignen sich beispielsweise *N*-Bromsuccinimid, *N*-Chlorsuccinimid, *N*-Iod-succinimid, Chlor, Brom oder Iod. Bevorzugt wird *N*-Bromsuccinimid verwendet.

Die Reaktion (III) → (IV) bzw. (IX) → (X) erfolgt im Allgemeinen in einem Temperaturbereich von -20°C bis +100°C, bevorzugt im Bereich von +0°C bis +50°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Der Verfahrensschritt (IV) + (V) → (I-A1) bzw. (X) + (V) → (1-B) erfolgt in einem unter den Reaktionsbedingungen inerten Lösungmittel. Geeignete Lösungsmittel sind beispielsweise Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, oder andere Lösungsmitteln wie 1,2-Dimethoxyethan (DME), Dimethylformamid (DMF), Dimethylsulfoxid (DMSO), *N*,*N'*-Dimethylpropylenharnstoff (DMPU), *N*-Methylpyrrolidon (NMP), Pyridin, Acetonitril, Toluol oder auch Wasser. Ebenso ist es möglich, Gemische der genannten Lösungsmittel einzusetzen. Bevorzugt sind Acetonitril und Wasser.

Gegebenenfalls kann die Umsetzung (IV) + (V) → (I-A1) bzw. (X) + (V) → (1-B) in Gegenwart eines geeigneten Palladium- und/oder Kupferkatalysators erfolgen. Als Palladium-Katalysator ist beispielsweise Palladium(II)-acetat, Tetrakis-(triphenylphosphin)-palladium(0), Bis(tri-tert.-butyl-phosphin)palladium(0), Bis-(triphenylphosphin)-palladium(II)-chlorid, Bis-(acetonitril)-palladium(II)-chlorid, [1,1'-Bis(diphenylphosphino)ferrocen]dichlorpalladium(II) und entsprechender Dichlormethan-Komplex, gegebenenfalls in Verbindung mit zusätzlichen Phosphanliganden wie beispielsweise (2-Biphenyl)di-*tert*.-butylphosphin, 2-Dicyclohexylphosphino-2',6'-dimethoxybiphenyl (SPHOS) Dicyclohexyl[2',4',6'-tris-(1-methylethyl)biphenyl-2-yl]phosphan (XPHOS), Bis(2-phenyl-phosphinophenyl)ether (DPEphos), 4,5-Bis(diphenylphosphino)-9,9-dimethylxanthen (Xantphos) [vgl. z.B. Hassan J. et al., Chem. Rev. 102, 1359-1469 (2002)] oder Chloro(2-dicyclohexylphosphino-2',4',6'-triisopropyl-1,1'-biphenyl)[2-(2'-amino-1,1'-biphenyl)palladium (II) [CAS: 1310584-14-5] geeignet.

Die Umsetzung (IV) + (V) → (I-A1) bzw. (X) + (V) → (1-B) erfolgt gegenbenenfalls in Gegenwart einer geeigneten Base. Geeignete Basen für diese Umsetzung sind die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium-, Natrium- oder Kalium-bis(trimethylsilyl)amid oder Lithiumdiisopropylamid, oder organische Amine wie Triethylamin, *N*-Methylmorpholin, *N*-Methylpiperidin, *N*,*N*-Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO®) oder Kaliumphosphat. Bevorzugt wird Kaliumphosphat verwendet.

Die Reaktion (IV) + (V) → (I-A1) bzw. (X) + (V) → (1-B) wird im Allgemeinen in einem Temperaturbereich von 0°C bis +200°C, bevorzugt bei +60°C bis +120°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (I-A1) + (VII) → (I-A2) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Aceton, Methylethylketon, Essigsäureethylester, Acetonitril, *N,N*-Dimethylformamid, *N,N-*Dimethylacetamid, Dimethylsulfoxid, *N*,*N'*-Dimethylpropylenharnstoff (DMPU), *N*-Methylpyrrolidon (NMP) oder Pyridin. Ebenso ist es möglich, Gemische der genannten Lösungsmittel einzusetzen. Bevorzugt wird Dimethylformamid oder Dimethylsulfoxid verwendet.

Als Basen für den Verfahrensschritt (I-A1) + (VII) → (I-A2) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat gegebenenfalls unter Zusatz eines Alkaliiodids wie beispielsweise Natriumiodid oder Kaliumiodid, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)amid oder Lithiumdiisopropylamid, oder organische Amine wie Triethylamin, *N*-Methylmorpholin, *N*-Methylpiperidin, *N*,*N*-Diisopropylethylamin, Pyridin, 4-(*N,N-*Dimethylamino)-pyridin (DMAP), 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO®). Bevorzugt wird Kaliumcarbonat, Cäsiumcarbonat oder Natriummethanolat verwendet.

Die Reaktion erfolgt im Allgemeinen in einem Temperaturbereich von 0°C bis +120°C, bevorzugt bei +20°C bis +80°C, gegebenenfalls in einer Mikrowelle. Die Umsetzung kann bei normalem, erhöhtem oder bei erniedrigtem Druck durchgeführt werden (z.B. von 0.5 bis 5 bar).

Als Amino- Schutzgruppe wird bevorzugt tert.-Butoxycarbonyl (Boc) oder Benzyloxycarbonyl (Z) verwendet. Als Schutzgruppe für eine Hydroxy- oder Carboxyl-Funktion wird vorzugsweise *tert.-*Butyl oder Benzyl eingesetzt. Die Abspaltung dieser Schutzgruppen wird nach üblichen Methoden, vorzugsweise durch Reaktion mit einer starken Säure wie Chlorwasserstoff, Bromwasserstoff oder Trifluoressigsäure in einem inerten Lösungsmittel wie Dioxan, Diethylether, Dichlormethan oder Essigsäure durchgeführt; gegebenenfalls kann die Abspaltung auch ohne ein zusätzliches inertes Lösungsmittel erfolgen. Im Falle von Benzyl und Benzyloxycarbonyl als Schutzgruppe können diese auch durch Hydrogenolyse in Gegenwart eines Palladium-Katalysators entfernt werden. Die Abspaltung der genannten Schutzgruppen kann gegebenenfalls simultan in einer Eintopf-Reaktion oder in separaten Reaktionschritten vorgenommen werden.

Die Verbindungen der Formel (II) sind literaturbekannt oder können hergestellt werden, indem
[A] eine Verbindung der Formel (XI) 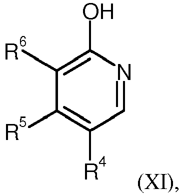 in welcher R⁴, R⁵ und R⁶ die oben angegebene Bedeutung haben,
   in einem inerten Lösungsmittel in Gegenwart einer geeigneten Base mit einer Verbindung der Formel (XII) 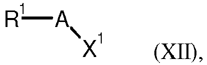 in welcher A und R¹ die oben angegebene Bedeutung haben und
   - X¹: für eine geeignete Abgangsgruppe, insbesondere Chlor, Brom, Iod, Mesylat, Triflat oder Tosylat, steht,
   zu einer Verbindung der Formel (XIII) 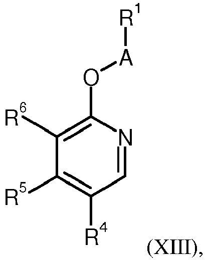 in welcher A, R¹, R⁴, R⁵ und R⁶ jeweils die oben angegebenen Bedeutungen haben,
   umgesetzt wird,
   diese dann mit *O*-(2-Mesitylenesulfonyl)hydroxylamin (MSH) in eine Verbindung (XIV) 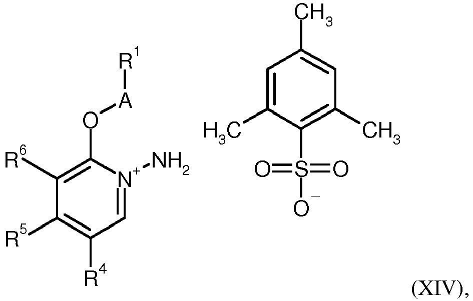 überführt wird,
   und diese anschliessend in einem inerten Lösungsmittel mit einer Verbindung der Formel (XV) 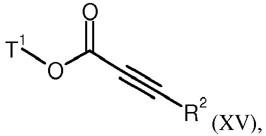 in welcher R² und T¹ jeweils die oben angegebenen Bedeutungen haben,
   umgesetzt wird,
   oder
[B] eine Verbindung der Formel (XVI) 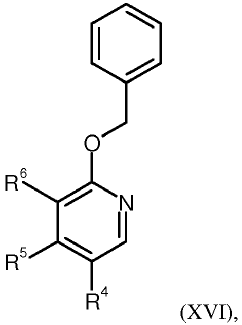 in welcher R⁴, R⁵ und R⁶ die oben angegebene Bedeutung haben,
   mit *O*-(2-Mesitylenesulfonyl)hydroxylamin (MSH) in eine Verbindung (XVII) 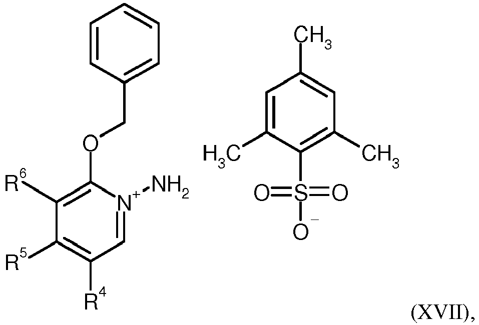 überführt wird,
   diese anschliessend in einem inerten Lösungsmittel mit einer Verbindung der Formel (IX) 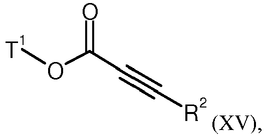 in welcher R² und T¹ jeweils die oben angegebenen Bedeutungen haben, zu einer Verbindung (XVIII)
   umgesetzt wird, 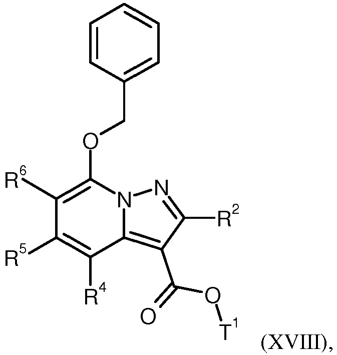 in welcher R², R⁴, R⁵ und R⁶ jeweils die oben angegebenen Bedeutungen haben und
   - T¹: für (C₁-C₄)-Alkyl oder Benzyl steht,
   von dieser im Folgenden nach den dem Fachmann bekannten Methoden die Benzylgruppe abspaltet und die resultierende Verbindung (XIX) 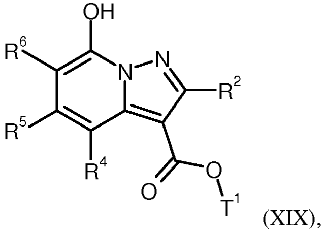 in welcher R², R⁴, R⁵ und R⁶ jeweils die oben angegebenen Bedeutungen haben und
   - T¹: für (C₁-C₄)-Alkyl oder Benzyl steht,
   in einem inerten Lösungsmittel unter Mitsunobu-Bedingungen mit einer Verbindung der Formel (XX) 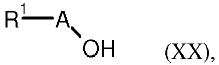 in welcher A und R¹ die oben angegebene Bedeutung haben,
   umgesetzt wird.

Die beschriebenen Verfahren werden durch die nachfolgenden Schemata (Schema 2 und Schema 3) beispielhaft verdeutlicht: 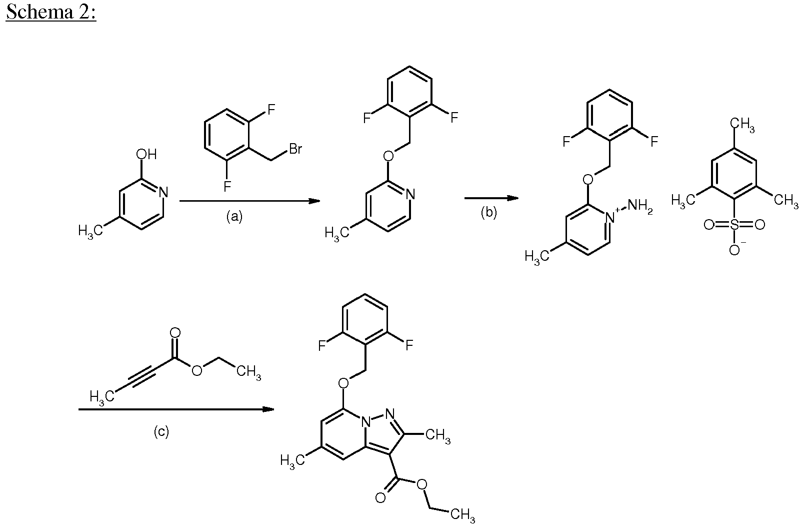 [(a) Ag₂CO₃, THF, Rückfluss; (b) *O*-(2-mesitylenesulfonyl)hydroxylamin (MSH), Dichlormethan, Raumtemperatur; (c) K₂CO₃, DMF, Raumtemperatur]. 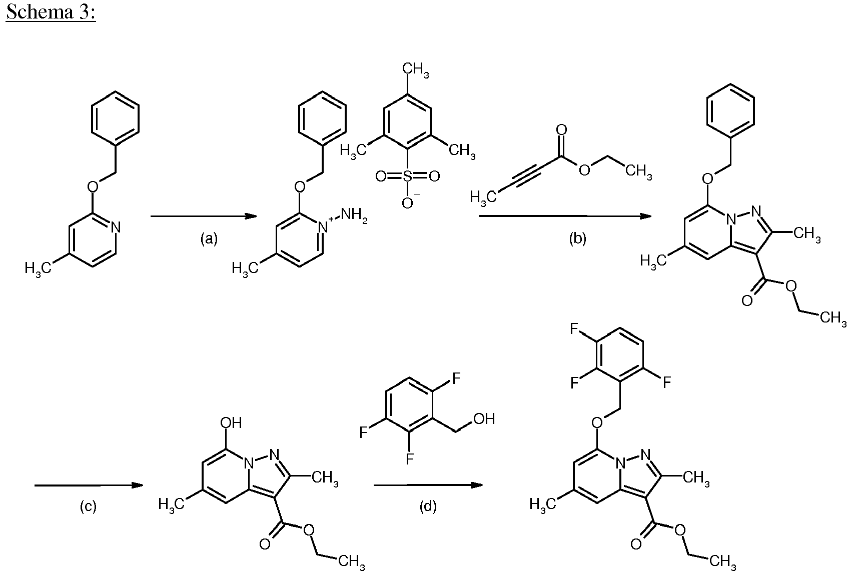 [(a) *O*-(2-mesitylenesulfonyl)hydroxylamin (MSH), Dichlormethan, Raumtemperatur; (b) K₂CO₃, DMF, Raumtemperatur; (c) Cyclohexen, Pd/C, Ethanol, Rückfluss; (d) Triphenylphosphin, Diisopropyl-(E)-diazen-1,2-dicarboxylat (DIAD), THF, Raumtemperatur].

Alternativ zu der in Schema 2 gezeigten Einführung von R¹ durch Umsetzung der Verbindungen (XI) mit Verbindungen der Formel (XII), ist es ebenso möglich - wie in Schema 4 gezeigt - Verbindungen (XXI) mit Alkoholen der Formel (XXII) zu Verbindungen (XXIII) umzusetzen.

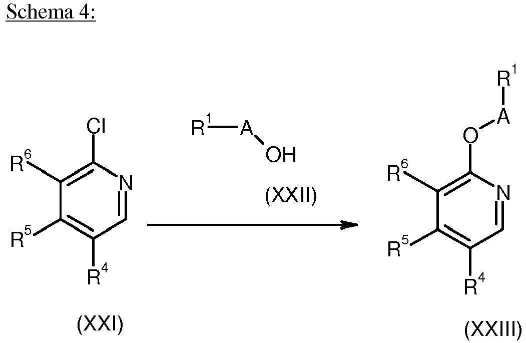

Typische Reaktionsbedingungen für derartige Umsetzungen finden sich in der Fachliteratur, z.B. Poon, K.W.C. Synlet 2005, 6, 841. Typischerweise wird in Anwesenheit einer Base wie Kaliumhydroxid oder Natriumhydrid, gegebenenfalls unter Zusatz eines 18-Krone-6-ethers, in einem inerten Lösemittel, z.B. THF oder Toluol, bei einer Temperatur zwischen 0 °C und dem Siedepunkt des verwendeten Lösemittels umgesetzt.

Inerte Lösungsmittel für den Verfahrensschritt (XI) + (XII) → (XIII) sind beispielsweise Ether wie Diethylether, Dioxan, Tetrahydrofuran, Dimethoxymethan, Glykoldimethylether oder Diethylenglykoldimethylether oder andere Lösungsmittel wie Aceton, Methylethylketon, Essigsäureethylester, Acetonitril, *N,N*-Dimethylformamid, *N,N*-Dimethylacetamid, Dimethylsulfoxid, *N*,*N'*-Dimethylpropylenharnstoff (DMPU), *N*-Methylpyrrolidon (NMP). Ebenso ist es möglich, Gemische der genannten Lösungsmittel einzusetzen. Bevorzugt wird Dimethoxyethan oder Tetrahydrofuran verwendet.

Als Basen für den Verfahrensschritt (XI) + (XII) → (XIII) eignen sich die üblichen anorganischen oder organischen Basen. Hierzu gehören bevorzugt Alkalihydroxide wie beispielsweise Lithium-, Natrium- oder Kaliumhydroxid, Carbonate wie Lithium-, Natrium-, Kalium-, Calcium-, Silber- oder Cäsiumcarbonat gegebenenfalls unter Zusatz eines Alkaliiodids wie beispielsweise Natriumiodid oder Kaliumiodid, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)-amid oder Lithiumdiisopropylamid, oder organische Amine wie Triethylamin, *N*-Methylmorpholin, *N*-Methylpiperidin, *N*,*N*-Diisopropylethylamin, Pyridin, 4-(*N*,*N*-Dimethylamino)-pyridin (DMAP), 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO®). Bevorzugt wird Natrium- tert.-butylat, Kalium-tert.-butylat, Silbercarbonat oder Cäsiumcarbonat verwendet.

Die Reaktion erfolgt im Allgemeinen in einem Temperaturbereich von 0°C bis +120°C, bevorzugt bei +20°C bis +80°C, gegebenenfalls in einer Mikrowelle. Die Umsetzung kann bei normalem, erhöhtem oder bei erniedrigtem Druck durchgeführt werden (z.B. von 0.5 bis 5 bar).

Inerte Lösungsmittel für den Verfahrensschritt (XIII) → (XIV) bzw. (XVI) → (XVII) sind beispielsweise Dichlormethan, 1,2-Dichlorethan oder andere Lösungsmittel wie Aceton, Methylethylketon, Essigsäureethylester, Acetonitril, *N,N*-Dimethylformamid, *N,N*-Dimethylacetamid, Dimethylsulfoxid, *N*,*N'*-Dimethylpropylenharnstoff (DMPU), *N*-Methylpyrrolidon (NMP). Ebenso ist es möglich, Gemische der genannten Lösungsmittel einzusetzen. Bevorzugt wird Dichlormethan verwendet.

Die Reaktion erfolgt im Allgemeinen in einem Temperaturbereich von 0°C bis +120°C, bevorzugt bei +20°C bis +80°C. Die Umsetzung kann bei normalem, erhöhtem oder bei erniedrigtem Druck durchgeführt werden (z.B. von 0.5 bis 5 bar).

Inerte Lösungsmittel für den Ringschluss zum Pyrazolo[1,5-a]pyridin-Grundgerüst (XIV) + (XV) → (II) bzw. (XVII) + (XV) → (XVIII) sind die üblichen organischen Lösungsmittel. Hierzu gehören bevorzugt Alkohole wie Methanol, Ethanol, n-Propanol, Isopropanol, n-Butanol, n-Pentanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, 1,2-Dichlorethan, Acetonitril, Dimethylformamid oder Dimethylsulfoxid. Ebenso ist es möglich, Gemische der genannten Lösungsmittel einzusetzen. Bevorzugt wird Ethanol verwendet.

Der Ringschluss erfolgt im Allgemeinen in einem Temperaturbereich von +20°C bis +150°C, bevorzugt bei +20°C bis +100°C, gegebenenfalls in einer Mikrowelle.

Der Ringschluss (XIV) + (XV) → (II) erfolgt optional in Gegenwart wasserziehender Reaktionszusätze, beispielsweise in Gegenwart von Molekularsieb (3Å oder 4Å Porengröße) oder mittels Wasserabscheider. Die Umsetzung (XIV) + (XV) → (II) erfolgt unter Verwendung eines Überschusses des Reagenzes der Formel (XV), beispielsweise mit 1 bis 20 Äquivalenten des Reagenzes (XV), gegebenenfalls unter Zusatz von Basen (wie z.B. Natriumhydrogencarbonat) wobei die Zugabe dieses Reagenzes einmalig oder in mehreren Portionen erfolgen kann.

Die Abspaltung der Benzylgruppe im Reaktionsschritt (XVIII) → (XIX) erfolgt hierbei nach üblichen, aus der Schutzgruppenchemie bekannten Methoden, vorzugsweise durch Hydrogenolyse in Gegenwart von eines Palladiumkatalysators, wie beispielsweise Palladium auf Aktivkohle, in einem inerten Lösungsmittel, wie beispielsweise Ethanol oder Essigsäureethylester [siehe auch z.B. T.W. Greene und P.G.M. Wuts, Protective Groups in Organic Synthesis, Wiley, New York, 1999].

Die Mitsunobu-Kondensationen (XIX) + (XX) → (II) erfolgt in Gegenwart eines Aktivierungsreagenzes, z.B. Diethyl-(E)-diazen-1,2-dicarboxylat (DEAD) oder Diisopropyl-(E)-diazen-1,2-dicarboxylat (DIAD), sowie eines Phosphinreagenzes, z.B. Triphenylphosphin oder Tributylphosphin, in einem inerten Lösemittel, z.B. THF, Dichlormethan, Toluol oder DMF, bei einer Temperatur zwischen 0 °C und dem Siedepunkt des verwendeten Lösemittels.

Die Verbindungen der Formel (VIII) sind literaturbekannt oder können hergestellt werden, indem
[C] eine Verbindung der Formel (XXIV) 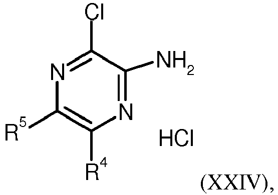 in welcher R⁴ und R⁵ oben angegebene Bedeutung haben,
in einem inerten Lösungsmittel in Gegenwart einer geeigneten Base mit einer Verbindung der Formel (XXV) 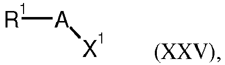 in welcher A und R¹ die oben angegebene Bedeutung haben und
- X¹: für Hydroxy steht,
zu einer Verbindung der Formel (XXVI) 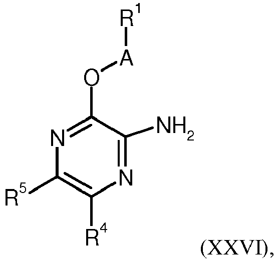 in welcher A, R¹, R⁴ und R⁵ jeweils die oben angegebenen Bedeutungen haben,
umgesetzt wird, und diese anschliessend in einem inerten Lösungsmittel mit einer Verbindung der Formel (XXVII) 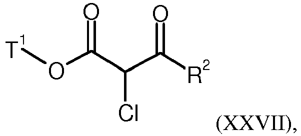 in welcher R² und T¹ jeweils die oben angegebenen Bedeutungen haben,
umgesetzt wird.

Das beschriebene Verfahren wird durch das nachfolgende Schema (Schema 5) beispielhaft verdeutlicht: 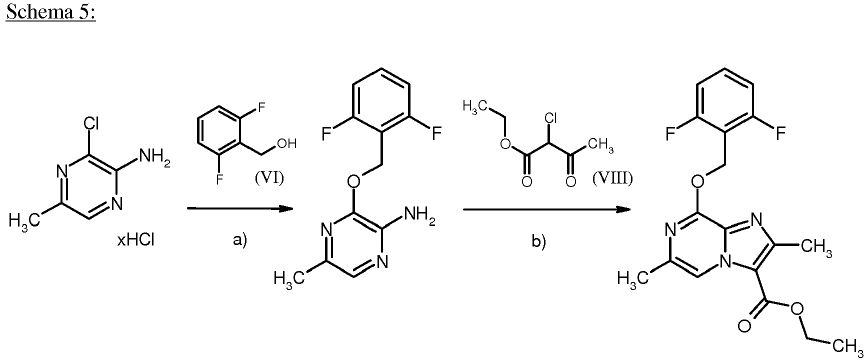 [(a) Kalium-tert-butylat, 1,2-Dimethoxyethan, 80°C; (b) Ethanol, Molekularsieb, Rückfluss].

Die gezeigte Synthesesequenz kann dahingehend modifiziert werden, dass die jeweiligen Reaktionsschritte in einer veränderten Reihenfolge durchlaufen werden. Ein Beispiel für eine solche modifizierte Synthesesequenz ist in Schema 6 gezeigt. 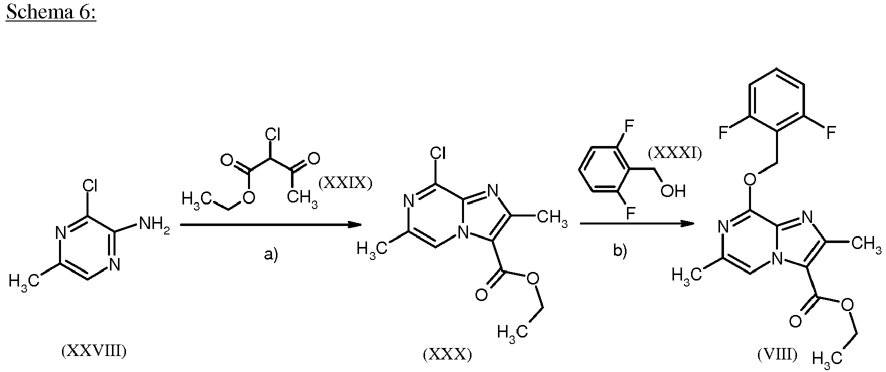 [a): EtOH, Molekularsieb, Rückfluss; b): Kalium-tert-butylat, 1,2-Dimethoxyethan, 80°C].

Inerte Lösungsmittel für den Verfahrensschritt (XXIV) + (XXV) → (XXVI) bzw. (XXX) + (XXXI) → (VIII) sind beispielsweise Ether wie Diethylether, Dioxan, Tetrahydrofuran, Dimethoxymethan, Glykoldimethylether oder Diethylenglykoldimethylether oder andere Lösungsmittel wie Aceton, Methylethylketon, Essigsäureethylester, Acetonitril, *N,N*-Dimethylformamid, *N,N*-Dimethylacetamid, Dimethylsulfoxid, *N*,*N'*-Dimethylpropylenharnstoff (DMPU), *N*-Methylpyrrolidon (NMP). Ebenso ist es möglich, Gemische der genannten Lösungsmittel einzusetzen. Bevorzugt wird Dimethoxyethan verwendet.

Als Basen für den Verfahrensschritt (XXIV) + (XXV) → (XXVI) bzw. (XXX) + (XXXI) → (VIII) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat gegebenenfalls unter Zusatz eines Alkaliiodids wie beispielsweise Natriumiodid oder Kaliumiodid, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)amid oder Lithiumdiisopropylamid, oder organische Amine wie Triethylamin, *N*-Methylmorpholin, *N*-Methylpiperidin, *N*,*N*-Diisopropylethylamin, Pyridin, 4-(*N,N*-Dimethylamino)-pyridin (DMAP), 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO®). Bevorzugt wird Natrium- oder Kalium-tert.-butylat verwendet.

Die Reaktion erfolgt im Allgemeinen in einem Temperaturbereich von 0°C bis +120°C, bevorzugt bei +20°C bis +80°C, gegebenenfalls in einer Mikrowelle. Die Umsetzung kann bei normalem, erhöhtem oder bei erniedrigtem Druck durchgeführt werden (z.B. von 0.5 bis 5 bar).

Inerte Lösungsmittel für den Ringschluss zum Imidazo[1,2-a]pyrazin-Grundgerüst (XXVI) + (XXVII) → (VIII) bzw. (XXVIII) + (XXIX) → (XXX) sind die üblichen organischen Lösungsmittel. Hierzu gehören bevorzugt Alkohole wie Methanol, Ethanol, n-Propanol, Isopropanol, n-Butanol, n-Pentanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, 1,2-Dichlorethan, Acetonitril, Dimethylformamid oder Dimethylsulfoxid. Ebenso ist es möglich, Gemische der genannten Lösungsmittel einzusetzen. Bevorzugt wird Ethanol oder Dimethylformamid verwendet.

Der Ringschluss erfolgt im Allgemeinen in einem Temperaturbereich von +50°C bis +150°C, bevorzugt bei +50°C bis +100°C, gegebenenfalls in einer Mikrowelle.

Der Ringschluss (XXVI) + (XXVII) → (VIII) bzw. (XXVIII) + (XXIX) → (XXX) erfolgt optional in Gegenwart wasserziehender Reaktionszusätze, beispielsweise in Gegenwart von Molekularsieb (3Å oder 4Å Porengröße) oder mittels Wasserabscheider. Die Umsetzung (XVI) + (XVII) → (VIII) bzw. (XXVIII) + (XXIX) → (XXX) erfolgt unter Verwendung eines Überschusses des Reagenzes der Formel (XXVII), beispielsweise mit 1 bis 20 Äquivalenten des Reagenzes (XXVII), gegebenenfalls unter Zusatz von Basen (wie z.B. Natriumhydrogencarbonat) wobei die Zugabe dieses Reagenzes einmalig oder in mehreren Portionen erfolgen kann.

Weitere erfindungsgemäße Verbindungen können gegebenenfalls auch hergestellt werden durch Umwandlungen von funktionellen Gruppen einzelner Substituenten, insbesondere den unter R³ aufgeführten, ausgehend von den nach obigen Verfahren erhaltenen Verbindungen der Formel (I-A) oder (I-B). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen.

Die erfindungsgemäßen Verbindungen besitzen wertvolle pharmakologische Eigenschaften und können zur Vorbeugung und Behandlung von Erkrankungen bei Menschen und Tieren verwendet werden. Die erfindungsgemäßen Verbindungen eröffnen eine weitere Behandlungsalternative und stellen somit eine Bereicherung der Pharmazie dar.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck (Hypertonie), resistente Hypertonie, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz und akute Phasen der Verschlechterung einer bestehenden chronischen Herzinsuffizienz (worsening heart failure).

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Desweiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Weiterer Gegenstand der vorliegenden Erfindung ist ein Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO; und/oder
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil; und/oder
- antithrombotisch wirkende Mittel, beispielhaft und vorzugsweise aus der Gruppe der Thrombozytenaggregationshemmer, der Antikoagulantien oder der profibrinolytischen Substanzen; und/oder
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, neutrale Endopeptidase (NEP) Hemmer und Kombinationen aus diesen Gruppen sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten; und/oder
- antifibrotisch wirkende Mittel, beispielhaft und vorzugsweise aus der Gruppe der Kinase-Inhibitoren oder TGF-beta- bzw. TNF-alpha-Modulatoren

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), Edoxaban (DU-176b), Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, neutrale Endopeptidase (NEP) Hemmer sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan oder einem dualen Angiotensin AII-Antagonisten/NEP-Inhibitor, wie beispielsweise und vorzugsweise LCZ696 (Valsartan/Sacubitril), verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Bei einer bevorzugten Ausführungsform der Erfindung werden die erfindungsgemäßen Verbindungen in Kombination mit einem Kinase-Inhibitor, wie beispielhaft und vorzugsweise Nintedanib, verabreicht.

Bei einer bevorzugten Ausführungsform der Erfindung werden die erfindungsgemäßen Verbindungen in Kombination mit einem TGF-beta- bzw. TNF-alpha-Modulator, wie beispielhaft und vorzugsweise Pirfenidon, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut (= getrocknet)
- aq.: wässrige Lösung
- ber.: berechnet
- Boc: tert-Butyloxycarbonyl
- br.: Verbreitertes Signal (NMR Kupplungsmuster)
- CAS-Nr.: Chemical Abstracts Service Nummer
- Cbz: Benzyloxycarbonyl
- δ: Verschiebung im NMR Spektrum (Angabe in ppm)
- d: Dublett (NMR-Kupplungsmuster)
- DC: Dünnschichtchromatographie
- DCI: direkte chemische Ionisation (bei MS)
- DMAP: 4-*N,N-*Dimethylaminopyridin
- DMF: Dimethylformamid
- DMSO: Dimethylsulfoxid
- EDCI: *N*-[3-(Dimethylamino)propyl]-N'-ethylcarbodiimid
- d. Th.: der Theorie (bei Ausbeute)
- *ent*: enantiomerenrein
- eq.: Äquivalent(e)
- ESI: Elektrospray-Ionisation (bei MS)
- Et: Ethyl
- gef.: gefunden
- h: Stunde(n)
- HATU: N-[(Dimethylamino)(3H-[1,2,3]triazolo[4,5-b]-pyridin-3-yloxy)methylen]-N-methylmethanaminiumhexafluorophosphat
- HOBT: 1H-Benzotriazol-1-ol
- HPLC: Hochdruck-, Hochleistungsflüssigchromatographie
- HRMS: hochaufgelöste Massenspektrometrie
- ID: Innendurchmesser
- konz.: konzentriert
- LC-MS: Flüssigchromatographie-gekoppelte Massenspektrometrie
- LiHMDS: Lithiumhexamethyldisilazid
- m: Multiplett
- Me: Methyl
- min: Minute(n)
- MS: Massenspektrometrie
- NMR: Kernresonanzspektrometrie
- PDA: Photodiodenarraydetektor
- Pd₂dba₃: Tris-(dibenzylidenaceton)-dipalladium
- Ph: Phenyl
- q: Quartett (NMR Kupplungsmuster)
- quint.: Quintett (NMR Kupplungsmuster)
- *rac*: racemisch
- *rel*: relative Stereochemie
- R_{F}: Retentionsfaktor (bei Dünnschichtchromatographie)
- RT: Raumtemperatur
- Rₜ: Retentionszeit (bei HPLC)
- s: Singulett (NMR Kupplungsmuster)
- t: Triplett (NMR Kupplungsmuster)
- TFA: Trifluoressigsäure
- THF: Tetrahydrofuran
- TBTU: (Benzotriazol-1-yloxy)bisdimethylaminomethyliumfluoroborat
- UPLC-MS: Ultradruck-Flüssigchromatographiegekoppelte Massenspektrometrie
- UV: Ultraviolett-Spektrometrie
- v/v: Volumen zu Volumen-Verhältnis (einer Lösung)
- Xantphos: 4,5-Bis(diphenylphosphino)-9,9-dimethylxanthene
- XPHOS: Dicyclohexyl-(2',4',6'-triisopropylbiphenyl-2-yl)-phosphin

### LC-MS- und HPLC-Methoden:

### Methode 1 (LC-MS):

Instrument: Micromass Quattro Premier mit Waters UPLC Acquity; Säule: Thermo Hypersil GOLD 1.9 µ 50 x 1 mm; Eluent A: 1 l Wasser + 0.5 ml 50%ige Ameisensäure, Eluent B: 1 l Acetonitril + 0.5 ml 50%ige Ameisensäure; Gradient: 0.0 min 90% A → 0.1 min 90% A → 1.5 min 10% A → 2.2 min 10% A Ofen: 50°C; Fluss: 0.33 ml/min; UV-Detektion: 210 nm

### Methode 2 (LC-MS):

Instrument: Waters ACQUITY SQD UPLC System; Säule: Waters Acquity UPLC HSS T3 1.8 µ 50 x 1 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 1.2 min 5% A → 2.0 min 5% A Ofen: 50°C; Fluss: 0.40 ml/min; UV-Detektion: 210 - 400 nm.

### Methode 3 (LC-MS):

Instrument: Micromass Quattro Premier mit Waters UPLC Acquity; Säule: Thermo Hypersil GOLD 1.9 µ 50 x 1 mm; Eluent A: 1 l Wasser + 0.5 ml 50%ige Ameisensäure, Eluent B: 1 l Acetonitril + 0.5 ml 50%ige Ameisensäure; Gradient: 0.0 min 97% A → 0.5 min 97% A → 3.2 min 5% A → 4.0 min 5% A Ofen: 50°C; Fluss: 0.3 ml/min; UV-Detektion: 210 nm.

### Methode 4 (präparative HPLC):

Chromatorex C18 10µ 250x20 mm Gradient: A = Wasser + 0.5% HCOOH, B = CH₃CN, 0 min = 5% B, 3 min = 5% B vorspülen ohne Substanz, dann Injektion, 5 min = 5% B, 25 min = 30% B, 38 min = 30% B, 38.1 min = 95% B, 43 min= 95% B, 43.01 min= 5% B, 48.0 min= 5% B Flussrate 20 ml/min, Wellenlänge 210 nm.

### Methode 5 (präparative HPLC):

Chromatorex C18 10µ 250x20 mm Gradient: A = Wasser + 0.5% HCOOH, B = CH₃CN, 0 min = 5% B, 3 min = 5% B vorspülen ohne Substanz, dann Injektion, 5 min = 5% B, 25 min = 50% B, 38 min = 50% B, 38.1min = 95% B, 43 min= 95% B, 43.01 min= 5% B, 48.0 min= 5% B Flussrate 20 ml/min, Wellenlänge 210 nm.

### Methode 6 (präparative HPLC):

XBridge Prep. C18 5µ 50x19 mm Gradient: A = Wasser + 0.5% NH₄OH, B = CH₃CN, 0 min = 5% B, 3 min = 5% B vorspülen ohne Substanz, dann Injektion, 5 min = 5% B, 25 min = 50% B, 38 min = 50% B, 38.1 min = 95% B, 43 min= 95% B, 43.01 min= 5% B, 48.0 min= 5% B Flussrate 15 ml/min, Wellenlänge 210 nm.

### Methode 7 (LC-MS):

Instrument MS: Waters (Micromass) QM; Instrument HPLC: Agilent 1100 Serie; Säule: Agient ZORBAX Extend-C18 3.0x50mm 3.5-Micron; Eluent A: 1 l Wasser + 0.01 mol Ammoniumcarbonat, Eluent B: 1 l Acetonitril; Gradient: 0.0 min 98% A → 0.2min 98% A → 3.0 min 5% A→ 4.5 min 5% A; Ofen: 40°C; Fluss: 1.75 ml/min; UV-Detektion: 210 nm.

### Methode 8 (LC-MS):

Instrument: Waters ACQUITY SQD UPLC System; Säule: Waters Acquity UPLC HSS T3 1.8 µ 30 x 2 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Methode 9 (präparative HPLC):

Instrument MS: Waters, Instrument HPLC: Waters (Säule Waters X-Bridge C18, 18 mm x 50 mm, 5 µm, Eluent A: Wasser + 0.05% Triethylamin, Eluent B: Acetonitril (ULC) + 0.05% Triethylamin, mit Gradient; Fluss: 40 ml/min; UV-Detektion: DAD; 210 - 400 nm).
bzw.:
Instrument MS: Waters, Instrument HPLC: Waters (Säule Phenomenex Luna 5µ C18(2) 100A, AXIA Tech. 50 x 21.2 mm, Eluent A: Wasser + 0.05% Ameisensäuren, Eluent B: Acetonitril (ULC) + 0.05% Ameisensäure, mit Gradient; Fluss: 40 ml/min; UV-Detektion: DAD; 210 - 400 nm).

### Methode 10 (LC-MS):

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril (ULC) + 0.025% Ameisensäure; Gradient: 0.0 min 98%A - 0.9 min 25%A - 1.0 min 5%A - 1.4 min 5%A - 1.41 min 98%A - 1.5 min 98%A; Ofen: 40°C; Fluss: 0.600 ml/min; UV-Detektion: DAD; 210 nm.

### Methode 11 (MS):

Instrument: Waters ZQ 2000; Elektrospray-Ionisierung; Eluent A: 1 l Wasser + 0.25 ml 99%ige Ameisensäure, Eluent B: 1 l Acetonitril + 0.25 ml 99%ige Ameisensäure; 25% A, 75% B; Fluss: 0.25 ml/min.

### Methode 12 (DCI-MS):

Instrument: Thermo Fisher-Scientific DSQ; chemische Ionisierung; Reaktantgas NH₃; Quellentemperatur: 200°C; Ionisierungsenergie 70eV.

### Methode 13 (LC-MS):

Instrument MS: Waters (Micromass) Quattro Micro; Instrument HPLC: Agilent 1100 Serie; Säule: YMC-Triart C18 3 µ 50 x 3 mm; Eluent A: 1 l Wasser + 0.01 mol Ammoniumcarbonat, Eluent B: 1 l Acetonitril; Gradient: 0.0 min 100% A → 2.75 min 5% A → 4.5 min 5% A; Ofen: 40°C; Fluss: 1.25 ml/min; UV-Detektion: 210 nm.

### Methode 14 (GC-MS):

Instrument: Thermo Scientific DSQII, Thermo Scientific Trace GC Ultra; Säule: Restek RTX-35MS, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).

### Methode 15 (LC-MS, analytisch):

Instrument: Agilent MS Quad 6150; HPLC: Agilent 1290; Säule: Waters Acquity UPLC HSS T3 1.8 µ 50 x 2.1 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 0.3 min 90% A → 1.7 min 5% A → 3.0 min 5% A Ofen: 50°C; Fluss: 1.20 ml/min; UV-Detektion: 205 - 305 nm.

### Methode 16 (LC-MS, analytisch):

Gerätetyp MS: Thermo Scientific FT-MS; Gerätetyp UHPLC+: Thermo Scientific UltiMate 3000; Säule: Waters, HSST3, 2.1 x 75 mm, C18 1.8 µm; Eluent A: 1 l Wasser + 0.01% Ameisensäure; Eluent B: 1 l Acetonitril + 0.01% Ameisensäure; Gradient: 0.0 min 10% B → 2.5 min 95% B → 3.5 min 95% B; Ofen: 50°C; Fluss: 0.90 ml/min; UV-Detektion: 210 nm/ Optimum Integration Path 210-300 nm.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Die in den folgenden Paragraphen angegebenen Multiplizitäten von Protonensignalen in ¹H-NMR-Spektren geben die jeweils beobachtete Signalform wieder und berücksichtigen keine Signalphänomene höherer Ordnung. Alle Angaben in ¹H-NMR-Spektren geben die Chemischen Verschiebungen δ in ppm an.

Zusätzlich können die Ausgangsverbindungen, Intermediate und Ausführungsbeispiele als Hydrate vorliegen. Eine quantitative Bestimmung des Wassergehaltes erfolgte nicht. Die Hydrate können unter Umständen einen Einfluss auf das ¹H-NMR-Spektrum haben und ggf. das Wasser-Signal in ¹H-NMR verschieben und/oder stark verbreitern.

Die Methyl-Gruppe des chemischen Systems "2-Methyl-pyrazolo[1,5-a]pyridin" erscheint in ¹H-NMR-Spektren als Singulett (oftmals in DMSO-d₆ und im Bereich von 2.40 - 2.60 ppm) und ist etweder klar als solches erkennbar, ist mit den Lösungsmittelsignalen überlagert oder liegt vollständig unter den Signalen der Lösungsmittel. Die Angabe dieses Signals in den ¹H-NMR-Spektren kann antizipierend erfolgen.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen eine ausreichend basische bzw. saure Funktionalität enthalten. Ein solches Salz kann durch verschiedene dem Fachmann bekannte Methoden in die entsprechende freie Base bzw. Säure überführt werden.

Wenn bei den im Folgenden beschriebenen Synthese-Intermediaten und Ausführungsbeispielen der Erfindung eine Verbindung in der Form eines Salzes der korrespondierenden Base bzw. Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃CO₂H", "x Na⁺" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

### Ausgangsverbindungen und Intermediate:

### Beispiel 1A

### 2-[(2,6-Difluorbenzyl)oxy]-4-methylpyridin

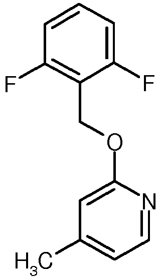

Eine Mischung von 5.00 g (24.2 mmol, 1.0 eq.) 2,6-Difluorbenzylbromid [CAS-Nr: 85118-00-9] und 3.16 g (29.0 mmol, 1.2 eq.) 2-Hydroxy-4-methylpyridin [CAS-Nr: 13466-41-6] wurden in 50 ml THF gelöst. Die Lösung wurde mit 7.99 g (29.0 mmol, 1.2 eq.) Silbercarbonat versetzt und unter Lichtausschluss über Nacht auf Rückfluss erhitzt. Anschließend wurde die Reaktionsmischung über Kieselgur filtriert, mit Essigsäureethylester eluiert und das Filtrat wurde eingeengt. Das Rohprodukt wurde mittels Biotage Isolera (100 g Kieselgelkartusche, Cyclohexan/Essigsäureethylester-Gradient, 0 % nach 10 % Essigsäureethylester) gereinigt. Es wurden 3.51 g der Titelverbindung (61 % d. Th.) erhalten.
DC (Kieselgel, Cyclohexan/Essigsäureethylester 10:1): R_{F} = 0.50
LC-MS (Methode 2): Rₜ = 1.17 min
MS (ESpos): m/z = 236 (M+H)⁺
¹H-NMR (400 MHz, DMSO-d₆): δ [ppm] = 2.26 (s, 3H), 5.35 (s, 2H), 6.66 (s, 1H), 6.86 (d, 1H), 7.12 - 7.21 (m, 2H), 7.47 - 7.56 (m, 1H), 8.06 (d, 1H).

### Beispiel 2A

### 1-Amino-2-[(2,6-difluorbenzyl)oxy]-4-methylpyridinium-2,4,6-trimethylbenzolsulfonat

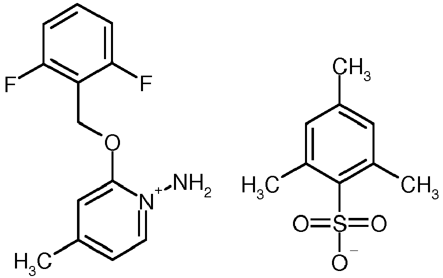

Eine Mischung von 3.4 ml (43.9 mmol, 10 eq.) Trifluoressigsäure und 0.33 ml Wasser wurden auf -5°C gekühlt. Bei dieser Temperatur wurden 1.88 g (6.59 mmol, 1.5 eq.) Ethyl-(1E)-N-[(mesitylsulfonyl)oxy-]ethanimidoat [CAS-Nr: 38202-27-6] portionsweise zugegeben. Nach 1.5 h wurden 30 ml Eiswasser hinzugefügt, kurz nachgerührt und das ausgefallene O-(2-Mesitylenesulfonyl)hydroxylamin (MSH) wurde durch eine vorher gekühlte Fritte abfiltriert und mit 30 ml Eiswasser gewaschen. Das wasserfeuchte *O*-(2-Mesitylenesulfonyl)hydroxylamin wurde in 12 ml Dichlormethan gelöst, mit Magnesiumsulfat getrocknet, filtriert und und das Filtrat wurde direkt zu einer Lösung von 1.03 g (4.39 mmol, 1.0 eq.) 2-[(2,6-Difluorbenzyl)oxy]-4-methylpyridin aus Beispiel 1A in 2 ml Dichlormethan getropft. Die Mischung wurde über Nacht bei RT gerührt. Anschließend wurde Diethylether zugetropft, der anfallende Niederschlag abfiltriert, mit Diethylether gewaschen und getrocknet. Es wurden 1.3 g der Titelverbindung isoliert (59% d. Th., Reinheit 90%).
¹H-NMR (400 MHz, DMSO-d₆): δ [ppm] = 2.17 (s, 3H), 2.46 - 2.57 (s, 3H und s, 6H überlagert mit den Lösungsmittelsignal), 5.64 (s, 2H), 6.74 (s, 2H), 7.23 - 7.48 (m, 4H), 7.60 - 7.70 (m, 1H), 7.86 (br s, 1H), 8.44 (d, 1H).

### Beispiel 3A

### Ethyl-7-[(2,6-difluorbenzyl)oxy]-2,5-dimethylpyrazolo[1,5-a]pyridin-3-carboxylat

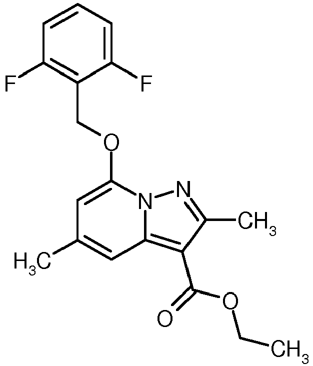

1.62 g (3.60 mmol, 1.0 eq.) 1-Amino-2-[(2,6-difluorbenzyl)oxy]-4-methylpyridinium-2,4,6-trimethylbenzolsulfonat aus Beispiel 2A wurden in 36 ml DMF gelöst und mit 0.84 ml (7.19 mmol, 2.0 eq.) Ethylbut-2-inoat [CAS-Nr: 4341-76-8] versetzt. Es wurden 0.994 g (7.19 mmol, 2.0 eq.) Kaliumcarbonat zugegeben und die Mischung wurde 1.5 h bei RT gerührt. Anschließend wurde das Gemisch auf 150 ml Wasser gegossen, kurz ausgerührt, der ausgefallene Feststoff abfiltriert, mit Wasser gewaschen und getrocknet. Es wurden 440 mg der Titelverbindung (45% d. Th., 87%) erhalten.
LC-MS (Methode 2): Rₜ = 1.22 min
MS (ESpos): m/z = 361 (M+H)⁺

### Beispiel 4A

### 7-[(2,6-Difluorbenzyl)oxy]-2,5-dimethylpyrazolo[1,5-a]pyridin-3-carbonsäure

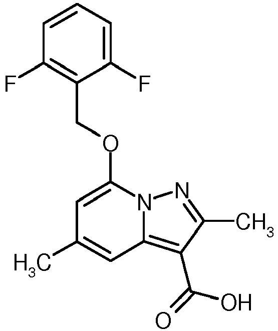

Eine Lösung von 0.440 g(1.22 mmol, 1 eq.) Ethyl-7-[(2,6-difluorbenzyl)oxy]-2,5-dimethylpyrazolo[1,5-a]pyridin-3-carboxylat aus Beispiel 3A in 12.7 ml Dioxan wurde mit 4.9 ml (4.88 mmol, 4.0 eq.) 1 N wässriger Natronlauge versetzt und 36 h bei 90°C gerührt. Anschließend wurde das Reaktionsgemisch eingeengt und der ausgefallene Feststoff abfiltriert. Das Filtrat wurde mit 6 N wässriger Salzsäure angesäuert, kurz ausgerührt, der ausgefallene Feststoff wurde abfiltriert, mit Wasser gewaschen und getrocknet. Es wurden 248 mg der Titelverbindung (61% d. Th., Reinheit 60%) erhalten, die ohne weitere Reinigung weiter umgesetzt wurde.
LC-MS (Methode 2): Rₜ = 0.96 min
MS (ESpos): m/z = 333 (M+H)⁺

### Beispiel 5A

### 2-(Benzyloxy)-4-methylpyridin

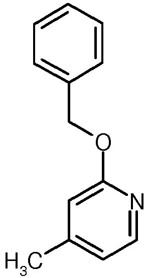

Eine Mischung von 13.6 ml (114 mmol, 1.0 eq.) Benzylbromid und 15.0 g (137 mmol, 1.2 eq.) 2-Hydroxy-4-methylpyridin [CAS-Nr: 13466-41-6] wurden in 470 ml THF gelöst. Die Lösung wurde mit 37.9 g (137 mmol, 1.2 eq.) Silbercarbonat versetzt und unter Lichtausschluss über Nacht auf Rückfluss erhitzt. Anschließend wurde die Reaktionsmischung über Kieselgur filtriert, mit Essigsäureethylester eluiert und das Filtrat wurde eingeengt. Das Rohprodukt wurde durch Kieselgelchromatographie (700 g Kieselgel, Cyclohexan/Essigsäureethylester, 95:5) gereinigt. Es wurden 21.4 g der Titelverbindung (94 % d. Th.) erhalten.
DC (Kieselgel, Cyclohexan/Essigsäureethylester 9:1): R_{F} = 0.41
LC-MS (Methode 2): Rₜ = 1.12 min
MS (ESpos): m/z = 200 (M+H)⁺
¹H-NMR (400 MHz, DMSO-d₆): δ [ppm] = 2.27 (s, 3H), 5.33 (s, 2H), 6.70 (s, 1H), 6.83 (d, 1H), 7.27 - 7.45 (m, 5H), 8.02 (d, 1H).

### Beispiel 6A

### 1-Amino-2-(benzyloxy)-4-methylpyridinium-2,4,6-trimethylbenzolsulfonat

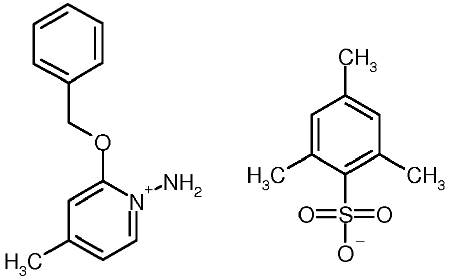

Eine Mischung von 18.0 ml Trifluoressigsäure (233 mmol, 10 eq.) und 2.66 ml Wasser wurden auf -5°C gekühlt. Bei dieser Temperatur wurden 9.99 g (35.0 mmol, 1.5 eq.) Ethyl-(1E)-N-[(mesitylsulfonyl)oxy-]ethanimidoat [CAS-Nr: 38202-27-6] portionsweise zugegeben. Nach 1.5 h wurden 150 ml Eiswasser zugegeben, kurz nachgerührt und mit 100 ml Dichlormethan extrahiert. Die organische Phase wurde mit Magnesiumsulfat getrocknet, filtriert und die erhaltene Lösung von *O*-(2-mesitylenesulfonyl)hydroxylamin (MSH) wurde direkt zu einer auf 0°C gekühlten Lösung von 4.65 g (23.3 mmol, 1.0 eq.) 2-(Benzyloxy)-4-methylpyridin aus Beispiel 5A in 50 ml Dichlormethan getropft. Es wurde für 2 h bei RT gerührt. Anschließend wurde 1 l Diethylether zugetropft, der ausgefallene Feststoff abfiltriert, mit 250 mL Diethylether gewaschen und an der Luft getrocknet. Es wurden 4.6 g der Titelverbindung isoliert (48% d. Th.).
LC-MS (Methode 2): Rₜ = 0.45 min;
MS (ESpos): m/z = 215 (C₁₃H₁₅N₂O) (M)⁺; Rₜ = 0.57 min;
MS (ESneg): m/z = 199 (C₉H₁₁O₃S)⁻;

### Beispiel 7A

### Ethyl-7-(benzyloxy)-2,5-dimethylpyrazolo[1,5-a]pyridin-3-carboxylat

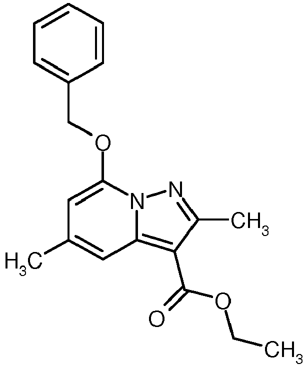

11.7 g (28.2 mmol, 1.0 eq.) l-Amino-2-(benzyloxy)-4-methylpyridinium-2,4,6-trimethylbenzolsulfonat aus Beispiel 6A wurden in 280 ml DMF gelöst und mit 6.6 ml (56 mmol, 2.0 eq.) Ethylbut-2-inoat [CAS-Nr: 4341-76-8] versetzt. Es wurden 7.8 g (56 mmol, 2.0 eq.) Kaliumcarbonat zugegeben und 1 h bei RT gerührt. Anschließend wurden 3.9 g (28 mmol, 1 eq.) Kaliumcarbonat zugegeben und die Mischung wurde für weitere 16 h bei RT gerührt. Dann wurde das Gemisch auf 540 ml Wasser gegossen, kurz ausgerührt, der ausgefallene Feststoff abfiltriert, mit 220 ml Wasser gewaschen und getrocknet. Es wurden 3.1 g der Titelverbindung (34% d. Th., Reinheit 87%) erhalten.
LC-MS (Methode 2): Rₜ = 1.20 min
MS (ESpos): m/z = 325 (M+H)⁺
¹H-NMR (400 MHz, DMSO-d₆): δ [ppm] = 1.34 (t, 3H), 2.43 (s, 3H), 4.27 (q, 2H), 5.43 (s, 2H), 6.60 (d, 1H), 7.37 - 7.49 (m, 4H), 7.52 - 7.59 (m, 2H), [s, 3H unter Lösungsmittelsignal].

### Beispiel 8A

### Ethyl-7-hydroxy-2,5-dimethylpyrazolo[1,5-a]pyridin-3-carboxylat

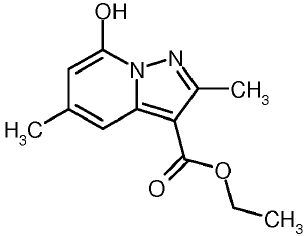

2 g (5.98 mmol) Ethyl-7-(benzyloxy)-2,5-dimethylpyrazolo[1,5-a]pyridin-3-carboxylat aus Beispiel 7A wurden unter Argon in 80 ml Ethanol vorgelegt, mit 636 mg (0.59 mmol, 10%ig) Palladium auf Aktivkohle und 18 ml (179.42 mmol) Cyclohexen versetzt. Das Reaktionsgemisch wurde 2.5 Stunden unter Rückfluss gerührt. Dann wurde über Kieselgur abfiltriert, mit Ethanol gewaschen und das Filtrat eingeengt. Der Rückstand wurde in DMSO und Acetonitril aufgenommen und mittels präparativer HPLC gereinigt (RP18 Säule, Laufmittel: Acetonitril/Wasser-Gradient unter Zusatz von 0.1% TFA). Die Produktfraktionen wurden vereinigt, eingeengt und lyophilisiert. Es wurden 1.2 g der Zielverbindung (86% d. Th.) erhalten.
LC-MS (Methode 7): Rₜ = 1.60 min
MS (ESpos): m/z = 235 (M+H)⁺
¹H-NMR (500 MHz, DMSO-d₆): δ = 1.33 (t, 3H), 2.35 (s, 3H), 2.54 (s, 3H; verdeckt unter Lösungsmittelpeak), 4.26 (q, 2H), 6.17 (d, 1H), 7.26 (s, 1H).

### Beispiel 9A

### Ethyl-2,5-dimethyl-7-[(2,3,6-trifluorbenzyl)oxy]pyrazolo[1,5-a]pyridin-3-carboxylat

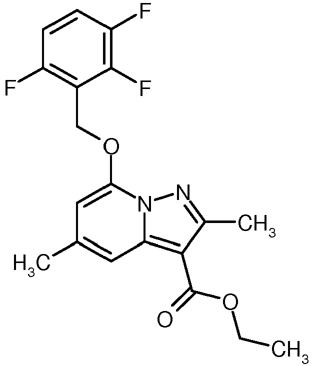

1.2 g (5.25 mmol) Ethyl-7-hydroxy-2,5-dimethylpyrazolo[1,5-a]pyridin-3-carboxylat aus Beispiel 8A wurden in 48 ml THF gelöst. 1.7 g (10.50 mmol) 2,3,6-Trifluorbenzylalkohol und 2.9 g (11.03 mmol) Triphenylphosphin wurden hinzugegeben. Anschließend wurde die Lösung mit 2.2 ml (11.03 mmol) Diisopropyl-(E)-diazen-1,2-dicarboxylat versetzt und 1 h bei RT gerührt. 120 ml tert-Butylmethylether wurden hinzugegeben, dann kurz ausgerührt, der enstandene Feststoff abfiltriert und im Hochvakuum getrocknet. Es wurden 1.2 g der Zielverbindung (62% d. Th.) erhalten.
LC-MS (Methode 2): Rₜ = 1.22 min
MS (ESpos): m/z = 379 (M+H)⁺
¹H-NMR (500 MHz, DMSO-d₆): δ = 1.34 (t, 3H), 2.46 (s, 3H), 2.51 (s, 3H; verdeckt unter Lösungsmittelpeak), 4.28 (d, 2H), 5.51 (s, 2H), 6.70 (s, 1H), 7.29 - 7.37 (m, 1H), 7.48 (s, 1H), 7.66 - 7.76 (m, 1H).

### Beispiel 10A

### 2,5-Dimethyl-7-[(2,3,6-trifluorbenzyl)oxy]pyrazolo[1,5-a]pyridin-3-carbonsäure

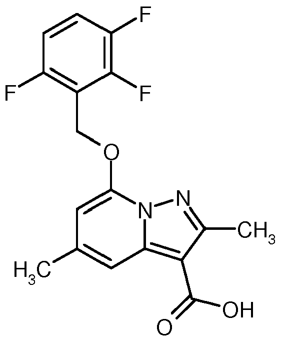

700 mg (1.81 mmol) Ethyl-2,5-dimethyl-7-[(2,3,6-trifluorbenzyl)oxy]pyrazolo[1,5-a]pyridin-3-carboxylat aus Beispiel 9A wurden in 18 ml Dioxan vorgelegt und auf 90°C erhitzt. Es wurden 4.5 ml Dioxan und 7.25 ml (14.50 mmol) 2 N wässrige Natronlauge hinzugegeben und die Reaktionsmischung wurde zwei Tage bei 90°C gerührt. Es wurden nochmals 3.63 ml (7.26 mmol) 2 N wässrige Natronlauge hinzugegeben und es wurde weitere 2 Stunden bei 90°C gerührt. Die Reaktionslösung wurde mit 15 ml IN wässriger Salzsäure versetzt und 30 min. gerührt. Dabei fiel Feststoff aus. Diese Suspension wurde filtriert, der abfiltrierte Feststoff wurde mit wenig Wasser gewaschen und im Hochvakuum getrocknet. Es wurden 358 mg der Zielverbindung (54% d. Th.) erhalten.
LC-MS (Methode 2): Rₜ = 0.97 min
MS (ESpos): m/z = 351 (M+H)⁺

### Beispiel 11A

### Methyl-2-cyclopropyl-7-[(2,6-difluorbenzyl)oxy]-5-methylpyrazolo[1,5-a]pyridin-3-carboxylat

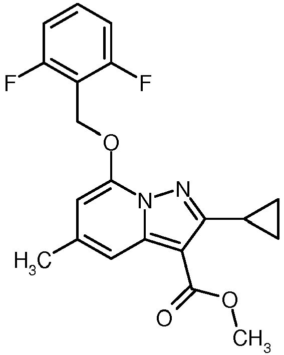

1.0 g (2.22 mmol) l-Amino-2-[(2,6-difluorbenzyl)oxy]-4-methylpyridinium-2,4,6-trimethylbenzolsulfonat aus Beispiel 2A wurden in 7.2 ml DMF gelöst und mit 496 mg (4.00 mmol) Methyl-3-cyclopropylprop-2-inoat versetzt. Es wurden 552 mg (4.00 mmol) Kaliumcarbonat zugegeben und die Mischung wurde für 3 h bei RT gerührt. Anschließend wurde das Gemisch auf 50 ml Wasser gegossen, kurz ausgerührt, der ausgefallene Feststoff abfiltriert, mit Wasser gewaschen und getrocknet. Es wurden 384 mg der Titelverbindung (46% d. Th.) erhalten.
LC-MS (Methode 2): Rₜ = 1.20 min
MS (ESpos): m/z = 373 (M+H)⁺
¹H-NMR (500 MHz, DMSO-d₆): δ [ppm] = 0.88 - 0.94 (m, 2H), 0.95 - 1.00 (m, 2H), 2.43 (s, 3H), 2.70 - 2.77 (m, 1H), 3.83 (s, 3H), 5.46 (s, 2H), 6.66 (s, 1H), 7.21 - 7.27 (m, 2H), 7.48 (s, 1H), 7.57 - 7.64 (m, 1H).

### Beispiel 12A

### 2-Cyclopropyl-7-[(2,6-difluorbenzyl)oxy]-5-methylpyrazolo[1,5-a]pyridin-3-carbonsäure

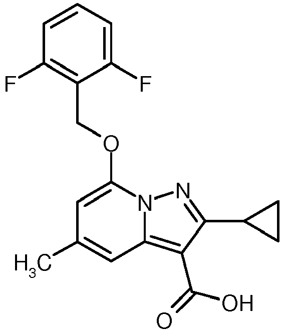

Eine Lösung von 384 mg (1.02 mmol) Methyl-2-cyclopropyl-7-[(2,6-difluorbenzyl)oxy]-5-methylpyrazolo[1,5-a]pyridin-3-carboxylat aus Beispiel 11A in 10.6 ml Dioxan wurde mit 10.2 ml (10.2 mmol) 1 N Natronlauge versetzt und das Gemisch wurde 7 h bei 100°C gerührt. Die Reaktionslösung wurde abgekühlt und mit 1 N Salzsäure auf pH 2 gestellt. Der dabei ausgefallene Feststoff wurde abfiltriert und im Hochvakuum getrocknet. Das Filtrat wurde nochmals mit 1 N Salzsäure versetzt. Der dabei ausgefallene Feststoff wurde abfiltriert und und im Hochvakuum zusammen mit dem zuvor isolierten Feststoff getrocknet. Es wurden insgesamt 361 mg der Titelverbindung (74%ig laut LC-MS, 73% d. Th.) erhalten und ohne weitere Reinigung umgesetzt.
LC-MS (Methode 2): Rₜ = 1.00 min
MS (ESpos): m/z = 359 (M+H)⁺
¹H-NMR (400 MHz, DMSO-d₆): δ [ppm] = 0.87 - 0.99 (m, 4H), 2.42 (s, 3H), 2.73 - 2.82 (m, 1H), 5.45 (s, 2H), 6.61 (s, 1H), 7.20 - 7.28 (m, 2H), 7.48 (s, 1H), 7.55 - 7.65 (m, 1H), 12.29 (br. s, 1H).

### Beispiel 13A

### 3-[(2,6-Difluorbenzyl)oxy]-5-methylpyrazin-2-amin

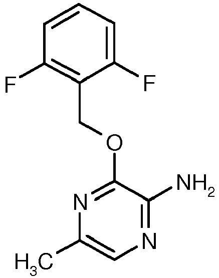

Zu einer Lösung von 2.71 g (2,6-Difluorphenyl)methanol [CAS-Nr.: 19064-18-7] (18.8 mmol, 1.3 eq.) in 120 ml 1,2-Dimethoxyethan wurden 4.86 g Kalium-tert-butylat (43.3 mmol, 3.0 eq.) gegeben und das Gemisch wurde 60 min bei RT gerührt. Anschließend wurden 2.60 g 2-Amino-3-chlor-5-methylpyrazin Hydrochlorid [CAS-Nr.: 89182-14-9] (14.4 mmol, 1.0 eq.) zugegeben und die Mischung wurde über Nacht bei 80°C gerührt. Nach Abkühlen auf Raumtemperatur wurde gesättigte wässrige Natriumhydrogencarbonatlösung zugegeben und die wässrige Phase dreimal mit Dichlormethan extrahiert. Die vereinigten organischen Phasen wurden mit gesättigter wässriger Natriumchloridlösung gewaschen, mit Magnesiumsulfat getrocknet, filtriert und eingeengt. Der Rückstand wurde mittels Biotage Isolera (340 g Kieselgelkartusche, Cyclohexan /Essigsäureethylester Gradient, 10% -> 72% Essigsäureethylester) gereingt. Es wurden 1.77 g der Titelverbindung (39% d. Th., Reinheit 85%) erhalten.
LC-MS (Methode 2): Rₜ = 0.94 min
MS (ESpos): m/z = 252 (M+H)⁺
¹H-NMR (400 MHz, DMSO-d₆): δ [ppm] = 2.20 (s, 3H), 5.35 (s, 2H), 5.88 (s, 2H), 7.09 - 7.23 (m, 2H), 7.37 (s, 1H), 7.46 - 7.57 (m, 1H).

### Beispiel 14A

### Ethyl-8-[(2,6-difluorbenzyl)oxy]-2,6-dimethylimidazo[1,2-a]pyrazin-3-carboxylat

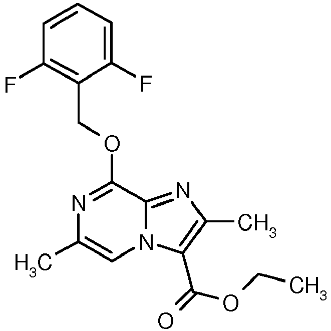

Eine Lösung von 1.77 g 3-[(2,6-Difluorbenzyl)oxy]-5-methylpyrazin-2-amin (7.05 mmol, 1.0 eq.) aus Beispiel 13A in 50 ml Ethanol wurde mit Molekularsieb 4A und 11.1 g Ethyl-2-chloroacetoacetat [CAS-Nr.: 609-15-4] (70.5 mmol, 10 eq.) versetzt und über Nacht auf Rückfluss erhitzt. Anschließend wurden 11.1 g Ethyl-2-chloroacetoacetat (70.5 mmol, 10.0 eq.) zugegeben und es wurde über Nacht auf Rückfluss erhitzt. Dann wurde abfiltriert, das Filtrat eingeengt, der erhaltene Rückstand mit Diethylether ausgerührt, abfiltriert und das Filtrat eingeengt. Der Rückstand wurde zweimal mittels Biotage Isolera (120 g Kieselgelkartusche, Cyclohexan/Essigsäureethylester Gradient) gereinigt. Es wurden 0.81 g der Titelverbindung (16% d. Th., Reinheit 52%) isoliert.
LC-MS (Methode 2): Rₜ = 1.28 min
MS (ESpos): m/z = 362 (M+H)⁺

### Beispiel 15A

### 8-[(2,6-Difluorbenzyl)oxy]-2,6-dimethylimidazo[1,2-a]pyrazin-3-carbonsäure

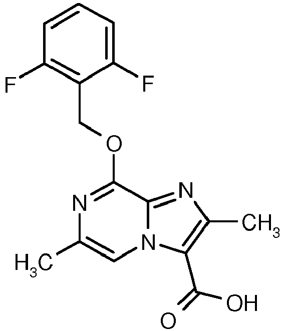

Eine Lösung von 800 mg Ethyl-8-[(2,6-difluorbenzyl)oxy]-2,6-dimethylimidazo[1,2-a]pyrazin-3-carboxylat (Reinheit 52%, 1.15 mmol, 1.0 eq.) aus Beispiel 14A in 10 ml Dioxan wurde mit 5.8 ml 1 N wässriger Natronlauge (5.8 mmol, 5 eq.) versetzt und 2 h bei RT gerührt. Anschließend wurde das Gemisch eingeengt, der Rückstand in Wasser aufgenommen und der unlösliche Feststoff wurde abfiltriert. Das Filtrat wurde mit 1 N wässriger Salzsäure angesäuert, der gebildete Feststoff wurde abfiltriert und getrocknet. Es wurden 354 mg der Titelverbindung (83% d. Th., Reinheit 90%) isoliert.
LC-MS (Methode 2): Rₜ = 0.99 min
MS (ESpos): m/z = 334 (M+H)⁺
¹H-NMR (400 MHz, DMSO-d₆): δ [ppm] = 2.41 (s, 3H), 2.54 (s, 3H verborgen unter Lösemittelsignal), 5.55 (s, 2H), 7.12 - 7.28 (m, 2H), 7.49 - 7.64 (m, 1H), 8.64 (s, 1H), 13.20 - 13.66 (br s, 1H).

### Beispiel 16A

### 2-Methyl-2-nitropropyltrifluormethansulfonat

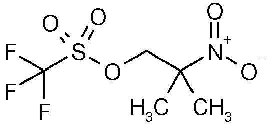

1.0 g (8.40 mmol) 2-Methyl-2-nitropropan-1-ol wurden in 20 ml Dichlormethan vorgelegt, mit 1.0 ml (12.59 mmol) Pyridin versetzt, auf 0°C abgekühlt und langsam mit 1.85 ml (10.91 mmol) Trifluormethansulfonsaeureanhydrid versetzt. Anschließend wurde 1 h bei 0°C gerührt. Der Reaktionsverlauf wurde durch DC kontrolliert (Cyclohexan/Essigsäureethylester 7/3, Anfärbereagenz Kaliumpermangantfärbereagenz). Die Reaktionslösung wurde je einmal mit Wasser und gesättigter, wässriger Natriumchloridlösung gewaschen. Die organische Phase wurde über Natriumsulfat getrocknet, filtriert und das Filtrat eingeengt. Es wurden 2.18 g der Zielverbindung (99% d. Th.) erhalten. Die Zielverbindung wurde bei -18°C gelagert und ohne weitere Reinigung eingesetzt.
MS (Methode 12):
MS (ESpos): m/z = 269 (M+NH₄)⁺
¹H-NMR (400 MHz, DMSO-*d*₆) δ = 1.64 (s, 6 H), 5.13 (s, 2 H).

### Beispiel 17A

### 3-Brom-7-[(2,6-difluorbenzyl)oxy]-2,5-dimethylpyrazolo[1,5-a]pyridin

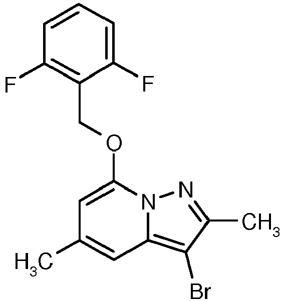

Eine Lösung von 5.0 g (15.1 mmol) 7-[(2,6-Difluorbenzyl)oxy]-2,5-dimethylpyrazolo[1,5-a]pyridin-3-carbonsäure aus Beispiel 4A in 60 ml DMF wurden mit 3.79 g (45.14 mmol) Natriumhydrogencarbonat versetzt. Eine Lösung von 2.81 g (15.80 mmol) *N*-Bromsuccinimid in 40 ml DMF wurde sehr langsam [2.6 ml/h] mittels Spritzenpumpe bei RT hinzugetropft. Anschließend wurden nochmals 134 mg (0.75 mmol) *N*-Bromsuccinimid in 2 ml DMF sehr langsam [2.6 ml/h] mittels Spritzenpumpe bei RT hinzugetropft. Die Reaktionslösung wurde mit Dichlormethan verdünnt und anschließend zweimal mit Wasser gewaschen. Die vereinigten wässrigen Phasen wurden zweimal mit Dichlormethan reextrahiert. Die vereinigten organischen Phasen wurden über Natriumsulfat getrocknet, filtriert und eingeengt. Der Rückstand wurde mit Wasser verrührt, der enthaltene Feststoff wurde abfiltriert und im Hochvakuum getrocknet. Es wurden 4.80 g der Titelverbindung (84% d. Th.) isoliert.
LC-MS (Methode 2): Rₜ = 1.25 min
MS (ESpos): m/z = 367 (M+H)⁺
¹H-NMR (400 MHz, DMSO-d₆): δ [ppm] = 2.30 (s, 3H), 2.40 (s, 3H), 5.43 (s, 2H), 6.49 (s, 1H), 6.92 (s, 1H), 7.20 - 7.30 (m, 2H), 7.57 - 7.67 (m, 1H).

### Beispiel 18A

### 3-Brom-2,5-dimethyl-7-[(2,3,6-trifluorbenzyl)oxy]pyrazolo[1,5-a]pyridin

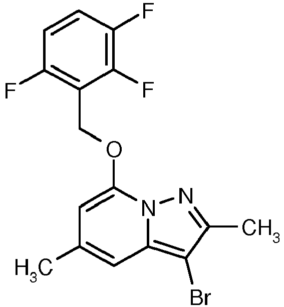

Eine Lösung von 103 mg (0.28 mmol) 2,5-Dimethyl-7-[(2,3,6-trifluorbenzyl)oxy]pyrazolo[1,5-a]pyridin-3-carbonsäure aus Beispiel 10A in 1.1 ml DMF wurden mit 70 mg (0.84 mmol) Natriumhydrogencarbonat versetzt. Eine Lösung von 52 mg (0.29 mmol) *N*-Bromsuccinimid in 0.75 ml DMF wurde sehr langsam [2.6 ml/h] mittels Spritzenpumpe bei RT hinzugetropft. Die Reaktionslösung wurde mit Dichlormethan verdünnt und anschließend zweimal mit Wasser gewaschen. Die vereinigten wässrigen Phasen wurden zweimal mit Dichlormethan reextrahiert. Die vereinigten organischen Phasen wurden über Natriumsulfat getrocknet, filtriert und eingeengt. Der Rückstand wurde mit Wasser verrührt, der enthaltene Feststoff wurde abfiltriert und im Hochvakuum getrocknet. Es wurden 65 mg der Titelverbindung (43% d. Th., Reinheit 71%) isoliert.
LC-MS (Methode 2): Rₜ = 1.29 min
MS (ESpos): m/z = 385 (M+H)⁺

### Beispiel 19A

### 3-Brom-2-cyclopropyl-7-[(2,6-difluorbenzyl)oxy]-5-methylpyrazolo[1,5-a]pyridin

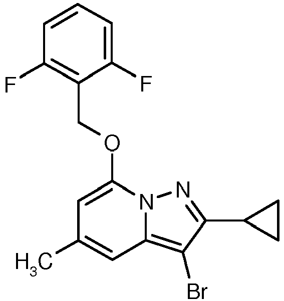

Eine Lösung von 3.0 g (6.2 mmol; Reinheit 74%) 2-Cyclopropyl-7-[(2,6-difluorbenzyl)oxy]-5-methylpyrazolo[1,5-a]pyridin-3-carbonsäure aus Beispiel 12A in 28.4 ml DMF wurden mit 1.79 g (21.35 mmol) Natriumhydrogencarbonat versetzt. Eine Lösung von 1.10 g (6.2 mmol) *N-*Bromsuccinimid in 19 ml DMF wurde sehr langsam [2.6 ml/h] mittels Spritzenpumpe bei RT hinzugetropft. Die Reaktionslösung wurde mit Dichlormethan verdünnt und anschließend zweimal mit Wasser gewaschen. Die vereinigten wässrigen Phasen wurden zweimal mit Dichlormethan reextrahiert. Die vereinigten organischen Phasen wurden über Natriumsulfat getrocknet, filtriert und eingeengt. Der Rückstand wurde mit Wasser verrührt, der enthaltene Feststoff wurde abfiltriert und im Hochvakuum getrocknet. Es wurden 2.70 g der Titelverbindung (98% d. Th., Reinheit 90%) isoliert.
LC-MS (Methode 15): Rₜ = 1.64 min
MS (ESpos): m/z = 393 (M+H)⁺

### Beispiel 20A

### 3-Brom-8-[(2,6-difluorbenzyl)oxy]-2,6-dimethylimidazo[1,2-a]pyrazin

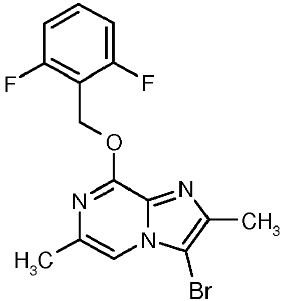

Eine Lösung von 120 mg (0.32 mmol, Reinheit 90%) 8-[(2,6-Difluorbenzyl)oxy]-2,6-dimethylimidazo[1,2-a]pyrazin-3-carbonsäure aus Beispiel 15A in 1.4 ml DMF wurden mit 91 mg (1.08 mmol) Natriumhydrogencarbonat versetzt. Eine Lösung von 57 mg (0.32 mmol) *N-*Bromsuccinimid in 1.0 ml DMF wurde innerhalb von 40 min bei RT hinzugetropft und das Gemisch wurde 5 min bei RT weitergerührt. Die Reaktionslösung wurde mit Dichlormethan verdünnt und anschließend zweimal mit Wasser gewaschen. Die vereinigten wässrigen Phasen wurden zweimal mit Dichlormethan reextrahiert. Die vereinigten organischen Phasen wurden eingeengt. Der Rückstand wurde mit Wasser verrührt, der enthaltene Feststoff wurde abfiltriert und im Hochvakuum getrocknet. Es wurden 118 mg der Titelverbindung (98% d. Th.) isoliert.
LC-MS (Methode 2): Rₜ = 1.21 min
MS (ESpos): m/z = 368 (M+H)⁺
¹H-NMR (400 MHz, DMSO-d₆): δ [ppm] = 2.30 (s, 3H), 2.39 (s, 3H), 5.55 (s, 2H), 7.17 - 7.24 (m, 2H), 7.52 - 7.62 (m, 1H), 7.84 (s, 1H).

### Beispiel 21A

### 7-[(2,6-Difluorbenzyl)oxy]-2,5-dimethyl-3-[1-(2-methyl-2-nitropropyl)-1H-pyrazol-4-yl]pyrazolo[1,5-a]pyridin

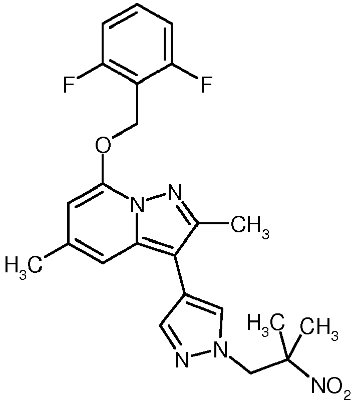

40 mg (0.11 mmol) 7-[(2,6-Difluorbenzyl)oxy]-2,5-dimethyl-3-(1H-pyrazol-4-yl)pyrazolo[1,5-a]pyridin aus Beispiel 1 wurden in 0.65 ml DMF vorgelegt, mit 41 mg (0.13 mmol) Cäsiumcarbonat sowie mit 89 mg (0.32 mmol) 2-Methyl-2-nitropropyltrifluormethansulfonat Beispiel 16A versetzt und 2 h bei RT gerührt. Anschließend wurden nochmals 89 mg (0.32 mmol) 2-Methyl-2-nitropropyltrifluormethansulfonat hinzugegeben und das Gemisch wurde 1 h bei RT gerührt. Es wurden nochmals 21 mg (0.06 mmol) Cäsiumcarbonat sowie mit 89 mg (0.32 mmol) 2-Methyl-2-nitropropyltrifluormethansulfonat hinzugegeben und die Mischung wurde über Nacht bei RT gerührt. Das Reaktionsgemisch wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Es wurden 28 mg der Zielverbindung (58% d. Th.) erhalten.
LC-MS (Methode 2): Rₜ = 1.15 min
MS (ESpos): m/z = 456 (M+H)⁺

### Beispiel 22A

### Ethyl-7-(cyclohexylmethoxy)-2,5-dimethylpyrazolo[1,5-a]pyridin-3-carboxylat

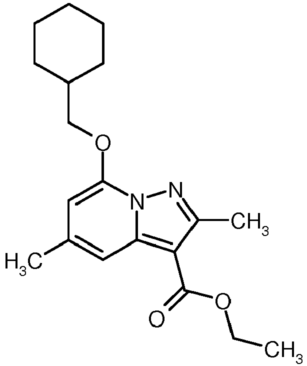

0.5 g (2.13 mmol) Ethyl-7-hydroxy-2,5-dimethylpyrazolo[1,5-a]pyridin-3-carboxylat aus Beispiel 8A, 416 mg (2.35 mmol) (Brommethyl)cyclohexan und 1.53 g (4.70 mmol) Cäsiumcarbonat wurden in 31 ml DMF vorgelegt und über Nacht bei 100°C gerührt. Das Reaktionsgemisch wurde auf 260 ml Wasser gegossen. Der Niederschlag wurde abfiltriert. Es wurden 196 mg der der Zielverbindung (28% d. Th.) erhalten. Die wässrige Phase wurde dreimal mit Essigsäureethylester extrahiert. Die vereinigten organischen Phasen wurden über Natriumsulfat getrocknet, filtriert und eingeengt. Es wurden nochmals 425 mg der Zielverbindung (55% d. Th.; Reinheit 92%) erhalten.
LC-MS (Methode 2): Rₜ = 1.33 min
MS (ESpos): m/z = 331 (M+H)⁺

### Beispiel 23A

### 7-(Cyclohexylmethoxy)-2,5-dimethylpyrazolo[1,5-a]pyridin-3-carbonsäure

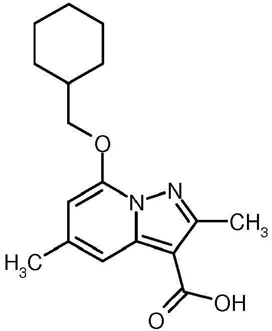

425 mg (1.18 mmol; Reinheit 92%) Ethyl-7-(cyclohexylmethoxy)-2,5-dimethylpyrazolo[1,5-a]pyridin-3-carboxylat aus Beispiel 22A wurden in 12.3 ml Dioxan vorgelegt und auf 90°C erhitzt.

Es wurden 9.47 ml (9.47 mmol) IN wässrige Natronlauge hinzugegeben und die Reaktionsmischung wurde 8 h bei 90°C gerührt. Die Reaktionslösung wurde abgekühlt und mit 2N wässriger Salzsäure auf pH 2 eingestellt. Das Gemisch wurde 30 min bei RT gerührt. Die Suspension wurde filtriert, der abfiltrierte Feststoff wurde mit wenig Wasser gewaschen und im Hochvakuum getrocknet. Der Feststoff wurde mit 10 ml Acetonitril versetzt, ausgerührt, abfiltriert und im Hochvakuum getrocknet. Es wurden 178 mg der Zielverbindung (50% d. Th.) erhalten.
LC-MS (Methode 2): Rₜ = 1.08 min
MS (ESpos): m/z = 303 (M+H)⁺

### Beispiel 24A

### 3-Brom-7-(cyclohexylmethoxy)-2,5-dimethylpyrazolo[1,5-a]pyridin

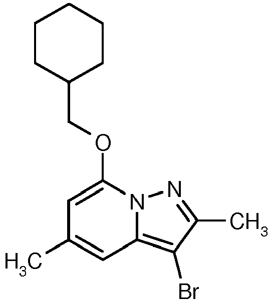

Eine Mischung von 178 mg (0.59 mmol) 7-(Cyclohexylmethoxy)-2,5-dimethylpyrazolo[1,5-a]pyridin-3-carbonsäure aus Beispiel 23A in 2.34 ml DMF wurde mit 148 mg (1.77 mmol) Natriumhydrogencarbonat versetzt. Eine Lösung von 105 mg (0.59 mmol) *N*-Bromsuccinimid in 1.56 ml DMF wurde sehr langsam [2.6 ml/h] mittels Spritzenpumpe bei RT hinzugetropft. Dann wurde nochmals eine Lösung von 5.3 mg (0.029 mmol) *N*-Bromsuccinimid in 77 µl DMF sehr langsam innerhalb von 75 min zur Reaktionslösung hinzugegeben. Es wurde 30 min bei RT gerührt, mit Dichlormethan verdünnt und anschließend zweimal mit Wasser gewaschen. Die vereinigten wässrigen Phasen wurden zweimal mit Dichlormethan reextrahiert. Die vereinigten organischen Phasen wurden über Natriumsulfat getrocknet, filtriert und einrotiert. Der Rückstand wurde mit Wasser verrührt, der enthaltene Feststoff wurde abfiltriert und im Hochvakuum getrocknet. Es wurden 179 mg der Titelverbindung (78% d. Th., Reinheit 87%) isoliert.
LC-MS (Methode 2): Rₜ = 1.47 min
MS (ESpos): m/z = 337 (M+H)⁺

### Beispiel 25A

### 7-[(2,6-Difluorbenzyl)oxy]-2,5-dimethylpyrazolo[1,5-a]pyridin-3-carboxamid

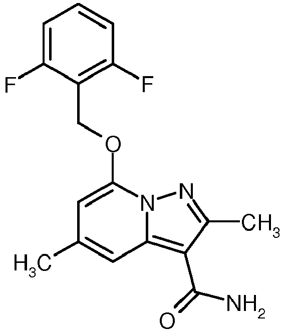

Unter Argon wurden 0.80 g (2.19 mmol; Reinheit 91%) 7-[(2,6-Difluorbenzyl)oxy]-2,5-dimethylpyrazolo[1,5-a]pyridin-3-carbonsäure aus Beispiel 4A in 24 ml DMF/Dichlorethan (1/1) vorgelegt und bei RT nacheinander mit 546 mg (2.85 mmol) 1-(3-Dimethylaminopropyl)-3-ethylcarbodiimidhydrochlorid sowie 436 mg (2.85 mmol) 1-Hydroxy-1H-benzotriazol Hydrat (HOBT) versetzt und 10 min bei RT gerührt. Dann wurden 586 mg (10.95 mmol) Ammoniumchlorid und 2.67 ml (15.34 mmol) *N,N*-Diisopropylethylamin zugegeben und das Gemisch wurde 10 min bei RT und 10 min bei 40°C gerührt. Anschließend wurden nochmals 126 mg (0.66 mmol) 1-(3-Dimethylaminopropyl)-3-ethylcarbodiimidhydrochlorid sowie 101 mg (0.66 mmol) 1-Hydroxy-1H-benzotriazol Hydrat (HOBT) hinzugegeben und das Gemisch wurde 30 min bei 40°C gerührt. Es wurde eingedampft, der Rückstand mit Wasser versetzt und 1 h gerührt. Der entstandene Feststoff wurde im Vakuum getrocknet. Es wurden 721 mg (84% d. Th.; Reinheit 85%) der Titelverbindung erhalten und ohne Reinigung weiter umgesetzt.
LC-MS (Methode 15): Rₜ = 1.05 min
MS (ESpos): m/z = 332 (M+H)⁺

### Beispiel 26A

### 7-[(2,6-Difluorbenzyl)oxy]-2,5-dimethylpyrazolo[1,5-a]pyridin-3-carbonitril

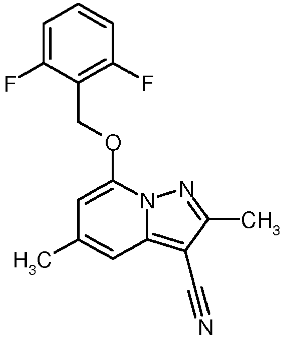

721 mg (1.85 mmol; Reinheit 85%) 7-[(2,6-Difluorbenzyl)oxy]-2,5-dimethylpyrazolo[1,5-a]pyridin-3-carboxamid aus Beispiel 25A wurden in 8.6 ml THF vorgelegt und mit 0.39 ml (4.81 mmol) Pyridin versetzt. Dann wurden 0.68 ml (4.81 mmol) Trifluoressigsäureanhydrid zugetropft und das Gemisch wurde 5 h bei RT gerührt. Anschließend wurde das Gemisch auf Wasser gegeben und 30 min bei RT gerührt. Der entstandene Feststoff wurde filtriert, mit Wasser gewaschen und im Vakuum getrocknet. Es wurden 605 mg (87% d. Th.; Reinheit 83%) der Titelverbindung erhalten.
LC-MS (Methode 2): Rₜ = 1.09 min
MS (ESpos): m/z = 314 (M+H)⁺

### Beispiel 27A

### 7-[(2,6-Difluorbenzyl)oxy]-N-hydroxy-2,5-dimethylpyrazolo[1,5-a]pyridin-3-carboximidamid

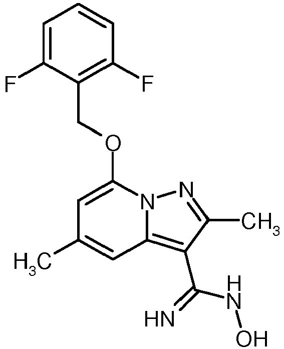

250 mg (0.66 mmol; Reinheit 83%) 7-[(2,6-Difluorbenzyl)oxy]-2,5-dimethylpyrazolo[1,5-a]pyridin-3-carbonitril aus Beispiel 26A wurden in 9.2 ml Ethanol suspendiert, mit 368 mg (5.3 mmol) Hydroxylamin Hydrochlorid sowie 0.74 ml (5.3 mmol) Triethylamin versetzt und über Nacht bei 80°C gerührt. Anschliessend wurde im Vakuum eingeengt, mit 8.9 Wasser und 0.45 ml Ethanol versetzt und 1 h gerührt. Der entstandene Feststoff wurde abfiltriert, mit 2.2 ml Wasser gewaschen und am Hochvakuum getrocknet. Der Rückstand wurde in Acetonitril aufgenommen, mit Wasser und Trifluoressigsäure versetzt und mittels präparativer HPLC gereinigt (RP18 Säule, Laufmittel: Acetonitril/Wasser-Gradient unter Zusatz von 0.1% TFA). Es wurden 73 mg (31% d. Th.) der Titelverbindung erhalten.
LC-MS (Methode 2): Rₜ = 0.62 min
MS (ESpos): m/z = 347 (M+H)⁺

### Beispiel 28A

### 7-[(2,6-Difluorbenzyl)oxy]-2,5-dimethylpyrazolo[1,5-a]pyridin-3-carboximidamid Acetat

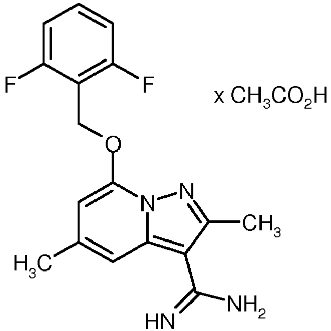

73 mg (0.21 mmol) 7-[(2,6-Difluorbenzyl)oxy]-N-hydroxy-2,5-dimethylpyrazolo[1,5-a]pyridin-3-carboximidamid aus Beispiel 27A wurden in 2.1 ml Essigsäure vorgelegt und mit 23.4 µl (0.25 mmol) Essigsäureanhydrid versetzt. Danach wurden 16 mg Palladium/Kohle (10%ig, feucht) zugegeben und es wurde 5 h bei Normaldruck hydriert. Es wurde über einen Millipore-Filter filtriert und mit Essigsäureethylester gewaschen. Nach Einengen wurde der Rückstand zweimal mit je 2 ml Toluol versetzt und im Vakuum eingeengt. Der Rückstand wurde im Hochvakuum getrocknet. Es wurden 65 mg (73% d. Th.; Reinheit 91%) der Titelverbindung erhalten.
LC-MS (Methode 2): Rₜ = 0.62 min
MS (ESpos): m/z = 331 (M-CH₃CO₂H+H)⁺

### Beispiel 29A

### 2-{7-[(2,6-Difluorbenzyl)oxy]-2,5-dimethylpyrazolo[1,5-a]pyridin-3-yl}-4-iod-5,5-dimethyl-5,7-dihydro-6H-pyrrolo[2,3-d]pyrimidin-6-on

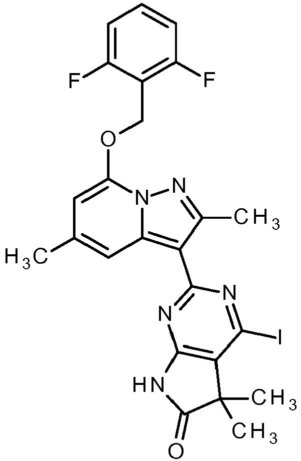

70 mg (0.12 mmol) 4-Amino-2-{7-[(2,6-difluorbenzyl)oxy]-2,5-dimethylpyrazolo[1,5-a]pyridin-3-yl}-5,5-dimethyl-5,7-dihydro-6H-pyrrolo[2,3-d]pyrimidin-6-on Trifluoracetat aus Beispiel 34 wurden unter Argon in 1.5 ml abs. Dioxan vorgelegt, bei RT nacheinander mit 59 µl (0.74 mmol) Diiodmethan, 121 mg (1.03 mmol) Isopentylnitrit und 200 mg 4A Molsieb versetzt und über Nacht bei 85°C gerührt. Die Reaktionsmischung wurde anschließend filtriert, der Rückstand (Molsieb) mit Essigsäureethylester gespült und das Lösungsmittel eingedampft. Anschließend wurde der Rückstand mit Acetonitril/Wasser verdünnt und mit wenig TFA versetzt. Die Lösung wurde mittels präparativer HPLC gereinigt (RP18 Säule, Laufmittel: Acetonitril/Wasser-Gradient unter Zusatz von 0.1% TFA). Die Produktfraktionen wurden eingedampft und mit gesättigter Natriumhydrogencarbonat-Lösung versetzt und dreimal mit Essigsäureethylester ausgeschüttelt. Das Lösungsmittel wurde einrotiert. Es wurden 23 mg (31% d. Th.) der Titelverbindung erhalten.
LC-MS (Methode 16): Rₜ = 2.48 min
MS (ESpos): m/z = 576 (M+H)⁺

### Ausführungsbeispiele

### Beispiel 1

### 7-[(2,6-Difluorbenzyl)oxy]-2,5-dimethyl-3-(1H-pyrazol-4-yl)pyrazolo[1,5-a]pyridin

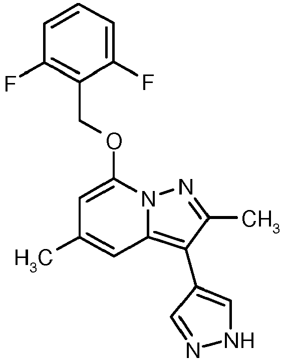

1.0 g (2.64 mmol) 3-Brom-7-[(2,6-difluorbenzyl)oxy]-2,5-dimethylpyrazolo[1,5-a]pyridin aus Beispiel 17A und 1.17 g (3.96 mmol) tert-Butyl-4-(4,4,5,5-tetramethyl-1,3,2-dioxaborolan-2-yl)-1H-pyrazol-1-carboxylat wurden in 32 ml abs. Acetonitril vorgelegt und mit Argon begast. Anschließend wurden 104 mg (0.13 mmol) Chloro(2-dicyclohexylphosphino-2',4',6'-triisopropyl-1,1'-biphenyl)[2-(2'-amino-1,1'-biphenyl)palladium (II) [CAS: 1310584-14-5] hinzugegeben und mit 15.9 ml (7.93 mmol) wässriger 0.5 M Kaliumphosphat-Lösung (sauerstofffrei) versetzt. Das Reaktionsgemisch wurde 12 h bei 60°C intensiv gerührt. Anschließend wurden nochmals 583 mg (1.98 mmol) tert-Butyl-4-(4,4,5,5-tetramethyl-1,3,2-dioxaborolan-2-yl)-1H-pyrazol-1-carboxylat und 104 mg (0.13 mmol) Chloro(2-dicyclohexylphosphino-2',4',6'-triisopropyl-1,1'-biphenyl)[2-(2'-amino-1,1'-biphenyl)palladium (II) [CAS: 1310584-14-5] hinzugegeben. Das Reaktionsgemisch wurde 5 h bei 60°C intensiv gerührt. Die Reaktionslösung wurde abgekühlt, mit Dichlormethan versetzt und dreimal mit Wasser gewaschen. Die vereinigten, wässrigen Phasen wurde zweimal mit Dichlormethan reextrahiert. Die vereinten organischen Phasen wurden über Natriumsulfat getrocknet, filtriert und eingeengt. Der Rückstand wurde in mehreren Portionen mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlormethan und wenig Methanol aufgenommen und zweimal mit wässriger, gesättigter Natriumhydrogencarbonat-Lösung gewaschen. Die vereinigten wässrigen Phasen wurden zweimal mit Dichlormethan reextrahiert. Die vereinigten organischen Phasen wurden über Natriumsulfat getrocknet, filtriert und eingeengt. Es wurden 231 mg (19% d. Th.) der Titelverbindung isoliert.
LC-MS (Methode 2): Rₜ = 0.87 min
MS (ESpos): m/z = 355 (M+H)⁺
¹H-NMR (500 MHz, DMSO-d₆): δ [ppm] = 2.34 - 2.40 (m, 6H), 5.43 (s, 2H), 6.38 (s, 1H), 7.13 (s, 1H), 7.22 - 7.30 (m, 2H), 7.57 - 7.67 (m, 1H), 7.76 (br. s, 1H), 7.98 (br. s, 1H), 12.98 (br. s, 1H).

In Analogie zu Beispiel 1 wurden die in Tabelle 1 gezeigten Beispielverbindungen hergestellt, indem 3-Brom-7-[(2,6-difluorbenzyl)oxy]-2,5-dimethylpyrazolo[1,5-a]pyridin aus Beispiel 17A mit den entsprechenden, kommerziell erhältlichen oder literaturbekannten Boronsäuren oder Boronsäureestern (1.5 - 2.5 Äquivalente), wässriger Kaliumphosphat-Lösung (3 Äquivalente) und Chloro(2-dicyclohexylphosphino-2',4',6'-triisopropyl-1,1'-biphenyl) [2-(2'-amino-1,1'-biphenyl)palladium (II) [CAS: 1310584-14-5] (0.05-0.1 Äquivalente) unter den beschriebenen Reaktionsbedingungen (Lösungsmittel: Acetonitril; Reaktionszeit: 4 - 24 h; Temperatur: 60°C) umgesetzt wurden.

Im Regelfall wurden zu Reaktionsbeginn 0.05 Äquivalente Chloro(2-dicyclohexylphosphino-2',4',6'-triisopropyl-1,1'-biphenyl)[2-(2'-amino-1,1'-biphenyl)palladium (II) [CAS: 1310584-14-5] und 1.5 Äquivalente Boronsäure oder Boronsäureester verwendet. Bei unvollständiger Umsetzung wurden ggf. nochmals 0.05 Äquivalente Chloro(2-dicyclohexylphosphino-2',4',6'-triisopropyl-1,1'-biphenyl)[2-(2'-amino-1,1'-biphenyl)palladium (II) [CAS: 1310584-14-5] und 1.0 Äquivalente Boronsäure oder Boronsäureester zum Reaktionsgemisch hinzugegeben.

### Beispielhafte Aufarbeitung der Reaktionsmischung:

Die Reaktionslösung wurde abgekühlt, mit Wasser und Dichlormethan (oder Essigsäureethylester) versetzt und dreimal mit Wasser gewaschen. Die vereinigten wässrigen Phasen wurde zweimal mit Dichlormethan (oder Essigsäureethylester) reextrahiert. Die vereinten organischen Phasen wurden über Natriumsulfat getrocknet, filtriert und eingeengt. Der Rückstand wurde mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlormethan und wenig Methanol aufgenommen und zweimal mit wässriger, gesättigter Natriumhydrogencarbonat-Lösung gewaschen. Die vereinigten wässrigen Phasen wurden zweimal mit Dichlormethan reextrahiert. Die vereinigten organischen Phasen wurden über Natriumsulfat getrocknet, filtriert und eingeengt.

Das Rohprodukt wurde gegebenenfalls zusätzlich mittels Dickschichtchromatographie gereinigt (Lösungsmittel: Dichlormethan/Methanol = 100/1 oder 50/1 oder 20/1 oder Dichlormethan/Cyclohexan = 10/1).

**Tabelle 1:**

| **Beispiel Nr.** | **IUPAC-Name (Ausbeute)** | **Analytischen Daten** |
|---|---|---|
| **2** | 1-(3-{7-[(2,6-Difluorbenzyl)oxy]-2,5-dimethylpyrazolo[1,5-a]pyridin-3-yl}phenyl)ethanon | LC-MS (Methode 2): Rₜ = 1.16 min |
| | 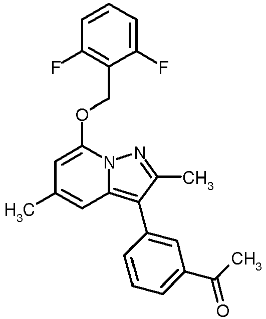 | MS (ESpos): m/z = 407 (M+H)⁺ |
| | Es wurde die Boronsäure eingesetzt. (62% d. Th.) | |
| **3** | N-(3-{7-[(2,6-Difluorbenzyl)oxy]-2,5-dimethylpyrazolo[1,5-a]pyridin-3-yl}phenyl)acetamid | LC-MS (Methode 2): Rₜ = 1.02 min |
| | 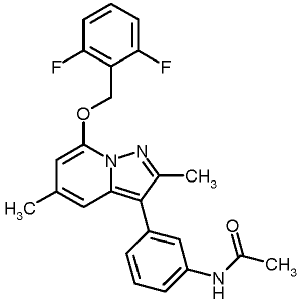 | MS (ESpos): m/z = 422 (M+H)⁺ |
| | Es wurde die Boronsäure eingesetzt. (30% d. Th.) | |
| **4** | 7-[(2,6-Difluorbenzyl)oxy]-2,5-dimethyl-3-[5-(morpholin-4-ylmethyl)-3-thienyl]pyrazolo[1,5-a]pyridin | LC-MS (Methode 2): Rₜ = 0.80 min |
| | 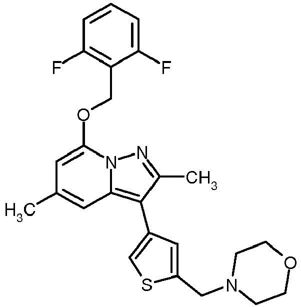 | MS (ESpos): m/z = 470 (M+H)⁺ |
| | | ¹H-NMR (400 MHz, DMSO-*d*₆) δ = 2.36 - 2.48 (m, 10H), 3.57 - 3.65 (m, 4H), 3.73 (s, 2H), 5.43 (s, 2H), 6.42 (s, 1H), 7.13 (s, 1H), 7.20 (s, 1H), 7.22 - 7.30 (m, 2H), 7.40 (s, 1H), 7.58 - 7.67 (m, 1H). |
| | Es wurde der Boronsäure-Pinakolester eingesetzt. (58% d. Th.) | |
| **5** | 7-[(2,6-Difluorbenzyl)oxy]-2,5-dimethyl-3-{1-[2-(morpholin-4-yl)ethyl]-1H-pyrazol-4-yl}pyrazolo[1,5-a]pyridin | LC-MS (Methode 2): Rₜ = 0.71 min |
| | 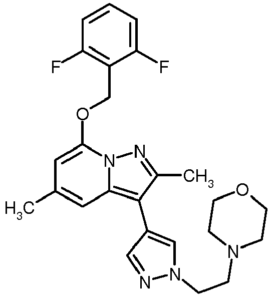 | MS (ESpos): m/z = 468 (M+H)⁺ |
| | | ¹H-NMR (400 MHz, DMSO-*d*₆) 5 = 2.36 - 2.48 (m, 10H), 2.70 - 2.82 (m, 2H), 3.53 - 3.64 (m, 4H), 4.24 - 4.34 (m, 2H), 5.43 (s, 2H), 6.38 (s, 1H), 7.13 (s, 1H), 7.22 - 7.30 (m, 2H), 7.58 - 7.67 (m, 1H), 7.70 (s, 1H 8.02 (s, 1H). |
| | Es wurde der Boronsäure-Pinakolester eingesetzt. (38% d. Th.) | |
| **6** | 3-(1-Benzyl-1H-pyrazol-4-yl)-7-[(2,6-difluorbenzyl)oxy]-2,5-dimethylpyrazolo[1,5-a]pyridin | LC-MS (Methode 2): Rₜ = 1.21 min |
| | 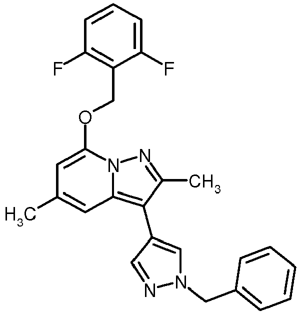 | MS (ESpos): m/z = 445 (M+H)⁺ |
| | Es wurde der Boronsäure-Pinakolester eingesetzt. (68% d. Th.) | |
| **7** | 7-[(2,6-Difluorbenzyl)oxy]-3-[3-(ethylsulfonyl)phenyl]-2,5-dimethylpyrazolo[1,5-a]pyridin | LC-MS (Methode 2): Rₜ = 1.14 min |
| | 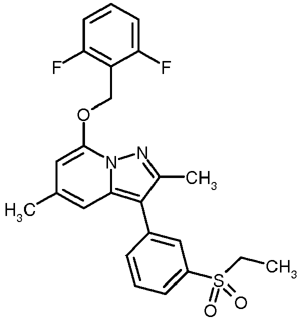 | MS (ESpos): m/z = 457 (M+H)⁺ |
| | Es wurde die Boronsäure eingesetzt. (65% d. Th.) | |
| **8** | 7-[(2,6-Difluorbenzyl)oxy]-3-[1-(4-fluorphenyl)-1H-pyrazol-4-yl]-2,5-dimethylpyrazolo[1,5-a]pyridin | LC-MS (Methode 2): Rₜ = 1.27 min |
| | 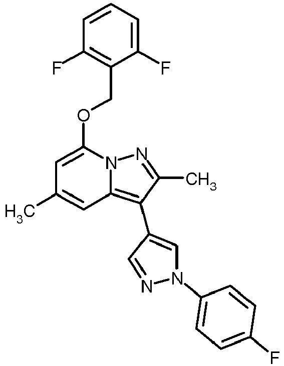 | MS (ESpos): m/z = 449 (M+H)⁺ |
| | Es wurde die Boronsäure eingesetzt. (18% d. Th.) | |
| **9** | 3-(6-Chlor-5-methylpyridin-3-yl)-7-[(2,6-difluorbenzyl)oxy]-2,5-dimethylpyrazolo[1,5-a]pyridin | LC-MS (Methode 2): Rₜ = 1.30 min |
| | 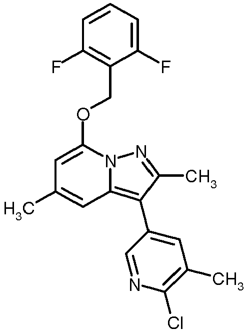 | MS (ESpos): m/z = 414 (M+H)⁺ |
| | Es wurde die Boronsäure eingesetzt. (4% d. Th.) | |
| **10** | N-(3-{7-[(2,6-Difluorbenzyl)oxy]-2,5-dimethylpyrazolo[1,5-a]pyridin-3-yl}benzyl)-N-methylethanamin | LC-MS (Methode 2): Rₜ = 0.81 min |
| | 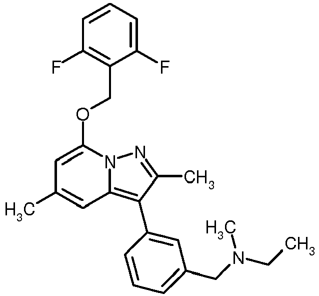 | MS (ESpos): m/z = 436 (M+H)⁺ |
| | Es wurde die Boronsäure eingesetzt. (36% d. Th.) | |
| **11** | 7-[(2,6-Difluorbenzyl)oxy]-2,5-dimethyl-3-[3-(pyrrolidin-1-ylmethyl)phenyl]pyrazolo[1,5-a]pyridin | LC-MS (Methode 2): Rₜ = 0.81 min |
| | 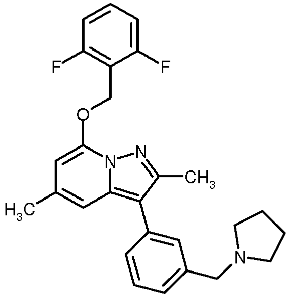 | MS (ESpos): m/z = 448 (M+H)⁺ |
| | Es wurde die Boronsäure eingesetzt. (34% d. Th.) | |
| **12** | 3-{7-[(2,6-Difluorbenzyl)oxy]-2,5-dimethylpyrazolo[1,5-a]pyridin-3-yl}benzamid | LC-MS (Methode 2): Rₜ = 0.95 min |
| | 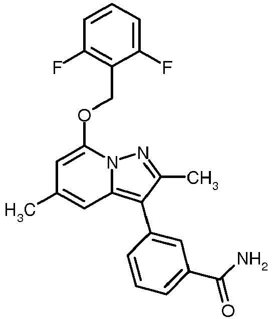 | MS (ESpos): m/z = 408 (M+H)⁺ |
| | Es wurde der Boronsäure-Pinakolester eingesetzt. (49% d. Th.) | |
| **13** | N-(3-{7-[(2,6-Difluorbenzyl)oxy]-2,5-dimethylpyrazolo[1,5-a]pyridin-3-yl}phenyl)methansulfonamid | LC-MS (Methode 2): Rₜ = 1.10 min |
| | 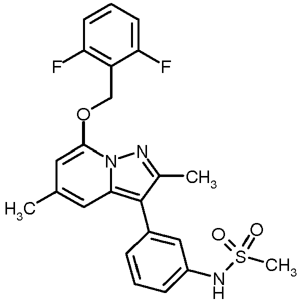 | MS (ESpos): m/z = 458 (M+H)⁺ |
| | Es wurde der Boronsäure-Pinakolester eingesetzt. (24% d. Th.) | |
| **14** | 7-[(2,6-Difluorbenzyl)oxy]-2,5-dimethyl-3-[3-(pyrrolidin-1-yl)phenyl]pyrazolo[1,5-a]pyridin | LC-MS (Methode 2): Rₜ = 1.51 min |
| | 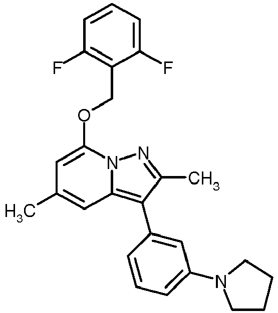 | MS (ESpos): m/z = 434 (M+H)^{+ 1}H-NMR (500 MHz, DMSO-*d*₆) δ = 1.94 - 2.02 (m, 4H), 2.35 - 2.42 (m, 6H), 3.23 - 3.33 (m, 4H, überlagert mit Lösungsmittelpeak), 5.43 (s, 2H), 6.38 (s, 1H), 6.49 (d, 1H), 6.53 (s, 1H), 6.65 (d, |
| | Es wurde die Boronsäure eingesetzt. (48% d. Th.) | 1H), 7.08 (s, 1H), 7.20 - 7.30 (m, 3H), 7.58 - 7.67 (m, 1H). |
| **15** | 7-[(2,6-Difluorbenzyl)oxy]-2,5-dimethyl-3-[2-(piperazin-1-yl)pyridin-4-yl]pyrazolo[1,5-a]pyridin | LC-MS (Methode 2): Rₜ = 0.72 min |
| | 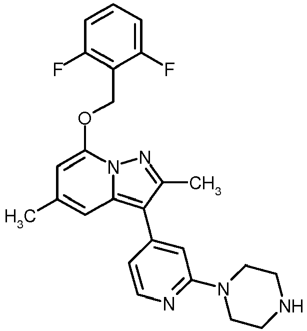 | MS (ESpos): m/z = 450 (M+H)⁺ |
| | Es wurde der Boronsäure-Pinakolester eingesetzt. (33% d. Th.) | |

### Beispiel 16

### 1-(4-{7-[(2,6-Difluorbenzyl)oxy]-2,5-dimethylpyrazolo[1,5-a]pyridin-3-yl}-1H-pyrazol-1-yl)-2-methylpropan-2-amin

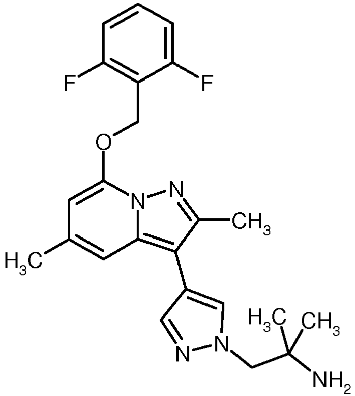

35 mg (0.08 mmol) 7-[(2,6-Difluorbenzyl)oxy]-2,5-dimethyl-3-[1-(2-methyl-2-nitropropyl)-1H-pyrazol-4-yl]pyrazolo[1,5-a]pyridin aus Beispiel 21A in 1 ml Ethanol wurden mit ca. 102 mg Raney-Nickel (50%ige wässrige Aufschlämmung) versetzt und über Nacht unter Normaldruck bei Raumtemperatur hydriert. Das Reaktionsgemisch wurde über Celite filtriert und mit Dichlormethan und 2 N Ammoniak-Lösung in Methanol gewaschen. Das Filtrat wurde eingeengt und der Rückstand mittels Dickschichtchromatographie gereinigt (Laufmittel: Dichlormethan/2 N Ammoniak-Lösung in Methanol (45/1)). Es wurden 20 mg der Zielverbindung (60% d. Th.) erhalten.
LC-MS (Methode 2): Rₜ = 0.72 min
MS (ESpos): m/z = 426 (M+H)⁺
¹H-NMR (500 MHz, DMSO-*d*₆) δ = 1.02 (s, 6 H), 1.69 (br. s, 2 H), 2.36 - 2.41 (m, 6 H), 4.02 (s, 2 H), 5.42 (s, 2 H), 6.38 (s, 1 H), 7.12 - 7.15 (m, 1 H), 7.23 - 7.30 (m, 2 H), 7.59 - 7.66 (m, 1 H), 7.73 (s, 1 H), 7.97 (s, 1 H).

### Beispiel 17

### 7-[(2,6-Difluorbenzyl)oxy]-3-(2,5-difluorpyridin-4-yl)-2,5-dimethylpyrazolo[1,5-a]pyridin

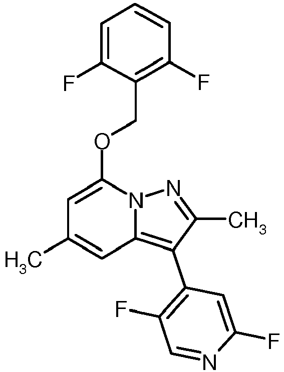

75 mg (0.20 mmol) 3-Brom-7-[(2,6-difluorbenzyl)oxy]-2,5-dimethylpyrazolo[1,5-a]pyridin (Beispiel 17A) wurden in einem Gemisch aus 1.44 ml Ethanol, 0.72 ml Wasser und 0.72 ml Toluol unter Argon nacheinander mit 79 mg (0.50 mmol) (2,5-Difluorpyridin-4-yl)borsäure, 126 mg (0.59 mmol) Kaliumphosphat und 10 mg (0.02 mmol) Bis(tri-tert-butyl-phosphin)palladium(0) versetzt. Die Suspension wurde mit Argon entgast und dann 30 min bei 120°C gerührt. Nach beendeter Reaktion wurde das Reaktionsgemisch eingeengt, der Rückstand in Essigsäureethylester/Wasser aufgenommen und ausgeschüttelt. Die wässrige Phase zweimal mit Essigsäureethylester extrahiert.

Die vereinten organischen Phasen wurden über Natriumsulfat getrocknet, filtriert, eingeengt und im Hochvakuum getrocknet. Der Rückstand wurde mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlormethan aufgenommen und zweimal mit wässriger, gesättigter Natriumhydrogencarbonat-Lösung gewaschen. Die vereinigten wässrigen Phasen wurden zweimal mit Dichlormethan reextrahiert. Die vereinigten organischen Phasen wurden über Natriumsulfat getrocknet, filtriert und eingeengt. Es wurden 75 mg der Zielverbindung (91% d. Th.) erhalten.
LC-MS (Methode 2): Rₜ = 1.19 min
MS (ESpos): m/z = 402 (M+H)⁺

### Beispiel 18

### Ethyl-5-{7-[(2,6-difluorbenzyl)oxy]-2,5-dimethylpyrazolo[1,5-a]pyridin-3-yl}nicotinat

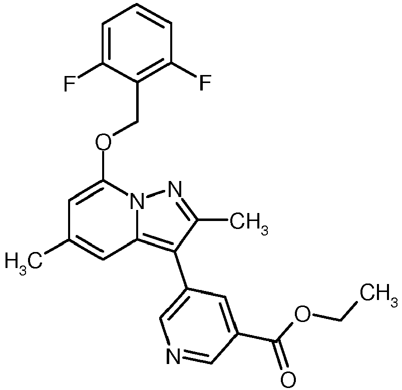

53 mg (0.14 mmol) 3-Brom-7-[(2,6-difluorbenzyl)oxy]-2,5-dimethylpyrazolo[1,5-a]pyridin (Beispiel 17A) wurden in einem Gemisch aus 0.26 ml Ethanol, 0.53 ml Wasser und 0.53 ml Toluol unter Argon nacheinander mit 60 mg (0.22 mmol) Ethyl-5-(4,4,5,5-tetramethyl-1,3,2-dioxaborolan-2-yl)nicotinat, 92 mg (0.43 mmol) Kaliumphosphat und 7 mg (0.014 mmol) Bis(tri-tert-butyl-phosphin)palladium(0) versetzt. Die Suspension wurde mit Argon entgast und dann 30 min bei 120°C gerührt. Nach beendeter Reaktion wurde das Reaktionsgemisch eingeengt, der Rückstand in Essigsäureethylester/Wasser aufgenommen und ausgeschüttelt. Die wässrige Phase wurde zweimal mit Essigsäureethylester extrahiert. Die vereinten organischen Phasen wurden über Natriumsulfat getrocknet, filtriert, eingeengt und im Hochvakuum getrocknet. Der Rückstand wurde mittels präparativer HPLC gereinigt (RP18 Säule, Laufmittel: Acetonitril/Wasser-Gradient unter Zusatz von 0.1% TFA). Die produkthaltigen Fraktionen wurden eingeengt und nochmals mittels Dickschichtchromatographie gereinigt (Laufmittel: Dichlormethan/Methanol = 100/1). Es wurden 34 mg der Zielverbindung (52% d. Th.) erhalten.
LC-MS (Methode 2): Rₜ = 1.18 min
MS (ESpos): m/z = 438 (M+H)⁺

### Beispiel 19

### 8-[(2,6-Difluorbenzyl)oxy]-2,6-dimethyl-3-(1H-pyrazol-4-yl)imidazo[1,2-a]pyrazin Trifluoracetat

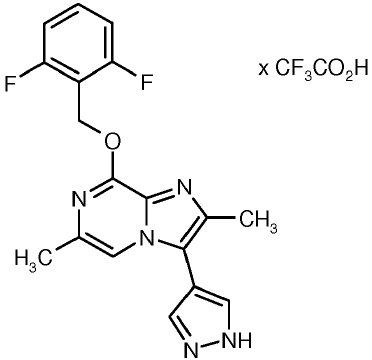

40 mg (0.11 mmol) 3-Brom-8-[(2,6-difluorbenzyl)oxy]-2,6-dimethylimidazo[1,2-a]pyrazin aus Beispiel 20A und 48 mg (0.16 mmol) tert-Butyl-4-(4,4,5,5-tetramethyl-1,3,2-dioxaborolan-2-yl)-1H-pyrazol-1-carboxylat wurden in 1.3 ml abs. Acetonitril vorgelegt und mit Argon begast. Anschließend wurden 4.3 mg (0.01 mmol) Chloro(2-dicyclohexylphosphino-2',4',6'-triisopropyl-1,1'-biphenyl)[2-(2'-amino-1,1'-biphenyl)palladium (II) [CAS: 1310584-14-5] hinzugegeben und mit 0.65 ml (0.33 mmol) wässriger 0.5 M Kaliumphosphat-Lösung (sauerstofffrei) versetzt. Das Reaktionsgemisch wurde über Nacht bei 60°C gerührt. Die Reaktionslösung wurde abgekühlt, mit TFA versetzt und mittels präparativer HPLC gereinigt (RP18 Säule, Laufmittel: Acetonitril/Wasser-Gradient unter Zusatz von 0.1% TFA). Es wurden 2.4 mg (4% d. Th., Reinheit 90%) der Titelverbindung isoliert.
LC-MS (Methode 2): Rₜ = 0.79 min
MS (ESpos): m/z = 356 (M-TFA+H)⁺
¹H-NMR (400 MHz, DMSO-d₆): δ [ppm] = 2.34 - 2.40 (m, 6H), 5.57 (s, 2H), 7.17 - 7.24 (m, 2H), 7.52 - 7.62 (m, 1H), 7.86 (s, 1H), 7.95 - 8.15 (m, 2H).

### Beispiel 20

### 6-{8-[(2,6-Difluorbenzyl)oxy]-2,6-dimethylimidazo[1,2-a]pyrazin-3-yl}-1,3,5-triazin-2,4-diamin Trifluoracetat

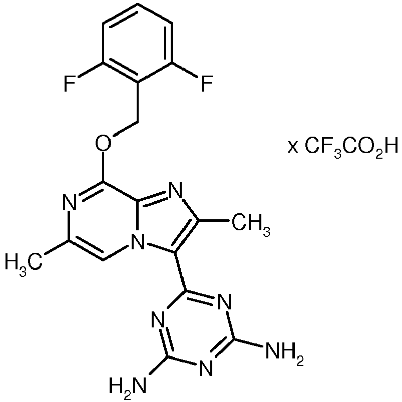

40 mg (0.29 mmol) Imidodikohlenstoffimiddiamid-Hydrochlorid [Biguanid-Hydrochlorid] wurden unter Argon in 0.87 ml abs. Methanol vorgelegt, mit 138 mg (0.15 ml, 0.64 mmol) Natriummethylat (25%ig in Methanol) versetzt und 30 min bei 50°C gerührt. Anschließend wurden 70 mg (0.19 mmol) Ethyl-8-[(2,6-difluorbenzyl)oxy]-2,6-dimethylimidazo[1,2-a]pyrazin-3-carboxylat aus Beispiel 14A zugegeben und über Nacht unter Rückfluss gerührt. Nach dem Abkühlen wurde das Gemisch mit Wasser und TFA versetzt und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, in Dichlormethan gelöst und mit gesättigter, wässriger Natriumhydrogencarbonatlösung gewaschen. Die wässrige Phase wurde zweimal mit Dichlormethan unter Zusatz von etwas Methanol extrahiert. Die vereinten organischen Phasen wurden über Natriumsulfat getrocknet, filtriert, das Filtrat eingeengt und der Rückstand mit Wasser ausgerührt. Der Niederschlag wurde abgesaugt und im Hochvakuum getrocknet. Es wurden 3.7 mg der Zielverbindung (3.7% d. Th.) erhalten.
LC-MS (Methode 2): Rₜ = 0.85 min
MS (ESpos): m/z = 399 (M-TFA+H)⁺

### Beispiel 21

### Methyl-3-(4-{7-[(2,6-difluorbenzyl)oxy]-2,5-dimethylpyrazolo[1,5-a]pyridin-3-yl}-1H-pyrazol-1-yl)propanoat Trifluoracetat

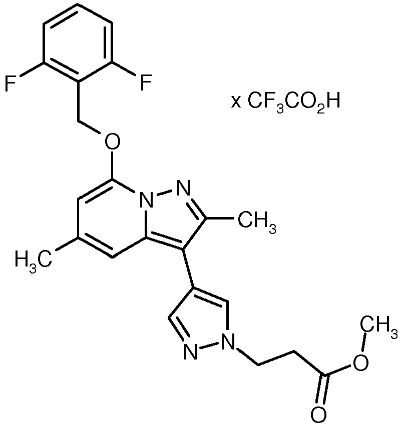

90 mg (0.24 mmol) 7-[(2,6-Difluorbenzyl)oxy]-2,5-dimethyl-3-(1H-pyrazol-4-yl)pyrazolo[1,5-a]pyridin aus Beispiel 1 in 1.5 ml DMF wurden mit 202 mg (0.62 mmol) Cäsiumcarbonat, 4 mg (0.02 mmol) Kaliumiodid und 52 mg (0.31 mmol) Methyl-3-brompropanoat versetzt und 2 h bei 60°C gerührt. Nach dem Abkühlen wurde das Gemisch mittels präparativer HPLC gereinigt (RP18 Säule, Laufmittel: Acetonitril/Wasser-Gradient unter Zusatz von 0.1% TFA). Es wurden 71 mg der Zielverbindung (53% d. Th.) erhalten.
LC-MS (Methode 2): Rₜ = 1.04 min
MS (ESpos): m/z = 441 (M-TFA+H)⁺
¹H-NMR (400 MHz, DMSO-*d*₆) δ = 2.35 - 2.43 (m, 6 H), 2.94 (t, 2 H), 3.62 (s, 3 H), 4.41 (t, 2 H), 5.42 (s, 2 H), 6.38 (s, 1 H), 7.14 (s, 1 H), 7.26 (t, 2 H), 7.57 - 7.67 (m, 1 H), 7.72 (s, 1 H), 8.00 (s, 1 H).

### Beispiel 22

### 3-(4-{7-[(2,6-Difluorbenzyl)oxy]-2,5-dimethylpyrazolo[1,5-a]pyridin-3-yl}-1H-pyrazol-1-yl)propansäure Trifluoracetat

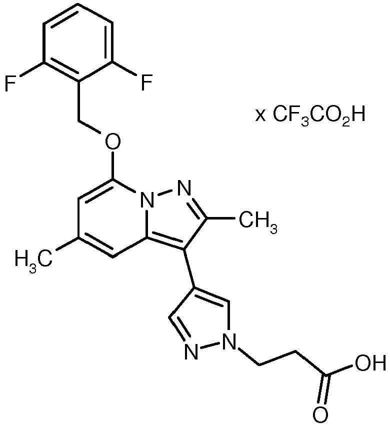

60 mg (0.11 mmol) Methyl-3-(4-{7-[(2,6-difluorbenzyl)oxy]-2,5-dimethylpyrazolo[1,5-a]pyridin-3-yl}-1H-pyrazol-1-yl)propanoat Trifluoracetat aus Beispiel 21 in 2.30 ml THF/Methanol (5/1) wurden mit 0.32 ml (0.32 mmol) 1 N wässriger Lithiumhydroxidlösung versetzt und 2 h bei Raumtemperatur gerührt. Die Reaktionslösung wurde mit 0.34 ml 1 N wässriger Salzsäure versetzt und anschließend mittels präparativer HPLC gereinigt (RP18 Säule, Laufmittel: Acetonitril/Wasser-Gradient unter Zusatz von 0.1% TFA). Es wurden 52 mg der Zielverbindung (87% d. Th.) erhalten.
LC-MS (Methode 2): Rₜ = 0.92 min
MS (ESpos): m/z = 427 (M-TFA+H)⁺
¹H-NMR (400 MHz, DMSO-*d*₆) δ = 2.33 - 2.42 (m, 6 H), 2.86 (t, 2 H), 4.37 (t, 2 H), 5.41 (s, 2 H), 6.38 (s, 1 H), 7.14 (s, 1 H), 7.27 (t, 2 H), 7.57 - 7.67 (m, 1 H), 7.72 (s, 1 H), 8.00 (s, 1 H), 12.39 (br. s, 1 H).

### Beispiel 23

### N-Cyclopropyl-3-(4-{7-[(2,6-difluorbenzyl)oxy]-2,5-dimethylpyrazolo[1,5-a]pyridin-3-yl}-1H-pyrazol-1-yl)propanamid

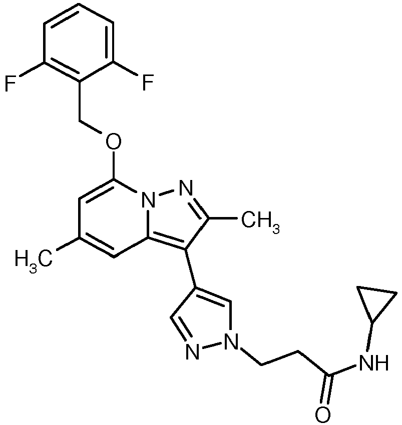

25 mg (0.05 mmol) 3-(4-{7-[(2,6-Difluorbenzyl)oxy]-2,5-dimethylpyrazolo[1,5-a]pyridin-3-yl}-1H-pyrazol-1-yl)propansäure Trifluoracetat aus Beispiel 22, 23 mg (0.06 mmol) HATU und 0.04 ml (0.23 mmol) *N,N*-Diisopropylethylamin in 0.16 ml DMF wurden 20 min bei RT gerührt, mit 6 µl (0.09 mmol) Cyclopropylamin versetzt und 1.5 h bei RT gerührt. Die Reaktionslösung wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, in Dichlormethan gelöst und zweimal mit gesättigter, wässriger Natriumhydrogencarbonatlösung gewaschen. Die wässrige Phase wurde zweimal mit Dichlormethan extrahiert. Die vereinten organischen Phasen wurden über Natriumsulfat getrocknet, filtriert und das Filtrat eingeengt. Es wurden 20 mg der Zielverbindung (91% d. Th.) erhalten.
LC-MS (Methode 2): Rₜ = 0.93 min
MS (ESpos): m/z = 466 (M+H)⁺
¹H-NMR (500 MHz, DMSO-*d*₆) δ = 0.30 - 0.36 (m, 2 H), 0.57 - 0.61 (m, 2 H), 2.34 - 2.40 (m, 6 H), 2.58 - 2.67 (m, 3 H), 4.38 (t, 2 H), 5.42 (s, 2 H), 6.38 (s, 1 H), 7.12 (s, 1 H), 7.25 (t, 2 H), 7.58 - 7.66 (m, 1 H), 7.70 (s, 1 H), 7.90 (s, 1 H), 8.01 (s, 1 H).

### Beispiel 24

### 2-(4-{7-[(2,6-Difluorbenzyl)oxy]-2,5-dimethylpyrazolo[1,5-a]pyridin-3-yl}-1H-pyrazol-1-yl)ethanol Trifluoracetat

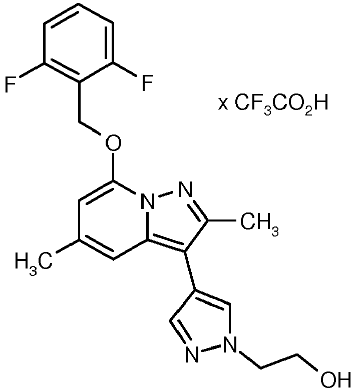

45 mg (0.12 mmol) 7-[(2,6-Difluorbenzyl)oxy]-2,5-dimethyl-3-(1H-pyrazol-4-yl)pyrazolo[1,5-a]pyridin aus Beispiel 1 in 0.63 DMF wurden mit 98 mg (0.30 mmol) Cäsiumcarbonat, 2 mg (0.01 mmol) Kaliumiodid und 0.012 ml (0.15 mmol) Iodethanol versetzt und 1.5 h bei 70°C gerührt. Anschließend wurden nochmals 0.018 ml (0.23 mmol) Iodethanol hinzugegeben und 4.5 h bei 70°C gerührt. Nach dem Abkühlen wurde das Gemisch mit Wasser versetzt und dreimal mit Essigsäureethylester extrahiert. Die vereinten organischen Phasen wurden einmal mit Wasser gewaschen. Die vereinten wässrigen Phasen wurden dreimal mit Dichlormethan extrahiert. Die gesammelten organischen Phasen wurden über Natriumsulfat getrocknet, filtriert, und eingeengt. Der Rückstand wurde mittels Dickschichtchromatographie gereinigt (Lösungsmittel: Dichlormethan/Ethanol = 30/1). Die Produktfraktionen wurden eingeengt und der Rückstand mittels präparativer HPLC gereinigt (RP18 Säule, Laufmittel: Acetonitril/Wasser-Gradient unter Zusatz von 0.1% TFA). Es wurden 5 mg der Zielverbindung (8% d. Th.) erhalten.
LC-MS (Methode 2): Rₜ = 0.89 min
MS (ESpos): m/z = 399 (M-TFA+H)⁺

### Beispiel 25

### 7-[(2,6-Difluorbenzyl)oxy]-3-{1-[2-(3,5-dimethyl-1H-pyrazol-4-yl)ethyl]-1H-pyrazol-4-yl}-2,5-dimethylpyrazolo[1,5-a]pyridin

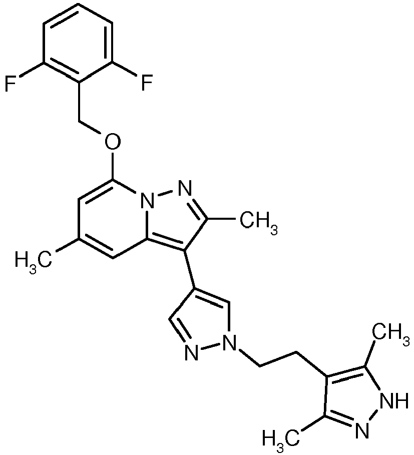

23 mg (0.06 mmol) 7-[(2,6-Difluorbenzyl)oxy]-2,5-dimethyl-3-(1H-pyrazol-4-yl)pyrazolo[1,5-a]pyridin aus Beispiel 1 in 0.3 ml DMF wurden mit 0.08 ml (0.08 mmol) Kalium-tert-butylat-Lösung (1 N in THF) versetzt, 5 min bei Raumtemperatur gerührt, anschließend mit 18 mg (0.09 mmol) 4-(2-Bromethyl)-3,5-dimethyl-1H-pyrazol und 1 mg (0.01 mmol) Kaliumiodid versetzt und über Nacht bei 70°C gerührt. Das Reaktionsgemisch wurde eingeengt, und der Rückstand wurde mittels Dickschichtchromatographie gereinigt (Lösungsmittel: Dichlormethan/Ethanol = 20/1).
Es wurden 10 mg der Zielverbindung (34% d. Th.) erhalten.
LC-MS (Methode 2): Rₜ = 0.88 min
MS (ESpos): m/z = 477 (M+H)⁺

### Beispiel 26

### N¹-(4-{7-[(2,6-Difluorbenzyl)oxy]-2,5-dimethylpyrazolo[1,5-a]pyridin-3-yl}-5-fluorpyridin-2-yl)-2-methylpropan-1,2-diamin Trifluoracetat

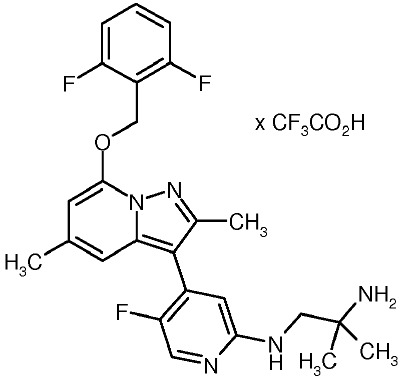

35 mg (0.085 mmol) 7-[(2,6-Difluorbenzyl)oxy]-3-(2,5-difluorpyridin-4-yl)-2,5-dimethylpyrazolo[1,5-a]pyridin aus Beispiel 17 wurden in 0.20 ml NMP vorgelegt. Bei Raumtemperatur wurden 89 mg (1.02 mmol) 2-Methylpropan-1,2-diamin zugegeben und das Gemisch wurde 2 h bei 150°C in einem geschlossenem Gefäß in der Mikrowelle gerührt. Die Reaktionslösung wurde mit Acetonitril/Wasser verdünnt, mit TFA versetzt und mittels präparativer HPLC gereinigt (RP18 Säule, Laufmittel: Acetonitril/Wasser-Gradient unter Zusatz von 0.1% TFA). Es wurden 2 mg der Zielverbindung (1% d. Th.) erhalten.
LC-MS (Methode 2): Rₜ = 0.61 min
MS (ESpos): m/z = 470 (M-TFA+H)⁺

### Beispiel 27

### 5-{7-[(2,6-Difluorbenzyl)oxy]-2,5-dimethylpyrazolo[1,5-a]pyridin-3-yl}nicotinsäure

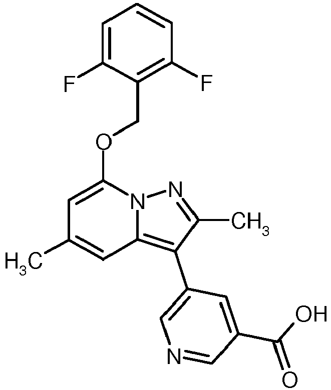

31 mg (0.07 mmol) Ethyl-5-{7-[(2,6-difluorbenzyl)oxy]-2,5-dimethylpyrazolo[1,5-a]pyridin-3-yl}nicotinat aus Beispiel 18 in 1.5 ml THF/Ethanol (5/1) wurden mit 0.35 ml (0.35 mmol) IN wässriger Lithiumhydroxid-Lösung versetzt und 4 h bei Raumtemperatur gerührt. Es wurden nochmals 0.35 ml (0.35 mmol) 1N wässriger Lithiumhydroxid-Lösung und 0.36 ml THF/Ethanol (5/1) hinzugegeben und das Gemisch wurde über Nacht bei Raumtemperatur gerührt. Es wurden 0.48 ml THF/Ethanol (5/1) hinzugegeben und das Gemisch wurde nochmals 3 h bei Raumtemperatur gerührt. Die Mischung wurde unter Eiskühlung mit 1 N wässriger SalzsäureLösung auf pH = 4 gebracht und anschließend am Rotationsverdampfer von den organischen Lösemittel befreit. Die Suspension wurde abfiltriert und mit Wasser gewaschen und getrocknet. Es wurden 26 mg (87% d. Th.) der Titelverbindung erhalten.
LC-MS (Methode 2): Rₜ = 0.90 min
MS (ESpos): m/z = 410 (M+H)⁺

### Beispiel 28

### 5-{7-[(2,6-Difluorbenzyl)oxy]-2,5-dimethylpyrazolo[1,5-a]pyridin-3-yl}nicotinamid

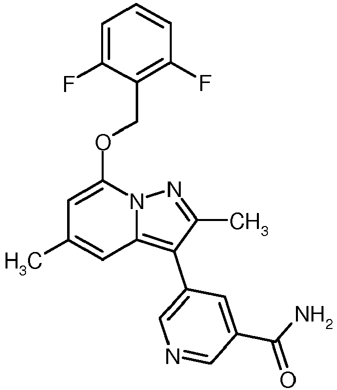

22 mg (0.05 mmol) 5-{7-[(2,6-Difluorbenzyl)oxy]-2,5-dimethylpyrazolo[1,5-a]pyridin-3-yl}nicotinsäure aus Beispiel 27 in 1.0 ml Dichlormethan wurden mit 15 mg (0.08 mmol) 1-(3-Dimethylaminopropyl)-3-ethylcarbodiimidhydrochlorid und 12 mg (0.08 mmol) 1-Hydroxy-1H-benzotriazol-Hydrat versetzt und 10 min bei Raumtemperatur gerührt. Anschließend wurden 14 mg (0.26 mmol) Ammoniumchlorid und 46 mg (0.36 mmol) *N,N*-Diisopropylethylamin zugegeben und es wurde über Nacht bei Raumtemperatur gerührt. Das Reaktionsgemisch wurde eingeengt und der Rückstand wurde mittels Dickschichtchromatographie gereinigt (Laufmittel: Dichlormethan/2 N Ammoniak in Methanol = 20/1). Es wurden 15 mg (70% d. Th.) der Titelverbindung erhalten.
LC-MS (Methode 2): Rₜ = 0.82 min
MS (ESpos): m/z = 409 (M+H)⁺
¹H-NMR (500 MHz, DMSO-*d*₆) δ = 2.40 (s, 3 H), 2.42 (s, 3 H), 5.47 (s, 2 H), 6.52 (s, 1 H), 7.15 (s, 1 H), 7.28 (t, 2 H), 7.59 - 7.69 (m, 2 H), 8.23 (br. s, 2 H), 8.78 (s, 1 H), 8.95 (s, 1 H).

### Beispiel 29

### 7-[(2,6-Difluorbenzyl)oxy]-2,5-dimethyl-3-(pyrimidin-5-yl)pyrazolo[1,5-a]pyridin

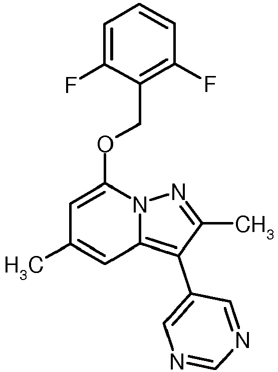

60 mg (0.16 mmol) 3-Brom-7-[(2,6-difluorbenzyl)oxy]-2,5-dimethylpyrazolo[1,5-a]pyridin aus Beispiel 17A und 30 mg (0.24 mmol) Pyrimidin-5-ylborsäure wurden in 2 ml abs. Acetonitril vorgelegt und mit Argon begast. Anschließend wurden 6 mg (0.008 mmol) Chloro(2-dicyclohexylphosphino-2',4',6'-triisopropyl-1,1'-biphenyl) [2-(2'-amino-1,1'-biphenyl)palladium (II) [CAS: 1310584-14-5] hinzugegeben und mit 0.98 ml (0.49 mmol) wässriger 0.5 M Kaliumphosphat-Lösung (sauerstofffrei) versetzt. Das Reaktionsgemisch wurde 5 h bei 60°C intensiv gerührt. Die Reaktionslösung wurde abgekühlt, mit Wasser versetzt und dreimal mit Essigsäureethylester extrahiert. Die vereinigten organischen Phasen wurde einmal mit Wasser gewaschen, über Natriumsulfat getrocknet, filtriert und eingeengt. Der Rückstand wurde in Acetonitril, Wasser und TFA gelöst und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlormethan und wenig Methanol aufgenommen und zweimal mit wässriger, gesättigter Natriumhydrogencarbonat-Lösung gewaschen. Die vereinigten wässrigen Phasen wurden zweimal mit Dichlormethan reextrahiert. Die vereinigten organischen Phasen wurden über Natriumsulfat getrocknet, filtriert und eingeengt. Es wurden 6 mg (10% d. Th.) der Titelverbindung isoliert.
LC-MS (Methode 2): Rₜ = 0.98 min
MS (ESpos): m/z = 367 (M+H)⁺

### Beispiel 30

### 2-Cyclopropyl-7-[(2,6-difluorbenzyl)oxy]-5-methyl-3-{-[2-(morpholin-4-yl)ethyl]-1H-pyrazol-4-yl}pyrazolo[1,5-a]pyridin

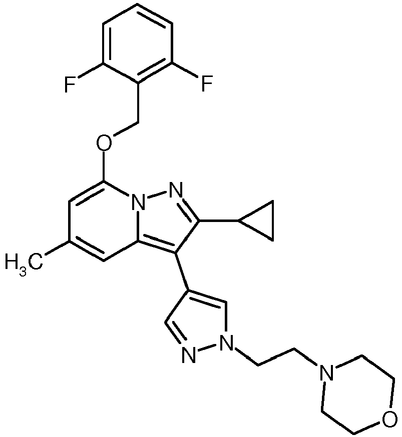

60 mg (0.14 mmol, Reinheit 90%) 3-Brom-2-cyclopropyl-7-[(2,6-difluorbenzyl)oxy]-5-methylpyrazolo[1,5-a]pyridin aus Beispiel 19A und 63 mg (0.21 mmol) 4-{2-[4-(4,4,5,5-Tetramethyl-1,3,2-dioxaborolan-2-yl)-1H-pyrazol-1-yl]ethyl}morpholin wurden in 1.7 ml abs. Acetonitril vorgelegt und mit Argon begast. Anschließend wurden 5.4 mg (0.007 mmol) Chloro(2-dicyclohexylphosphino-2',4',6'-triisopropyl-1,1'-biphenyl)[2-(2'-amino-1,1'-biphenyl)palladium (II) [CAS: 1310584-14-5] hinzugegeben und mit 0.82 ml (0.41 mmol) wässriger 0.5 M Kaliumphosphat-Lösung (sauerstofffrei) versetzt. Das Reaktionsgemisch wurde 5 min bei 100°C intensiv gerührt. Die Reaktionslösung wurde abgekühlt, in Acetonitril, Wasser und TFA gelöst und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlormethan und wenig Methanol aufgenommen und zweimal mit wässriger, gesättigter Natriumhydrogencarbonat-Lösung gewaschen. Die vereinigten wässrigen Phasen wurden zweimal mit Dichlormethan reextrahiert. Die vereinigten organischen Phasen wurden über Natriumsulfat getrocknet, filtriert und eingeengt. Es wurden 45 mg (65% d. Th.) der Titelverbindung isoliert.
LC-MS (Methode 2): Rₜ = 0.82 min
MS (ESpos): m/z = 494 (M+H)⁺

### Beispiel 31

### 2-Cyclopropyl-7-[(2,6-difluorbenzyl)oxy]-5-methyl-3-(1H-pyrazol-4-yl)pyrazolo[1,5-a]pyridin

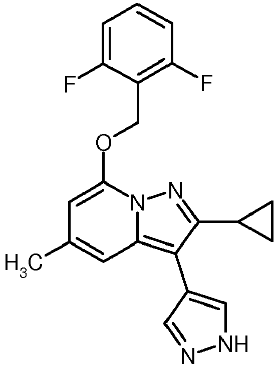

60 mg (0.14 mmol, Reinheit 90%) 3-Brom-2-cyclopropyl-7-[(2,6-difluorbenzyl)oxy]-5-methylpyrazolo[1,5-a]pyridin aus Beispiel 19A und 61 mg (0.21 mmol) tert-Butyl-4-(4,4,5,5-tetramethyl-1,3,2-dioxaborolan-2-yl)-1H-pyrazol-1-carboxylat wurden in 0.8 ml abs. Acetonitril vorgelegt und mit Argon begast. Anschließend wurden 5.4 mg (0.007 mmol) Chloro(2-dicyclohexylphosphino-2',4',6'-triisopropyl-1,1'-biphenyl)[2-(2'-amino-1,1'-biphenyl)palladium (II) [CAS: 1310584-14-5] hinzugegeben und mit 0.82 ml (0.41 mmol) wässriger 0.5 M Kaliumphosphat-Lösung (sauerstofffrei) versetzt. Das Reaktionsgemisch wurde 10 min bei 100°C intensiv gerührt. Die Reaktionslösung wurde abgekühlt und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlormethan aufgenommen und zweimal mit wässriger, gesättigter Natriumhydrogencarbonat-Lösung gewaschen. Die vereinigten wässrigen Phasen wurden zweimal mit Dichlormethan reextrahiert. Die vereinigten organischen Phasen wurden über Natriumsulfat getrocknet, filtriert und eingeengt. Es wurden 9 mg der Titelverbindung isoliert.

Die produktrelevanten Fraktionen [d.h. die noch Boc-geschützte Zielverbindung] der präparativen HPLC wurden eingeengt. Der Rückstand wurde mit 0.1 ml Diethylether und 0.2 ml Chlorwasserstofflösung (2 N in Diethylether) versetzt und über Nacht bei RT gerührt. Es wurde anschließend 1 ml Chlorwasserstofflösung (2 N in Diethylether) hinzugegeben und das Gemisch wurde nochmals über Nacht bei RT gerührt. Die Reaktionsgemisch wurde mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlormethan aufgenommen und zweimal mit wässriger, gesättigter Natriumhydrogencarbonat-Lösung gewaschen. Die vereinigten wässrigen Phasen wurden zweimal mit Dichlormethan reextrahiert. Die vereinigten organischen Phasen wurden über Natriumsulfat getrocknet, filtriert und zusammen mit der zuvor erhaltenen Fraktion eingeengt. In Summe wurden 14 mg (26% d. Th.) der Titelverbindung isoliert.
LC-MS (Methode 2): Rₜ = 1.01 min
MS (ESpos): m/z = 381 (M+H)⁺

### Beispiel 32

### 2,5-Dimethyl-3-{1-[2-(morpholin-4-yl)ethyl]-1H-pyrazol-4-yl}-7-[(2,3,6-trifluorbenzyl)oxy]pyrazolo[1,5-a]pyridin Trifluoracetat

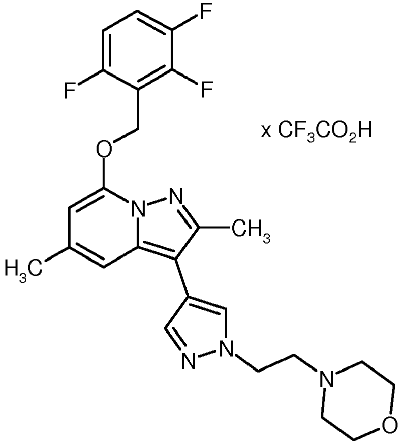

25 mg (0.05 mmol, Reinheit 71%) 3-Brom-2,5-dimethyl-7-[(2,3,6-trifluorbenzyl)oxy]pyrazolo[1,5-a]pyridin aus Beispiel 18A und 21 mg (0.07 mmol) 4-{2-[4-(4,4,5,5-Tetramethyl-1,3,2-dioxaborolan-2-yl)-1H-pyrazol-1-yl]ethyl}morpholin wurden in 0.6 ml abs. Acetonitril vorgelegt und mit Argon begast. Anschließend wurden 2 mg (0.002 mmol) Chloro(2-dicyclohexylphosphino-2',4',6'-triisopropyl-1,1'-biphenyl)[2-(2'-amino-1,1'-biphenyl)palladium (II) [CAS: 1310584-14-5] hinzugegeben und mit 0.28 ml (0.14 mmol) wässriger 0.5 M Kaliumphosphat-Lösung (sauerstofffrei) versetzt. Das Reaktionsgemisch wurde 7.5 h bei 60°C intensiv gerührt. Es wurden nochmals 2 mg (0.002 mmol) Chloro(2-dicyclohexylphosphino-2',4',6'-triisopropyl-1,1'-biphenyl)[2-(2'-amino-1,1'-biphenyl)palladium (II) [CAS: 1310584-14-5] und 14 mg (0.05 mmol) 4-{2-[4-(4,4,5,5-Tetramethyl-1,3,2-dioxaborolan-2-yl)-1H-pyrazol-1-yl]ethyl}morpholin hinzugegeben und das Gemisch wurde 20 min bei 100°C gerührt. Das Reaktionsgemisch wurde abgekühlt, mit Wasser versetzt und dreimal mit Essigsäureethylester extrahiert. Die vereinigten, organischen Phasen wurde einmal mit Wasser gewaschen, über Natriumsulfat getrocknet, filtriert und eingeengt. Der Rückstand wurde in Acetonitril, Wasser und TFA gelöst und mittels präparativer HPLC gereinigt (RP18 Säule, Laufmittel: Acetonitril/Wasser-Gradient unter Zusatz von 0.1% TFA). Es wurden 3.5 mg (13% d. Th.) der Titelverbindung isoliert.
LC-MS (Methode 2): Rₜ = 0.76 min
MS (ESpos): m/z = 486 (M-TFA+H)⁺

### Beispiel 33

### 7-(Cyclohexylmethoxy)-2,5-dimethyl-3-{1-[2-(morpholin-4-yl)ethyl]-1H-pyrazol-4-yl}pyrazolo[1,5-a]pyridin

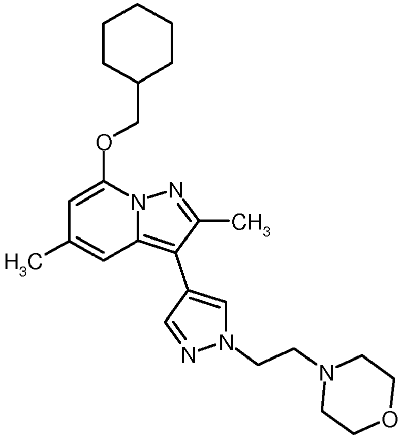

35 mg (0.09 mmol, Reinheit 87%) 3-Brom-7-(cyclohexylmethoxy)-2,5-dimethylpyrazolo[1,5-a]pyridin aus Beispiel 24A und 42 mg (0.14 mmol) 4-{2-[4-(4,4,5,5-Tetramethyl-1,3,2-dioxaborolan-2-yl)-1H-pyrazol-1-yl]ethyl}morpholin wurden in 1.1 ml abs. Acetonitril vorgelegt und mit Argon begast. Anschließend wurden 3.5 mg (0.005 mmol) Chloro(2-dicyclohexylphosphino-2',4',6'-triisopropyl-1,1'-biphenyl)[2-(2'-amino-1,1'-biphenyl)palladium (II) [CAS: 1310584-14-5] hinzugegeben und es wurde mit 0.54 ml (0.27 mmol) wässriger 0.5 M Kaliumphosphat-Lösung (sauerstofffrei) versetzt. Das Reaktionsgemisch wurde über Nacht bei 60°C intensiv gerührt. Anschließend wurden nochmals 3.5 mg (0.005 mmol) Chloro(2-dicyclohexylphosphino-2',4',6'-triisopropyl-1,1'-biphenyl)[2-(2'-amino-1,1'-biphenyl)palladium (II) [CAS: 1310584-14-5] und 10 mg (0.03 mmol) 4-{2-[4-(4,4,5,5-Tetramethyl-1,3,2-dioxaborolan-2-yl)-1H-pyrazol-1-yl]ethyl}morpholin hinzugegeben und das Gemisch wurde 25 min bei 100°C intensiv gerührt. Die Reaktionslösung wurde abgekühlt, mit Wasser versetzt und dreimal mit Essigsäureethylester extrahiert. Die vereinigten organischen Phasen wurden einmal mit Wasser gewaschen, über Natriumsulfat getrocknet, filtriert und eingedampft. Der Rückstand wurde in Acetonitril, Wasser und TFA gelöst und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlormethan und wenig Methanol aufgenommen und zweimal mit wässriger, gesättigter Natriumhydrogencarbonat-Lösung gewaschen. Die vereinigten wässrigen Phasen wurden zweimal mit Dichlormethan extrahiert. Die vereinigten organischen Phasen wurden über Natriumsulfat getrocknet, filtriert und eingedampft. Es wurden 22 mg (55% d. Th.) der Titelverbindung isoliert.
LC-MS (Methode 2): Rₜ = 0.82 min
MS (ESpos): m/z = 438 (M+H)⁺

### Beispiel 34

### 4-Amino-2-{7-[(2,6-difluorbenzyl)oxy]-2,5-dimethylpyrazolo[1,5-a]pyridin-3-yl}-5,5-dimethyl-5,7-dihydro-6H-pyrrolo[2,3-d]pyrimidin-6-on Trifluoracetat

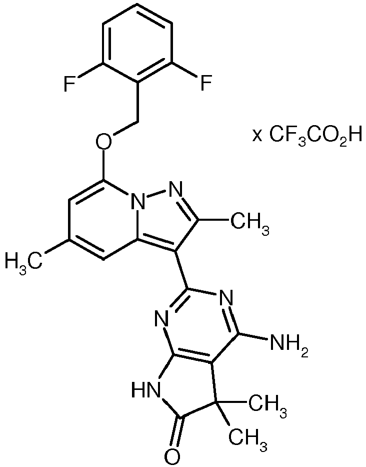

65 mg (0.15 mmol; Reinheit 91%) 7-[(2,6-Difluorbenzyl)oxy]-2,5-dimethylpyrazolo[1,5-a]pyridin-3-carboximidamid Acetat aus Beispiel 28A wurden unter Argon in 1.7 ml tert.-Butanol vorgelegt, bei RT nacheinander mit 26 mg (0.23 mmol) Kalium-tert.-butylat und 38 mg (0.23 mmol) Methyl-3,3-dicyan-2,2-dimethylpropanoat versetzt und über Nacht zum Rückfluss erhitzt. Dann wurde abgekühlt und das Gemisch wurde eingedampft. Der Rückstand wurde in Acetonitril aufgenommen, mit Wasser und Trifluoressigsäure versetzt und mittels präparativer HPLC gereinigt (RP18 Säule, Laufmittel: Acetonitril/Wasser-Gradient unter Zusatz von 0.1% TFA). Es wurden 19 mg (21 % d. Th.) der Titelverbindung erhalten.
LC-MS (Methode 2): Rₜ = 0.97 min
MS (ESpos): m/z = 465 (M-TFA+H)⁺
¹H-NMR (400 MHz, DMSO-*d*₆) δ = 1.32 (s, 6 H), 2.44 (s, 3 H), 2.68 (s, 3 H), 5.43 (s, 2 H), 6.38 - 6.60 (m, 3 H), 7.27 (t, 2 H), 7.58 - 7.68 (m, 2 H), 8.12 (s, 1 H), 10.75 (br. s, 1 H).

### Beispiel 35

### 4-[(2-Amino-2-methylpropyl)amino]-2-{7-[(2,6-difluorbenzyl)oxy]-2,5-dimethylpyrazolo[1,5-a]pyridin-3-yl}-5,5-dimethyl-5,7-dihydro-6H-pyrrolo[2,3-d]pyrimidin-6-on

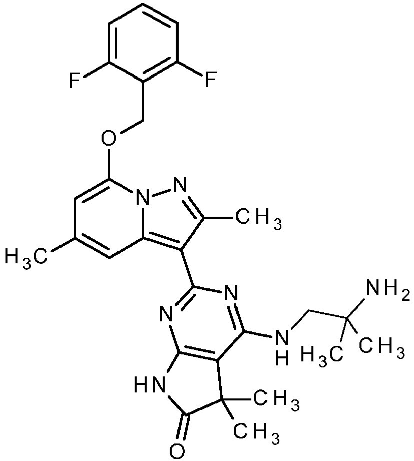

20 mg (0.035 mmol) 2-{7-[(2,6-Difluorbenzyl)oxy]-2,5-dimethylpyrazolo[1,5-a]pyridin-3-yl}-4-iod-5,5-dimethyl-5,7-dihydro-6H-pyrrolo[2,3-d]pyrimidin-6-on aus Beispiel 29A wurden unter Argon in 0.15 ml 1-Methyl-2-pyrrolidon (NMP) vorgelegt und mit 23 mg (0.26 mmol) 2-Methylpropan-1,2-diamin versetzt und 135 min bei 120°C gerührt. Dann wurde abgekühlt und das Gemisch wurde mittels präparativer HPLC gereinigt (RP18 Säule, Laufmittel: Acetonitril/Wasser-Gradient unter Zusatz von 0.1% TFA). Die Produktfraktionen wurden eingedampft und der Rückstand in Dichlormethan aufgenommen. Die Lösung wurde zweimal mit 2 ml gesättigter, wässriger Natriumhydrogencarbonatlösung ausgeschüttelt. Die organische Phase wurde über Natriumsulfat getrocknet, filtriert, eingedampft und lyophilisiert. Es wurden 5.3 mg (28% d. Th.) der Titelverbindung erhalten.
LC-MS (Methode 2): Rₜ = 0.81 min
MS (ESpos): m/z = 536 (M+H)⁺

### B. Bewertung der pharmakologischen Wirksamkeit

Es werden die folgenden Abkürzungen verwendet:
- ATP: Adenosintriphosphat
- Brij35: Polyoxyethylen(23)laurylether
- BSA: Rinderserumalbumin
- DTT: Dithiothreitol
- TEA: Triethanolamin

Die pharmakologische Wirkung der erfindungsgemäßen Verbindungen kann in folgenden Assays gezeigt werden:

### B-1. Vermessung von sGC Enzymaktivität mittels PPi Nachweis

Lösliche Guanylylcyclase (sGC) setzt unter Stimulation GTP zu cGMP und Pyrophosphat (PPi) um. PPi wird mit Hilfe des in WO 2008/061626 beschriebenen Verfahrens nachgewiesen. Das im Test entstehende Signal nimmt mit fortschreitender Umsetzung zu und dient als Maß für die sGC-Enzymaktivität. Mit Hilfe einer PPi Referenzkurve kann das Enzym in bekannter Weise charakterisiert werden, z.B. hinsichtlich Umsatzrate, Stimulierbarkeit oder Michaelis Konstante.

### Durchführung des Tests

Zur Durchführung des Tests wurden 29 µL Enzymlösung (0-10 nM lösliche Guanylylcyclase (hergestellt nach Hönicka et al., Journal of Molecular Medicine 77(1999)14-23), in 50 mM TEA, 2 mM Magnesiumchlorid, 0.1% BSA (FraktionV), 0.005% Brij 35, pH 7.5) in die Mikroplatte vorgelegt und 1 µL der Stimulatorlösung (0-10 µM 3-Morpholinosydnonimine, SIN-1, Merck in DMSO) hinzugegeben. Es wurde 10 min bei RT inkubiert. Anschließend wurden 20 µl Detektionsmix (1,2 nM Firefly Luciferase (*Photinus pyralis* Luziferase, Promega), 29 µM Dehydro-Luziferin (hergestellt nach Bitler & McElroy, Arch. Biochem. Biophys. 72 (1957) 358), 122 µM Luziferin (Promega), 153 µM ATP (Sigma) und 0,4 mM DTT (Sigma) in 50 mM TEA, 2 mM Magnesiumchlorid, 0.1% BSA (Fraktion V), 0.005% Brij 35, pH 7,5) zugegeben. Die Enzymreaktion wurde durch Zugabe von 20 µl Substratlösung (1.25 mM Guanosin-5'-triphosphat (Sigma) in 50 mM TEA, 2 mM Magnesiumchlorid, 0.1% BSA (Fraktion V), 0.005% Brij 35, pH 7.5) gestartet und kontinuierlich luminometrisch vermessen.

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative MEC-Werte (MEC = minimal effektive Konzentration) für die erfindungsgemäßen Verbindungen sind in der nachstehenden Tabelle wiedergegeben (zum Teil als Mittelwerte aus Einzelbestimmungen):

**Tabelle A:**

| Beispiel | MEC [µM] |
|---|---|
| 1 | 0.03 |
| 2 | 0.53 |
| 3 | 0.065 |
| 4 | 0.07 |
| 5 | 0.07 |
| 6 | 0.1 |
| 7 | 0.065 |
| 8 | 1.0 |
| 9 | 3.0 |
| 10 | 0.065 |
| 11 | 0.03 |
| 12 | 0.055 |
| 13 | 0.1 |
| 14 | 2.0 |
| 15 | 0.3 |
| 16 | 0.1 |
| 17 | 2.0 |
| 18 | 10.0 |
| 19 | 0.3 |
| 20 | 2.0 |
| 21 | 0.03 |
| 22 | 0.10 |
| 23 | 0.03 |
| 24 | 0.065 |
| 25 | 0.065 |
| 27 | 2.0 |
| 28 | 0.03 |
| 29 | 0.30 |
| 30 | 0.01 |
| 31 | 0.03 |
| 32 | 0.10 |
| 33 | 0.10 |
| 34 | 1.0 |
| 35 | 3.0 |

### B-3. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

### B-4. Blutdruckmessung an narkotisierten Ratten

Männliche Wistar-Ratten mit einem Körpergewicht von 300 - 350 g werden mit Thiopental (100 mg/kg i.p.) anästhesiert. Nach der Tracheotomie wird in die Femoralarterie ein Katheter zur Blutdruckmessung eingeführt. Die zu prüfenden Substanzen werden als Lösungen entweder oral mittels Schlundsonde oder über die Femoralvene intravenös verabreicht (Stasch et al. Br. J. Pharmacol. 2002; 135: 344-355).

### B-5. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
Implantierbare Sender (Physiotel® Telemetrietransmitter)
Empfänger (Physiotel® Receiver), die über einen Multiplexer (DSI Data Exchange Matrix) mit einem

Datenakquisitionscomputer verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5% iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen
Systolischer Blutdruck (SBP)
Diastolischer Blutdruck (DBP)
Arterieller Mitteldruck (MAP)
Herzfrequenz (HR)
Aktivität (ACT)

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur:

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994.

### B-6. Bestimmung pharmakokinetischer Kenngrößen nach intravenöser und oraler Gabe

Die pharmakokinetischen Parameter der erfindungsgemäßen Verbindungen werden in männlichen CD-1-Mäusen, männlichen Wistar-Ratten und weiblichen Beagle-Hunden bestimmt. Die intravenöse Gabe erfolgt bei Mäusen und Ratten mittels einer speziesspezifischen Plasma/DMSO-Formulierung und bei Hunden mittels einer Wasser/PEG400/Ethanol-Formulierung. Die orale Gabe der gelösten Substanz mittels Schlundsonde wird in allen Spezies basierend auf einer Wasser/PEG400/Ethanol-Formulierung durchgeführt. Den Ratten wird zur vereinfachten Blutabnahme vor der Substanzgabe ein Silikonkatheter in die rechte *Vena jugularis externa* gelegt. Die Operation erfolgt mindestens einen Tag vor dem Versuch unter Isofluran-Narkose und unter Gabe eines Analgetikums (Atropin/Rimadyl (3/1) 0.1 mL s.c.). Die Blutabnahme (in der Regel mehr als 10 Zeitpunkte) erfolgt in einem Zeitfenster, welches terminale Zeitpunkte von mindestens 24 bis maximal 72 Stunden nach Substanzgabe beinhaltet. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen, Kalibrierproben und Qualifier wird ein interner Standard zugesetzt (dies kann auch eine chemisch nicht verwandte Substanz sein) und es folgt eine Proteinfällung mittels Acetonitril im Überschuss. Nach Zugabe einer Puffer-Lösung, die an die LC-Bedingungen angepasst ist, und folgendem Vortexen wird bei 1000 g zentrifugiert. Der Überstand wird mittels LC-MS/MS unter Verwendung von C18-reversed-phase-Säulen und variablen Eluenten-Gemischen vermessen. Die Quantifizierung der Substanzen erfolgt anhand der Peakhöhen oder -flächen aus extrahierten Ionenchromatogrammen spezifischer selected ion monitoring-Experimente.

Aus den ermittelten Plasmakonzentration-Zeit-Verläufen werden die pharmakokinetischen Kenngrößen wie AUC, Cₘₐₓ, t_{1/2} (terminale Halbwertszeit), F (Bioverfügbarkeit), MRT (Mean Residence Time) und CL (Clearance) mittels eines validierten pharmakokinetischen Rechenprogramms berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (mittels LC-MS/MS; s.o.) und durch Quotientenbildung der C_{Blut}/C_{Plasma}-Wert ermittelt.

### B-7. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inkubationsansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### B-8. Caco-2 Permeabilitäts-Test

Die Permeabilität einer Testsubstanz wurde mit Hilfe der Caco-2 Zelllinie, einem etablierten *in vitro* Modell für Permeabilitätsvorhersagen an der gastrointestinalen Barriere, bestimmt (Artursson, P. and Karlsson, J. (1991). Correlation between oral drug absorption in humans and apparent drug permeability coefficients in human intestinal epithelial (Caco-2) cells. Biochem. Biophys.175 (3), 880-885). Die Caco-2 Zellen (ACC No. 169, DSMZ, Deutsche Sammlung von Mikroorganismen und Zellkulturen, Braunschweig, Deutschland) wurden in 24-Well Platen mit Einsatz ausgesät und 14 bis 16 Tage kultiviert. Für die Permeabilitätsstudien wurde die Testsubstanz in DMSO gelöst und mit Transportpuffer (Hanks Buffered Salt Solution, Gibco/Invitrogen, mit 19.9 mM Glukose und 9.8 mM HEPES) auf die finale Testkonzentration verdünnt. Um die Permeabilität von apikal nach basolateral (PₐₚₚA-B) der Testsubstanz zu bestimmen, wurde die Lösung mit der Testsubstanz auf die apikale Seite des Caco-2 Zellmonolayers gegeben und Transportpuffer auf die basolaterale Seite. Um die Permeabilität von basolateral nach apikal (PₐₚₚB-A) der Testsubstanz zu bestimmen, wurde die Lösung mit der Testsubstanz auf die basolaterale Seite des Caco-2 Zellmonolayers gegeben und Transportpuffer auf die apikale Seite. Zu Beginn des Experiments wurden Proben aus dem jeweiligen Donor-Kompartiment genommen, um die Massenbilanz sicher zu stellen. Nach einer Inkubation von zwei Stunden bei 37° C wurden Proben aus beiden Kompartimenten genommen. Die Proben wurden mittels LC-MS/MS analysiert und die apparenten Permeabilitätskoeffizienten (Pₐₚₚ) berechnet. Die Permeabilität von Lucifer Yellow wurde für jeden Zellmonolayer bestimmt, um die Integrität der Zellschicht sicher zu stellen. Die Permeabilität von Atenolol (Marker für niedrige Permeabilität) und Sulfasalazin (Marker für aktive Exkretion) wurde in jedem Testlauf als Qualitätskontrolle mitbestimmt.

### B-9. hERG Kaliumstrom Assay.

Der sogenannte hERG (human ether-a-go-go related gene) Kaliumstrom trägt wesentlich zur Repolarisierung des humanen kardialen Aktionspotentials bei (Scheel et al., 2011). Eine Inhibition dieses Stroms durch Pharmaka kann in seltenen Fällen potentiell letale Herzrhythmusstörungen zur Folge haben, und wird deshalb frühzeitig während der Arzneimittelentwicklung untersucht.

Der hier verwendete funktionelle hERG Assay basiert auf einer recombinanten HEK293 ZellLinie, die das KCNH2(HERG)-Gen stabil exprimiert (Zhou et al., 1998). Diese Zellen werden mittels der "whole-cell voltage-clamp" Technik (Hamill et al., 1981) in einem automatisierten System (Patchliner™; Nanion, München, D) untersucht, welches die Membranspannung kontrolliert und den hERG Kalium-Strom bei Zimmertemperatur misst. Die PatchControlHT™ Software (Nanion) steuert Patchliner System, Datenerfassung und Datenanalyse. Die Spannungskontrolle erfolgt durch 2 EPC-10 quadro Verstärker unter Kontrolle der PatchMasterPro™ Software (beide: HEKA Elektronik, Lambrecht, D). NPC-16 Chips mit mittlerem Widerstand (∼2 MΩ; Nanion) dienen als planares Substrat für die Voltage-Clamp Experimente.

NPC-16 Chips werden mit intra- und extrazellulärer Lösung (vgl. Himmel, 2007) sowie mit Zellsuspension befüllt. Nach Bildung eines Giga-Ohm-Seals und Herstellen des Ganzzell-Modus (einschliesslich mehrerer automatisierter Qualitätskontrollschritte) wird die Zellmembran auf das Haltepotential -80 mV geklemmt. Das nachfolgende Spannungsklemm-Protokoll ändert die Kommandospannung auf +20 mV (Dauer 1000 ms), -120 mV (Dauer 500 ms), und zurück zum Haltepotential -80 mV; dies wird alle 12 s wiederholt. Nach einer initialen Stabilisierungsphase (ca 5-6 Minuten) wird Testsubstanzlösung in aufsteigenden Konzentrationen (z.B. 0.1, 1, und 10 µmol/L) zupipettiert (Exposition ca 5-6 Minuten pro Konzentration), gefolgt von mehreren Auswaschschritten.

Die Amplitude des einwärtsgerichteten "Tail"-Stroms, der durch eine Potentialänderung von +20 mV auf -120 mV erzeugt wird, dient zur Quantifizierung des hERG Kaliumstroms, und wird als Funktion der Zeit dargestellt (IgorPro™ Software). Die Stromamplitude am Ende verschiedener Zeitabschnitte (z.B. Stabilisierungsphase vor Testsubstanz, erste/zweite/dritte Konzentration Testsubstanz) dient zur Erstellung einer Konzentrations-Wirkungs-Kurve, aus der die halbmaximale Hemmkonzentration IC₅₀ der Testsubstanz errechnet wird.
Hamill OP, Marty A, Neher E, Sakmann B, Sigworth FJ. Improved patch-clamp techniques for high-resolution current recording from cells and cell-free membrane patches. Pfluegers Arch 1981; 391:85-100.
Himmel HM. Suitability of commonly used excipients for electrophysiological in-vitro safety pharmacology assessment of effects on hERG potassium current and on rabbit Purkinje fiber action potential. J Pharmacol Toxicol Methods 2007;56:145-158.
Scheel O, Himmel H, Rascher-Eggstein G, Knott T. Introduction of a modular automated voltage-clamp platform and its correlation with manual human ether-a-go-go related gene voltage-clamp data. Assay Drug Dev Technol 2011;9:600-607.
Zhou ZF, Gong Q, Ye B, Fan Z, Makielski JC, Robertson GA, January CT. Properties of hERG channels stably expressed in HEK293 cells studied at physiological temperature. Biophys J 1998;74:230-241.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel® (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Wässrige NatriumchloridLösung, Glucoselösung 5% und/oder PEG 400-Lösung 30%) gelöst. Die erhaltene Lösung wird steril filtriert und in sterile und pyrogenfreie Injektionsbehältnisse abgefüllt.

## Patentansprüche

1. Verbindung der Formel (I-A) oder (I-B), in welcher 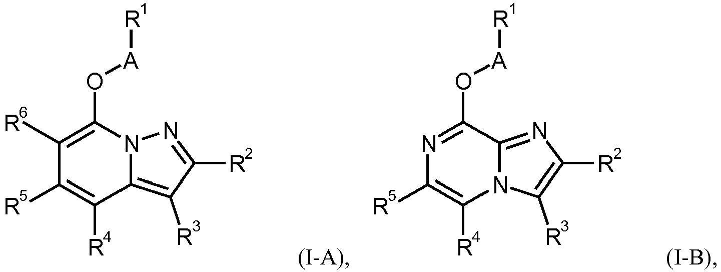
A für CH₂, CD₂ oder CH(CH₃) steht,
R¹ für (C₄-C₆)-Alkyl, (C₃-C₇)-Cycloalkyl, Pyridyl oder Phenyl steht,
wobei (C₄-C₆)-Alkyl bis zu sechsfach mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
wobei Pyridyl mit 1 oder 2 Substituenten unabhängig voneinander ausgewählt aus der Gruppe Halogen, Cyano und (C₁-C₄)-Alkyl substituiert ist,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl und (C₁-C₄)-Alkoxy substituiert sein kann,
R² für Wasserstoff, (C₁-C₄)-Alkyl, (C₁-C₄)-Alkoxymethyl, Cyclopropyl, Cyclobutyl, Monofluormethyl, Difluormethyl oder Trifluormethyl steht,
R³ für Phenyl oder 5- bis 10-gliedriges Heteroaryl steht,
wobei Phenyl mit 1 bis 3 Substituenten unabhängig voneinander ausgewählt aus der Gruppe Halogen, Cyano, Trifluormethyl, Difluormethyl, (C₁-C₆)-Alkyl, (C₁-C₄)-Alkoxycarbonyl, (C₁-C₄)-Alkylcarbonyl, Hydroxycarbonyl, -(C=O)NR⁷R⁸, (C₁-C₄)-Alkylsulfonyl, (C₃-C₆)-Cycloalkylsulfonyl, (C₁-C₄)-Alkylthio, (C₁-C₄)-Alkoxy, Trifluormethoxy, Difluormethoxy, Phenoxy, Hydroxy, Amino, Morpholinyl, Piperidinyl, Pyrrolidinyl, Piperazinyl und (C₃-C₆)-Cycloalkyl substituiert sein kann,
worin Amino mit 1 oder 2 Substituenten unabhängig voneinander ausgewählt aus (C₁-C₆)-Alkyl, (C₁-C₄)-Alkylcarbonyl, (C₃-C₆)-Cycloalkylsulfonyl, (₁-C₄)-Alkylsulfonyl und Methoxy-(C₁-C₄)-alkyl substituiert sein kann,
worin (C₁-C₆)-Alkyl mit Amin substituiert sein kann,
worin (C₁-C₆)-Alkyl mit 1 oder 2 Substituenten ausgewählt aus der Gruppe Fluor, Trifluormethoxy, -(C=O)NR⁷R⁸, (C₁-C₄)-Alkoxy, (C₃-C₆)-Cycloalkyl, Morpholinyl, Piperidinyl, Pyrrolidinyl, Piperazinyl, Hydroxy und Amino substituiert sein kann,
worin Amino mit 1 oder 2 Substituenten unabhängig voneinander ausgewählt aus (C₁-C₆)-Alkyl, (C₁-C₄)-Alkylcarbonyl, (C₁-C₄)-Alkoxycarbonyl, (C₃-C₆)-Cycloalkylsulfonyl, (C₁-C₄)-Alkylsulfonyl und Methoxy-(C₁-C₄)-alkyl substituiert sein kann,
und worin
R⁷ und R⁸ jeweils unabhängig voneinander für Wasserstoff, (C₁-C₄)-Alkyl oder (C₃-C₇)-Cycloalkyl stehen,
wobei 5- bis 10-gliedriges Heteroaryl mit 1 bis 3 Substituenten unabhängig voneinander ausgewählt aus der Gruppe Fluor, Chlor, Cyano, (C₁-C₆)-Alkyl, (C₁-C₄)-Alkoxy, Amino, (C₁-C₄)-Alkoxycarbonyl, Hydroxycarbonyl, -(C=O)NR⁷R⁸, Phenyl, Pyridyl, Pyrimidyl, 1,3-Thiazol-5-yl, Pyrrolidinyl, Piperidinyl, Morpholinyl, Piperazinyl und (C₃-C₇)-Cycloalkyl substituiert sein kann,
worin (C₁-C₆)-Alkyl mit 1 bis 3 Substituenten ausgewählt aus der Gruppe Fluor, Cyano, Hydroxy, Amino, Trifluormethyl, Difluormethyl, (C₁-C₄)-Alkylsulfonyl, (C₁-C₄)-Alkylcarbonyl, (C₁-C₄)-Alkoxycarbonyl, Hydroxy-carbonyl, -(C=O)NR7R⁸ (C₁-C₄)-Alkoxy, Phenoxy, Phenyl, Pyridyl, Pyrimidyl, 5-gliedriges Heteroaryl, (C₃-C₇)-Cycloalkyl, Morpholinyl, Piperidinyl, Pyrrolidinyl, Piperazinyl, 1,1-Dioxidothiomorpholin-4-yl und Azetidin substituiert sein kann,
worin 5-gliedriges Heteroaryl mit 1 bis 3 Substituenten ausgewählt aus der Gruppe Halogen, (C₁-C₄)-Alkyl und (C₁-C₄)-Alkoxy substituiert sein kann,
worin
R⁷ und R⁸ jeweils unabhängig voneinander für Wasserstoff, (C₁-C₄)-Alkyl oder (C₃-C₇)-Cycloalkyl stehen,
worin Piperidinyl mit 1 bis 4 Substituenten Fluor substituiert sein kann,
worin Phenyl mit 1 bis 3 Substituenten ausgewählt aus der Gruppe Halogen, (C₁-C₄)-Alkyl und (C₁-C₄)-Alkoxy substituiert sein kann,
worin Azetidin mit Hydroxy substituiert sein kann,
worin Amino mit 1 oder 2 Substituenten (C₁-C₄)-Alkyl substituiert sein kann,
und
worin Piperazinyl mit 1 bis 3 Substituenten unabhängig voneinander ausgewählt aus der Gruppe (C₁-C₄)-Alkyl, (C₃-C₇)-Cycloalkyl und Trifluormethyl substituiert sein kann,
worin (C₃-C₇)-Cycloalkyl mit 1 oder 2 Substituenten unabhängig ausgewählt aus der Gruppe Halogen, (C₁-C₆)-Alkyl, (C₁-C₄)-Alkoxy-carbonyl und Hydroxycarbonyl substituiert sein kann,
worin (C₁₁-C₄)-Alkoxy mit Amino substituiert sein kann,
worin Amino mit 1 oder 2 Substituenten unabhängig voneinander ausgewählt aus (C₁-C₆)-Alkyl, (C₁-C₄)-Alkylcarbonyl, (C₃-C₆)-Cycloalkylsulfonyl und(C₁-C₄)-Alkylsulfonyl substituiert sein kann,
worin (C₁-C₆)-Alkyl mit Amino substituiert sein kann,
und
worin (C₁-C₆)-Alkyl bis zu fünffach mit Fluor substituiert sein kann,
worin Phenyl, Pyridyl und Pyrimidyl mit 1 oder 2 Substituenten ausgewählt aus der Gruppe Methyl, Ethyl und Fluor substituiert sein können,
worin
R⁷ und R⁸ jeweils unabhängig voneinander für Wasserstoff, (C₁-C₄)-Alkyl oder (C₃-C₇)-Cycloalkyl stehen,
oder
R³ für eine Gruppe der Formel 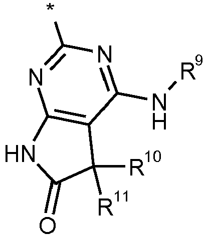 steht, wobei
* für die Anknüpfstelle an das Pyrazolopyridin bzw. das Imidazopyrazin steht,
R⁹ für Wasserstoff oder (C₁-C₆)-Alkyl steht,
wobei (C₁-C₆)-Alkyl mit Amino substituiert sein kann,
und
worin (C₁-C₆)-Alkyl bis zu fünffach mit Fluor substituiert sein kann,
R¹⁰ für Wasserstoff, Methyl oder Ethyl steht,
R¹¹ für Wasserstoff, Methyl, Ethyl, Trifluormethyl oder Cyclopropyl steht,
oder
R¹⁰ und R¹¹ zusammen mit dem Kohlenstoffatom, an das sie gebunden sind, einen 3- bis 6-gliedrigen Carbocyclus bilden,
R⁴ für Wasserstoff steht,
R⁵ für Wasserstoff, Halogen, Cyano, Difluormethyl, Trifluormethyl, (C₁-C₄)-Alkyl, (C₃-C₇)-Cycloalkyl, (C₂-C₄)-Alkinyl, (C₁-C₄)-Alkylamino, Difluormethoxy, Trifluormethoxy, (C₁-C₄)-Alkoxy, Amino, 4- bis 7-gliedriges Heterocyclyl oder 5- oder 6-gliedriges Heteroaryl steht,
R⁶ für Wasserstoff, Cyano oder Halogen steht,
sowie ihre *N*-Oxide, Salze, Solvate, Salze der *N*-Oxide und Solvate der *N*-Oxide und Salze.

2. Verbindung der Formel (I-A) oder (I-B) nach Anspruch 1, in welcher
A für CH₂ oder CD₂ steht,
R¹ für Cyclohexyl, Pyridyl oder Phenyl steht,
wobei Cyclohexyl bis zu vierfach mit Fluor substituiert sein kann,
wobei Pyridyl mit 1 oder 2 Substituenten Fluor substituiert ist,
und
wobei Phenyl mit 1 bis 4 Substituenten unabhängig voneinander ausgewählt aus der Gruppe Fluor, Chlor, Cyano, Methyl und Methoxy substituiert sein kann,
R² für Wasserstoff, (C₁-C₄)-Alkyl, Cyclopropyl, Difluormethyl oder Trifluormethyl steht,
R³ für Phenyl oder 5- oder 6-gliedriges Heteroaryl steht,
wobei Phenyl mit 1 bis 3 Substituenten unabhängig voneinander ausgewählt aus der Gruppe Fluor, Chlor, Cyano, Trifluormethyl, Difluormethyl, (C₁-C₆)-Alkyl, (C₁-C₄)-Alkoxycarbonyl, (C₁-C₄)-Alkylcarbonyl, Hydroxycarbonyl, -(C=O)NR⁷R⁸, (C₁-C₄)-Alkylsulfonyl, (C₃-C₆)-Cycloalkylsulfonyl, (C₁-C₄)-Alkoxy, Trifluormethoxy, Difluormethoxy, Hydroxy, Amino, Morpholinyl, Piperidinyl, Pyrrolidinyl, Piperazinyl und Cyclopropyl substituiert sein kann,
worin Amino mit 1 oder 2 Substituenten unabhängig voneinander ausgewählt aus (C₁-C₆)-Alkyl, (C₁-C₄)-Alkylcarbonyl und (C₁-C₄)-Alkylsulfonyl substituiert sein kann,
worin (C₁-C₆)-Alkyl mit 1 oder 2 Substituenten ausgewählt aus der Gruppe Fluor, Trifluormethoxy, -(C=O)NR⁷R⁸,(C₁-C₄)-Alkoxy, (C₃-C₆)-Cycloalkyl, Morpholinyl, Piperidinyl, Pyrrolidinyl, Piperazinyl, Hydroxy und Amino substituiert sein kann,
worin Amino mit 1 oder 2 Substituenten unabhängig voneinander ausgewählt aus (C₁-C₆)-Alkyl, (C₁-C₄)-Alkylcarbonyl und (C₁-C₄)-Alkylsulfonyl substituiert sein kann,
und worin
R⁷ und R⁸ jeweils unabhängig voneinander für Wasserstoff, (C₁-C₄)-Alkyl oder Cyclopropyl stehen,
wobei 5- oder 6-gliedriges Heteroaryl mit 1 bis 3 Substituenten unabhängig voneinander ausgewählt aus der Gruppe Fluor, Chlor, Cyano, (C₁-C₆)-Alkyl, (C₁-C₄)-Alkoxy, Amino, (C₁-C₄)-Alkoxycarbonyl, Hydroxycarbonyl, -(C=O)NR⁷R⁸, Phenyl, Pyridyl, Pyrimidyl, Pyrrolidinyl, Piperidinyl, Morpholinyl, Piperazinyl und Cyclopropyl substituiert sein kann,
worin (C₁-C₆)-Alkyl mit 1 bis 3 Substituenten ausgewählt aus der Gruppe Fluor, Cyano, Hydroxy, Amino, Trifluormethyl, Difluormethyl, (C₁-C₄)-Alkylsulfonyl, (C₁-C₄)-Alkoxycarbonyl, Hydroxycarbonyl,-(C=O)NR⁷R⁸, (C₁-C₄)-Alkoxy, Phenyl, Pyridyl, Pyrimidyl, 5-gliedriges Heteroaryl, (C₃-C₇)-Cycloalkyl, Morpholinyl, Piperidinyl, Pyrrolidinyl, Piperazinyl, 1,1-Dioxidothiomorpholin-4-yl und Azetidin substituiert sein kann,
worin 5-gliedriges Heteroaryl mit 1 bis 3 Substituenten ausgewählt aus der Gruppe Fluor, Chlor, Methyl, Ethyl und Methoxy substituiert sein kann,
worin
R⁷ und R⁸ jeweils unabhängig voneinander für Wasserstoff, Methyl, Ethyl oder Cyclopropyl stehen,
worin Piperidinyl mit 1 bis 4 Substituenten Fluor substituiert sein kann,
worin Phenyl mit 1 bis 3 Substituenten ausgewählt aus der Gruppe Fluor, Methyl, Ethyl und Methoxy substituiert sein kann,
worin Amino mit 1 oder 2 Substituenten unabhängig voneinander ausgewählt aus (C₁-C₆)-Alkyl oder (C₁-C₄)-Alkylcarbonyl substituiert sein kann,
worin (C₁-C₆)-Alkyl mit Amino substituiert sein kann,
worin Phenyl, Pyridyl und Pyrimidyl mit 1 oder 2 Substituenten ausgewählt aus der Gruppe Methyl, Ethyl und Fluor substituiert sein können,
und worin
R⁷ und R⁸ jeweils unabhängig voneinander für Wasserstoff, Methyl, Ethyl oder Cyclopropyl stehen,
oder
R³ für eine Gruppe der Formel 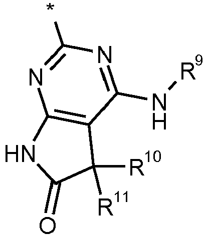 steht, wobei
* für die Anknüpfstelle an das Pyrazolopyridin bzw. das Imidazopyrazin steht,
R⁹ für Wasserstoff oder (C₁-C₆)-Alkyl steht,
wobei (C₁-C₆)-Alkyl mit Amino substituiert sein kann,
R¹⁰ für Methyl oder Ethyl steht,
R¹¹ für Methyl, Ethyl, Trifluormethyl oder Cyclopropyl steht,
R⁴ für Wasserstoff steht,
R⁵ für Wasserstoff, Fluor, Chlor, Cyano, Difluormethyl, Trifluormethyl, (C₁-C₄)-Alkyl, (C₃-C₅)-Cycloalkyl steht,
R⁶ für Wasserstoff steht,
sowie ihre *N*-Oxide, Salze, Solvate, Salze der *N*-Oxide und Solvate der *N*-Oxide und Salze.

3. Verbindung der Formel (I-A) nach Anspruch 1 oder 2, in welcher
A für CH₂ oder CD₂ steht,
R¹ für Cyclohexyl, Pyridyl oder Phenyl steht,
wobei Cyclohexyl bis zu vierfach mit Fluor substituiert sein kann,
wobei Pyridyl mit 1 oder 2 Substituenten Fluor substituiert ist,
und
wobei Phenyl mit 1 bis 4 Substituenten unabhängig voneinander ausgewählt aus der Gruppe Fluor, Chlor, Cyano, Methyl und Methoxy substituiert sein kann,
R² für Wasserstoff, (C₁-C₄)-Alkyl, Cyclopropyl, Difluormethyl oder Trifluormethyl steht,
R³ für Phenyl oder 5- oder 6-gliedriges Heteroaryl steht,
wobei Phenyl mit 1 bis 3 Substituenten unabhängig voneinander ausgewählt aus der Gruppe Fluor, Chlor, Cyano, Trifluormethyl, Difluormethyl, (C₁-C₆)-Alkyl, (C₁-C₄)-Alkoxycarbonyl, (C₁-C₄)-Alkylcarbonyl, Hydroxycarbonyl, -(C=O)NR⁷R⁸, (C₁-C₄)-Alkylsulfonyl, (C₃-C₆)-Cycloalkylsulfonyl, (C₁-C₄)-Alkoxy, Trifluormethoxy, Difluormethoxy, Hydroxy, Amino, Morpholinyl, Piperidinyl, Pyrrolidinyl, Piperazinyl und Cyclopropyl substituiert sein kann,
worin Amino mit 1 oder 2 Substituenten unabhängig voneinander ausgewählt aus (C₁-C₆)-Alkyl, (C₁-C₄)-Alkylcarbonyl und (C₁-C₄)-Alkylsulfonyl substituiert sein kann,
worin (C₁-C₆)-Alkyl mit 1 oder 2 Substituenten ausgewählt aus der Gruppe Fluor, Trifluormethoxy, -(C=O)NR⁷R⁸, (C₁-C₄)-Alkoxy, (C₃-C₆)-Cycloalkyl, Morpholinyl, Piperidinyl, Pyrrolidinyl, Piperazinyl, Hydroxy und Amino substituiert sein kann,
worin Amino mit 1 oder 2 Substituenten unabhängig voneinander ausgewählt aus (C₁-C₆)-Alkyl, (C₁-C₄)-Alkylcarbonyl und (C₁-C₄)-Alkylsulfonyl substituiert sein kann,
und worin
R⁷ und R⁸ jeweils unabhängig voneinander für Wasserstoff, (C₁-C₄)-Alkyl oder Cyclopropyl stehen,
wobei 5- oder 6-gliedriges Heteroaryl mit 1 bis 3 Substituenten unabhängig voneinander ausgewählt aus der Gruppe Fluor, Chlor, Cyano, (C₁-C₆)-Alkyl, (C₁-C₄)-Alkoxy, Amino, (C₁-C₄)-Alkoxycarbonyl, Hydroxycarbonyl, -(C=O)NR⁷R⁸, Phenyl, Pyridyl, Pyrimidyl, Pyrrolidinyl, Piperidinyl, Morpholinyl, Piperazinyl und Cyclopropyl substituiert sein kann,
worin (C₁-C₆)-Alkyl mit 1 bis 3 Substituenten ausgewählt aus der Gruppe Fluor, Cyano, Hydroxy, Amino, Trifluormethyl, Difluormethyl, (C₁-C₄)-Alkylsulfonyl, (C₁-C₄)-Alkoxycarbonyl, Hydroxycarbonyl, -(C=O)NR⁷R⁸, (C₁-C₄)-Alkoxy, Phenyl, Pyridyl, Pyrimidyl, 5-gliedriges Heteroaryl, (C₃-C₇)-Cycloalkyl, Morpholinyl, Piperidinyl, Pyrrolidinyl, Piperazinyl, 1,1-Dioxidothiomorpholin-4-yl und Azetidin substituiert sein kann,
worin 5-gliedriges Heteroaryl mit 1 bis 3 Substituenten ausgewählt aus der Gruppe Fluor, Chlor, Methyl, Ethyl und Methoxy substituiert sein kann,
worin
R⁷ und R⁸ jeweils unabhängig voneinander für Wasserstoff, Methyl, Ethyl oder Cyclopropyl stehen,
worin Piperidinyl mit 1 bis 4 Substituenten Fluor substituiert sein kann,
worin Phenyl mit 1 bis 3 Substituenten ausgewählt aus der Gruppe Fluor, Methyl, Ethyl und Methoxy substituiert sein kann,
worin Amino mit 1 oder 2 Substituenten unabhängig voneinander ausgewählt aus (C₁-C₆)-Alkyl oder (C₁-C₄)-Alkylcarbonyl, substituiert sein kann,
worin (C₁-C₆)-Alkyl mit Amino substituiert sein kann,
worin Phenyl, Pyridyl und Pyrimidyl mit 1 oder 2 Substituenten ausgewählt aus der Gruppe Methyl, Ethyl und Fluor substituiert sein können,
und worin
R⁷ und R⁸ jeweils unabhängig voneinander für Wasserstoff, Methyl, Ethyl oder Cyclopropyl stehen,
R⁴ für Wasserstoff steht,
R⁵ für Wasserstoff, Fluor, Chlor, Cyano, Difluormethyl, Trifluormethyl, (C₁-C₄)-Alkyl, (C₃-C₅)-Cycloalkyl steht,
R⁶ für Wasserstoff steht,
sowie ihre *N*-Oxide, Salze, Solvate, Salze der *N*-Oxide und Solvate der *N*-Oxide und Salze.

4. Verbindung der Formel (I-A) nach Anspruch 1, 2 oder 3, in welcher
A für CH₂ steht,
R¹ für Phenyl steht,
wobei Phenyl bis zu dreifach mit Fluor substituiert ist,
R² für Methyl steht,
R³ für Phenyl, Pyridyl, Pyrimidyl oder 4-Pyrazolyl steht,
wobei Phenyl mit 1 bis 3 Substituenten unabhängig voneinander ausgewählt aus der Gruppe Fluor, (C₁-C₆)-Alkyl, Hydroxycarbonyl, -(C=O)NR⁷R⁸, Methylsulfonyl, Ethylsulfonyl, Amino, Morpholinyl, Piperidinyl, Pyrrolidinyl und Piperazinyl substituiert sein kann,
worin Amino mit 1 oder 2 Substituenten unabhängig voneinander ausgewählt aus (C₁-C₆)-Alkyl, Methylcarbonyl, Ethylcarbonyl, Methylsulfonyl und Ethylsulfonyl substituiert sein kann,
worin (C₁-C₆)-Alkyl mit 1 oder 2 Substituenten ausgewählt aus der Gruppe Fluor, Morpholinyl, Piperidinyl, Pyrrolidinyl, Piperazinyl und Amino substituiert sein kann,
worin Amino mit 1 oder 2 Substituenten unabhängig voneinander ausgewählt aus (C₁-C₄)-Alkyl, Methylcarbonyl, Ethylcarbonyl, Methylsulfonyl und Ethylsulfonyl substituiert sein kann,
und worin
R⁷ und R⁸ jeweils unabhängig voneinander für Wasserstoff oder Cyclopropyl stehen,
wobei Pyridyl und 4-Pyrazolyl mit 1 oder 2 Substituenten unabhängig voneinander ausgewählt aus der Gruppe Fluor, Chlor, (C₁-C₆)-Alkyl, Methoxy, Amino, Hydroxycarbonyl, -(C=O)NR⁷R⁸, Phenyl, Pyridyl, Pyrrolidinyl, Piperidinyl, Morpholinyl und Piperazinyl substituiert sein können,
worin (C₁-C₆)-Alkyl mit 1 bis 3 Substituenten ausgewählt aus der Gruppe Fluor, Hydroxy, Amino, Hydroxycarbonyl, -(C=O)NR⁷R⁸, Methoxy, Phenyl, Pyridyl, Pyrazolyl, (C₃-C₇)-Cycloalkyl, Morpholinyl, Piperidinyl, Pyrrolidinyl und Piperazinyl substituiert sein kann,
worin Pyrazolyl mit 1 oder 2 Substituenten Methyl substituiert sein kann,
und worin
R⁷ und R⁸ jeweils unabhängig voneinander für Wasserstoff, Methyl, Ethyl oder Cyclopropyl stehen,
worin Amino mit 1 oder 2 Substituenten (C₁-C₆)-Alkyl substituiert sein kann,
worin (C₁-C₆)-Alkyl mit Amino substituiert sein kann,
worin Phenyl, Pyridyl und Pyrimidyl mit 1 oder 2 Substituenten ausgewählt aus der Gruppe Methyl, Ethyl und Fluor substituiert sein können,
und worin
R⁷ und R⁸ jeweils unabhängig voneinander für Wasserstoff, Methyl oder Cyclopropyl stehen,
R⁴ für Wasserstoff steht,
R⁵ für Wasserstoff oder Methyl steht,
R⁶ für Wasserstoff steht,
sowie ihre *N*-Oxide, Salze, Solvate, Salze der *N*-Oxide und Solvate der *N*-Oxide und Salze.

5. Verbindung der Formel (I-A) nach Anspruch 1, 2, 3 oder 4 in welcher
A für CH₂ steht,
R¹ für eine Phenyl-Gruppe der Formel 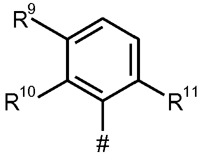 steht, wobei
# für die Anknüpfstelle an A steht,
und
R⁹ für Wasserstoff oder Fluor steht,
R¹⁰ für Fluor steht,
R¹¹ für Fluor steht,
R² für Methyl steht,
R³ für Phenyl, 3-Pyridyl, 4-Pyridyl oder 4-Pyrazolyl steht,
wobei Phenyl in 3-Position mit Fluor, (C₁-C₆)-Alkyl, Hydroxycarbonyl,-(C=O)NR⁷R⁸, Methylsulfonyl, Ethylsulfonyl, Amino, Morpholinyl, Piperidinyl, Pyrrolidinyl und Piperazinyl substituiert sein kann,
worin Amino mit 1 oder 2 Substituenten unabhängig voneinander ausgewählt aus (C₁-C₆)-Alkyl, Methylcarbonyl, Ethylcarbonyl, Methylsulfonyl und Ethylsulfonyl substituiert sein kann,
worin (C₁-C₆)-Alkyl mit 1 oder 2 Substituenten ausgewählt aus der Gruppe Fluor, Morpholinyl, Piperidinyl, Pyrrolidinyl, Piperazinyl und Amino substituiert sein kann,
worin Amino mit 1 oder 2 Substituenten unabhängig voneinander ausgewählt aus (C₁-C₄)-Alkyl, Methylcarbonyl, Ethylcarbonyl, Methylsulfonyl und Ethylsulfonyl substituiert sein kann,
und worin
R⁷ und R⁸ jeweils unabhängig voneinander für Wasserstoff oder Cyclopropyl stehen,
wobei 3-Pyridyl und 4-Pyridyl jeweils mit 1 oder 2 Substituenten unabhängig voneinander ausgewählt aus der Gruppe Fluor, Chlor, (C₁-C₆)-Alkyl, Methoxy, Amino, Hydroxycarbonyl, -(C=O)NR⁷R⁸, Phenyl, Pyridyl, Pyrrolidinyl, Piperidinyl, Morpholinyl und Piperazinyl substituiert sein können,
worin Amino mit 1 oder 2 Substituenten (C₁-C₆)-Alkyl substituiert sein kann,
worin (C₁-C₆)-Alkyl mit Amino substituiert sein kann,
worin Phenyl, Pyridyl und Pyrimidyl mit 1 oder 2 Substituenten ausgewählt aus der Gruppe Methyl, Ethyl und Fluor substituiert sein können,
und worin
R⁷ und R⁸ jeweils unabhängig voneinander für Wasserstoff, Methyl oder Cyclopropyl stehen,
wobei 4-Pyrazolyl an 1-Position mit (C₁-C₆)-Alkyl, Phenyl oder Pyridyl substituiert sein kann,
worin (C₁-C₆)-Alkyl mit 1 bis 3 Substituenten ausgewählt aus der Gruppe Fluor, Hydroxy, Amino, Methoxycarbonyl, Ethoxycarbonyl, Hydroxy-carbonyl, -(C=O)NR⁷R⁸, Methoxy, Phenyl, Pyridyl, Pyrazolyl, (C₃-C₇)-Cycloalkyl, Morpholinyl, Piperidinyl, Pyrrolidinyl und Piperazinyl substituiert sein kann,
worin Pyrazolyl mit 1 bis 3 Substituenten Methyl substituiert sein kann,
worin
R⁷ und R⁸ jeweils unabhängig voneinander für Wasserstoff, Methyl, Ethyl oder Cyclopropyl stehen,
worin Phenyl und Pyridyl mit 1 oder 2 Substituenten ausgewählt aus der Gruppe Methyl, Ethyl und Fluor substituiert sein können,
R⁴ für Wasserstoff steht,
R⁵ für Wasserstoff oder Methyl steht,
R⁶ für Wasserstoff steht,
sowie ihre *N*-Oxide, Salze, Solvate, Salze der *N*-Oxide und Solvate der *N*-Oxide und Salze.

6. Verbindung der Formel (I-A) nach Anspruch 1, 2, 3, 4 oder 5 in welcher
A für CH₂ steht,
R¹ für eine Phenyl-Gruppe der Formel 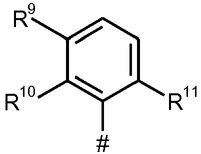 steht, wobei
# für die Anknüpfstelle an A steht,
und
R⁹ für Wasserstoff oder Fluor steht,
R¹⁰ für Fluor steht,
R¹¹ für Fluor steht,
R² für Methyl steht,
R³ für Phenyl, 3-Pyridyl, 4-Pyridyl oder 4-Pyrazolyl steht,
wobei Phenyl in 3-Position mit (C₁-C₆)-Alkyl, Hydroxycarbonyl, -(C=O)NR⁷R⁸, Methylsulfonyl, Ethylsulfonyl, Amino und Pyrrolidinyl substituiert sein kann,
worin Amino mit 1 oder 2 Substituenten unabhängig voneinander ausgewählt aus (C₁-C₆)-Alkyl, Methylcarbonyl und Methylsulfonyl substituiert sein kann,
worin (C₁-C₆)-Alkyl mit 1 oder 2 Substituenten ausgewählt aus der Gruppe Fluor, Pyrrolidinyl und Amino substituiert sein kann,
worin Amino mit 1 oder 2 Substituenten unabhängig voneinander ausgewählt aus (C₁-C₄)-Alkyl, Methylcarbonyl und Methylsulfonyl substituiert sein kann,
und worin
R⁷ und R⁸ jeweils unabhängig voneinander für Wasserstoff oder Cyclopropyl stehen,
wobei 3-Pyridyl und 4-Pyridyl jeweils mit 1 oder 2 Substituenten unabhängig voneinander ausgewählt aus der Gruppe Fluor, Chlor, (C₁-C₆)-Alkyl, Amino, Hydroxycarbonyl, -(C=O)NR⁷R⁸, Phenyl und Piperazinyl substituiert sein können,
worin Amino mit 1 oder 2 Substituenten (C₁-C₆)-Alkyl substituiert sein kann,
worin (C₁-C₆)-Alkyl mit Amino substituiert sein kann,
worin Phenyl mit 1 oder 2 Substituenten ausgewählt aus der Gruppe Methyl, Ethyl und Fluor substituiert sein kann,
und worin
R⁷ und R⁸ jeweils für Wasserstoff steht,
wobei 4-Pyrazolyl an 1-Position mit (C₁-C₆)-Alkyl oder Phenyl substituiert sein kann,
worin (C₁-C₆)-Alkyl mit 1 bis 3 Substituenten ausgewählt aus der Gruppe Hydroxy, Amino, Methoxycarbonyl, Hydroxycarbonyl, -(C=O)NR⁷R⁸, Phenyl, Pyrazolyl, (C₃-C₇)-Cycloalkyl und Morpholinyl substituiert sein kann,
worin Pyrazolyl mit 1 oder 3 Substituenten Methyl substituiert sein kann,
worin
R⁷ für Wasserstoff steht,
R⁸ für Cyclopropyl steht,
worin Phenyl und Pyridyl mit 1 oder 2 Substituenten Fluor substituiert sein können,
R⁴ für Wasserstoff steht,
R⁵ für Wasserstoff oder Methyl steht,
R⁶ für Wasserstoff steht,
sowie ihre *N*-Oxide, Salze, Solvate, Salze der *N*-Oxide und Solvate der *N*-Oxide und Salze.

7. Verbindung der Formel (I-B) nach Anspruch 1 oder 2 in welcher
A für CH₂ oder CD₂ steht,
R¹ für oder Phenyl steht, wobei Phenyl mit 1 bis 3 Substituenten Fluor substituiert ist,
R² für Methyl steht,
R³ für 5- oder 6-gliedriges Heteroaryl steht,
wobei 5- oder 6-gliedriges Heteroaryl mit 1 bis 3 Substituenten unabhängig voneinander ausgewählt aus der Gruppe (C₁-C₄)-Alkyl und Amino substituiert sein kann,
worin (C₁-C₄)-Alkyl mit 1 bis 3 Substituenten ausgewählt aus der Gruppe Hydroxy, Amino, (C₁-C₄)-Alkoxycarbonyl, Hydroxycarbonyl, Phenyl, 5-gliedriges Heteroaryl, (C₃-C₇)-Cycloalkyl, Morpholinyl und 1,1-Dioxidothiomorpholin-4-yl substituiert sein kann,
worin Amino mit (C₁-C₆)-Alkyl substituiert sein kann,
worin (C₁-C₆)-Alkyl mit Amino substituiert sein kann,
oder
R³ für eine Gruppe der Formel 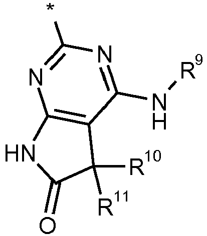 steht, wobei
* für die Anknüpfstelle an das Imidazopyrazin steht,
R⁹ für Wasserstoff oder (C₁-C₆)-Alkyl steht,
wobei (C₁-C₆)-Alkyl mit Amino substituiert sein kann,
R¹⁰ für Methyl oder Ethyl steht,
R¹¹ für Methyl, Ethyl oder Trifluormethyl steht,
R⁴ für Wasserstoff steht,
R⁵ für Wasserstoff oder Methyl steht,
R⁶ für Wasserstoff steht,
sowie ihre *N*-Oxide, Salze, Solvate, Salze der *N*-Oxide und Solvate der *N*-Oxide und Salze

8. Verfahren zur Herstellung von Verbindungen der Formel (I-A) bzw. (I-B), wie in den Ansprüchen 1 bis 7 definiert, **dadurch gekennzeichnet, dass** man
[A] eine Verbindung der Formel (II) 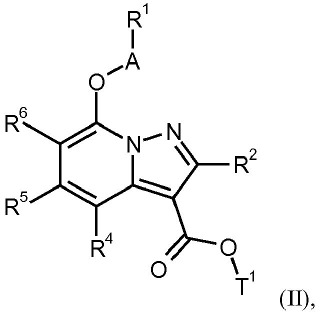 in welcher A, R¹, R², R⁴, R⁵ und R⁶ jeweils die oben angegebenen Bedeutungen haben und
T¹ für (C₁-C₄)-Alkyl oder Benzyl steht,
in einem inerten Lösungsmittel in Gegenwart einer geeigneten Base oder Säure zu einer Carbonsäure der Formel (III) 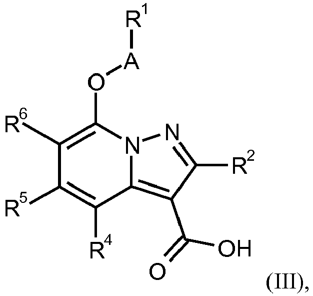 in welcher A, R¹, R², R⁴, R⁵ und R⁶ jeweils die oben angegebenen Bedeutungen haben,
umsetzt und diese dann mit einem Halogen-Äquivalent in Anwesenheit einer geeigneten Base in eine Verbindung der Formel (IV) 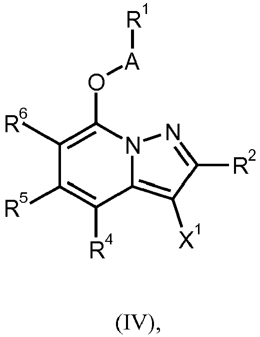 in welcher A, R¹, R², R⁴, R⁵ und R⁶ jeweils die oben angegebenen Bedeutungen haben und
X¹ für Chlor, Brom oder Iod steht,
überführt, und diese im Anschluß in einem inerten Lösungsmittel in Gegenwart eines geeigneten Übergangsmetallkatalysators mit einer Verbindung der Formel (V), 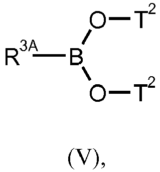 in welcher
R^{3A} die oben für R³ angegebenen Bedeutungen hat und
T² für Wasserstoff oder (C₁-C₄)-Alkyl steht, oder beide Reste T² zusammen eine -C(CH₃)₂-C(CH₃)₂-Brücke bilden,
zu einer Verbindung der Formel (I-A1) 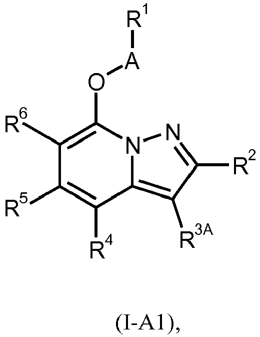 umsetzt, in welcher A, R¹, R², R⁴, R⁵ und R⁶ jeweils die oben angegebenen Bedeutungen haben,
und anschließend für den Fall, dass R^{3A} für 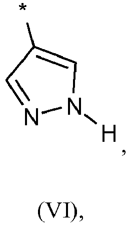 steht, diese Verbindungen in einem inerten Lösungsmittel in Gegenwart einer geeigneten Base mit einer Verbindung der Formel (VII) 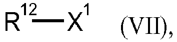 in welcher
X¹ für eine geeignete Abgangsgruppe, insbesondere Chlor, Brom, Iod, Mesylat, Triflat oder Tosylat steht,
und
R¹² für (C₁-C₆)-Alkyl steht,
worin (C₁-C₆)-Alkyl mit 1 bis 3 Substituenten ausgewählt aus der Gruppe Fluor, Cyano, Hydroxy, Amino, Trifluormethyl, Difluormethyl, (C₁-C₄)-Alkylsulfonyl, (C₁-C₄)-Alkylcarbonyl, (C₁-C₄)-Alkoxycarbonyl, Hydroxy-carbonyl, -(C=O)NR⁷R⁸ (C₁-C₄)-Alkoxy, Phenoxy, Phenyl, Pyridyl, Pyrimidyl, 5-gliedriges Heteroaryl, (C₃-C₇)-Cycloalkyl, Morpholinyl, Piperidinyl, Pyrrolidinyl, Piperazinyl, 1,1-Dioxidothiomorpholin-4-yl und Azetidin substituiert sein kann,
worin 5-gliedriges Heteroaryl mit 1 bis 3 Substituenten ausgewählt aus der Gruppe Halogen, (C₁-C₄)-Alkyl und (C₁-C₄)-Alkoxy substituiert sein kann,
worin
R⁷ und R⁸ jeweils unabhängig voneinander für Wasserstoff, (C₁-C₄)-Alkyl oder (C₃-C₇)-Cycloalkyl stehen,
worin Piperidinyl mit 1 bis 4 Substituenten Fluor substituiert sein kann,
worin Phenyl mit 1 bis 3 Substituenten ausgewählt aus der Gruppe Halogen, (C₁-C₄)-Alkyl und (C₁-C₄)-Alkoxy substituiert sein kann,
worin Azetidin mit Hydroxy substituiert sein kann,
worin Amino mit 1 oder 2 Substituenten (C₁-C₄)-Alkyl substituiert sein kann,
und
worin Piperazinyl mit 1 bis 3 Substituenten unabhängig voneinander ausgewählt aus der Gruppe (C₁-C₄)-Alkyl, (C₃-C₇)-Cycloalkyl und Trifluormethyl substituiert sein kann,
zu einer Verbindung der Formel (I-A2) 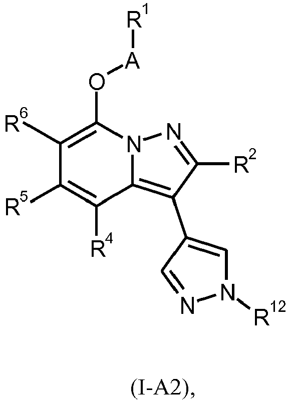 umsetzt,
in welcher A, R¹, R², R⁴, R⁵, R⁶ und R¹² jeweils die oben angegebenen Bedeutungen haben und anschliessend gegebenenfalls vorhandene Schutzgruppen abspaltet, und die resultierenden Verbindungen der Formel (I-A) gegebenenfalls mit den entsprechenden (i) Lösungsmitteln und/oder (ii) Säuren oder Basen in ihre Solvate, Salze und/oder Solvate der Salze überführt,
bzw.
[B] eine Verbindung der Formel (VIII) 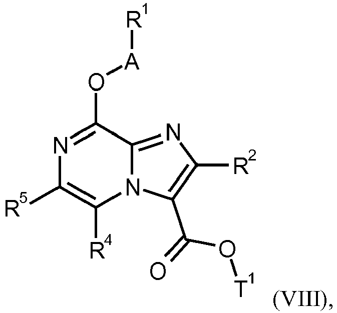 in welcher A, R¹, R², R⁴ und R⁵ jeweils die oben angegebenen Bedeutungen haben und
T¹ für (C₁-C₄)-Alkyl oder Benzyl steht,
in einem inerten Lösungsmittel in Gegenwart einer geeigneten Base oder Säure zu einer Carbonsäure der Formel (IX) 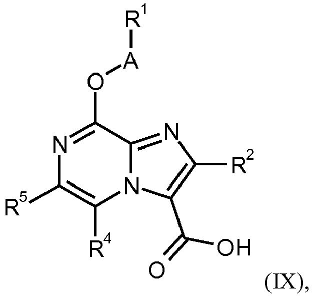 in welcher A, R¹, R², R⁴ und R⁵ jeweils die oben angegebenen Bedeutungen haben,
umsetzt und diese dann mit einem Halogen-Äquivalent in Anwesenheit einer Base in eine Verbindung der Formel (X) 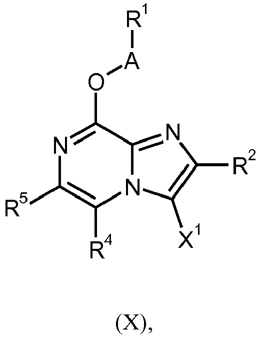 in welcher A, R¹, R², R⁴ und R⁵ jeweils die oben angegebenen Bedeutungen haben und
X¹ für Chlor, Brom oder Iod steht,
überführt, und diese im Anschluß in einem inerten Lösungsmittel in Gegenwart eines geeigneten Übergangsmetallkatalysators mit einer Verbindung der Formel (V), 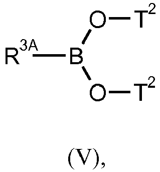 in welcher
R^{3A} die oben für R³ angegebenen Bedeutungen hat und
T² für Wasserstoff oder (C₁-C₄)-Alkyl steht, oder beide Reste T² zusammen eine -C(CH₃)₂-C(CH₃)₂-Brücke bilden,
umsetzt, gegebenenfalls vorhandene Schutzgruppen abspaltet, und die resultierenden Verbindungen der Formel (I-B) gegebenenfalls mit den entsprechenden (i) Lösungsmitteln und/oder (ii) Säuren oder Basen in ihre Solvate, Salze und/oder Solvate der Salze überführt.

9. Verbindung der Formel (I-A) oder (I-B), wie in einem der Ansprüche 1 bis 7 definiert, zur Behandlung und/oder Prophylaxe von Krankheiten.

10. Verwendung einer Verbindung der Formel (I-A) oder (I-B), wie in einem der Ansprüche 1 bis 7 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen und Arteriosklerose.

11. Arzneimittel enthaltend eine Verbindung der Formel (I-A) oder (I-B), wie in einem der Ansprüche 1 bis 7 definiert, in Kombination mit einem inerten, nicht-toxischen, pharmazeutisch geeigneten Hilfsstoff.

12. Arzneimittel enthaltend eine Verbindung der Formel (I-A) oder (I-B), wie in einem der Ansprüche 1 bis 7 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

13. Arzneimittel nach Anspruch 11 oder 12 zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen und Arteriosklerose.

## Claims

1. Compound of the formula (I-A) or (I-B) in which 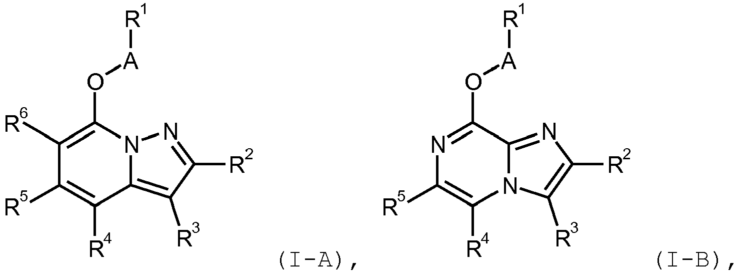
A represents CH₂, CD₂ or CH(CH₃),
R¹ represents (C₄-C₆) -alkyl, (C₃-C₇) -cycloalkyl, pyridyl or phenyl,
where (C₄-C₆) -alkyl may be up to hexasubstituted by fluorine,
where (C₃-C₇)-cycloalkyl may be substituted by 1 to 4 substituents independently of one another selected from the group consisting of fluorine, trifluoromethyl and (C₁-C₄)-alkyl, where pyridyl is substituted by 1 or 2 substituents independently of one another selected from the group consisting of halogen, cyano and (C₁-C₄)-alkyl,
and
where phenyl may be substituted by 1 to 4 substituents independently of one another selected from the group consisting of halogen, cyano, monofluoromethyl, difluoromethyl, trifluoromethyl, (C₁-C₄)-alkyl and (C₁-C₄)-alkoxy,
R² represents hydrogen, (C₁-C₄)-alkyl, (C₁-C₄)-alkoxymethyl, cyclopropyl, cyclobutyl, monofluoromethyl, difluoromethyl or trifluoromethyl,
R³ represents phenyl or 5- to 10-membered heteroaryl,
where phenyl may be substituted by 1 to 3 substituents independently of one another selected from the group of halogen, cyano, trifluoromethyl, difluoromethyl, (C₁-C₆)-alkyl, (C₁-C₄)-alkoxycarbonyl, (C₁-C₄)-alkylcarbonyl, hydroxycarbonyl, -(C=O)NR⁷R⁸, (C₁-C₄)-alkylsulphonyl, (C₃-C₆)-cycloalkylsulphonyl, (C₁-C₄)-alkylthio, (C₁-C₄)-alkoxy, trifluoromethoxy, difluoromethoxy, phenoxy, hydroxy, amino, morpholinyl, piperidinyl, pyrrolidinyl, piperazinyl, and (C₃-C₆)-cycloalkyl,
in which amino may be substituted by 1 or 2 substituents independently of one another selected from the group consisting of (C₁-C₆)-alkyl, (C₁-C₄)-alkylcarbonyl, (C₃-C₆)-cycloalkylsulphonyl, (C₁-C₄)-alkylsulphonyl and methoxy-(C₁-C₄)-alkyl, in which (C₁-C₆)-alkyl may be substituted by amine,
in which (C₁-C₆)-alkyl may be substituted by 1 or 2 substituents selected from the group consisting of fluorine, trifluoromethoxy, -(C=O)NR⁷R⁸, (C₁-C₄)-alkoxy, (C₃-C₆)-cycloalkyl, morpholinyl, piperidinyl, pyrrolidinyl, piperazinyl, hydroxy and amino,
in which amino may be substituted by 1 or 2 substituents independently of one another selected from the group consisting of (C₁-C₆)-alkyl, (C₁-C₄)-alkylcarbonyl, (C₁-C₄)-alkoxycarbonyl, (C₃-C₆)-cycloalkylsulphonyl, (C₁-C₄)-alkylsulphonyl and methoxy-(C₁-C₄)-alkyl,
and in which
R⁷ and R⁸ each independently of one another represent hydrogen, (C₁-C₄)-alkyl or (C₃-C₇)-cycloalkyl,
where 5- to 10-membered heteroaryl may be substituted by 1 to 3 substituents independently of one another selected from the group consisting of fluorine, chlorine, cyano, (C₁-C₆)-alkyl, (C₁-C₄)-alkoxy, amino, (C₁-C₄)-alkoxycarbonyl, hydroxycarbonyl, - (C=O)NR⁷R⁸, phenyl, pyridyl, pyrimidyl, 1,3-thiazol-5-yl, pyrrolidinyl, piperidinyl, morpholinyl, piperazinyl and (C₃-C₇)-cycloalkyl,
in which (C₁-C₆)-alkyl may be substituted by 1 to 3 substituents selected from the group consisting of fluorine, cyano, hydroxy, amino, trifluoromethyl, difluoromethyl, (C₁-C₄)-alkylsulphonyl, (C₁-C₄)-alkylcarbonyl, (C₁-C₄)-alkoxycarbonyl, hydroxycarbonyl,-(C=O)NR⁷R⁸ (C₁-C₄)-alkoxy, phenoxy, phenyl, pyridyl, pyrimidyl, 5-membered heteroaryl, (C₃-C₇)-cycloalkyl, morpholinyl, piperidinyl, pyrrolidinyl, piperazinyl, 1,1-dioxidothiomorpholin-4-yl and azetidine,
in which 5-membered heteroaryl may be substituted by 1 to 3 substituents selected from the group consisting of halogen, (C₁-C₄)-alkyl and (C₁-C₄)-alkoxy,
in which
R⁷ and R⁸ each independently of one another represent hydrogen, (C₁-C₄)-alkyl or (C₃-C₇) -cycloalkyl,
in which piperidinyl may be substituted by 1 to 4 fluorine substituents,
in which phenyl may be substituted by 1 to 3 substituents selected from the group consisting of halogen, (C₁-C₄)-alkyl and (C₁-C₄)-alkoxy, in which azetidine may be substituted by hydroxy,
in which amino may be substituted by 1 or 2 (C₁-C₄)-alkyl substituents, and
in which piperazinyl may be substituted by 1 to 3 substituents independently of one another selected from the group consisting of (C₁-C₄)-alkyl, (C₃-C₇) -cycloalkyl and trifluoromethyl,
in which (C₃-C₇)-alkyl may be substituted by 1 or 2 substituents independently of one another selected from the group consisting of halogen, (C₁-C₆)-alkyl, (C₁-C₄)-alkoxycarbonyl and hydroxycarbonyl,
in which (C₁-C₄)-alkoxy may be substituted by amino,
in which amino may be substituted by 1 or 2 substituents independently of one another selected from (C₁-C₆)-alkyl, (C₁-C₄)-alkylcarbonyl, (C₃-C₆)-cycloalkylsulphonyl and (C₁-C₄)-alkylsulphonyl,
in which (C₁-C₆)-alkyl may be substituted by amino,
and
in which (C₁-C₆) -alkyl may be substituted up to five times by fluorine,
in which phenyl, pyridyl and pyrimidyl may be substituted by 1 or 2 substituents selected from the group consisting of methyl, ethyl and fluorine,
in which
R⁷ and R⁸ each independently of one another represent hydrogen, (C₁-C₄)-alkyl or (C₃-C₇)-cycloalkyl,
or
R³ represents a group of the formula 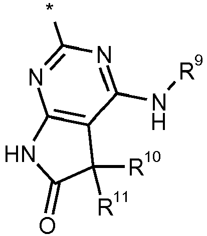 where
* represents the point of attachment to the pyrazolopyridine or the imidazopyrazine,
R⁹ represents hydrogen or (C₁-C₆) -alkyl, where (C₁-C₆)-alkyl may be substituted by amino,
and
in which (C₁-C₆)-alkyl may be substituted up to five times by fluorine,
R¹⁰ represents hydrogen, methyl or ethyl,
R¹¹ represents hydrogen, methyl, ethyl, trifluoromethyl or cyclopropyl,
or
R¹⁰ and R¹¹ together with the carbon atom to
which they are attached form a 3- to 6-membered carbocycle,
R⁴ represents hydrogen,
R⁵ represents hydrogen, halogen, cyano, difluoromethyl, trifluoromethyl, (C₁-C₄)-alkyl, (C₃-C₇)-cycloalkyl, (C₂-C₄) -alkynyl, (C₁-C₄)-alkylamino, difluoromethoxy, trifluoromethoxy, (C₁-C₄)-alkoxy, amino, 4- to 7-membered heterocyclyl or 5- or 6-membered heteroaryl,
R⁶ represents hydrogen, cyano or halogen,
and the *N*-oxides, salts, solvates, salts of the *N-*oxides and solvates of the *N*-oxides and salts thereof.

2. Compound of the formula (I-A) or (I-B) according to Claim 1 in which
A represents CH₂ or CD₂,
R¹ represents cyclohexyl, pyridyl or phenyl, where cyclohexyl may be substituted up to
four times by fluorine, where pyridyl is substituted by 1 or 2
fluorine substituents,
and
where phenyl may be substituted by 1 to 4 substituents independently of one another selected from the group consisting of fluorine, chlorine, cyano, methyl and methoxy,
R² represents hydrogen, (C₁-C₄)-alkyl, cyclopropyl, difluoromethyl or trifluoromethyl,
R³ represents phenyl or 5- or 6-membered heteroaryl,
where phenyl may be substituted by 1 to 3 substituents independently of one another selected from the group consisting of fluorine, chlorine, cyano, trifluoromethyl, difluoromethyl, (C₁-C₆)-alkyl, (C₁-C₄)-alkoxycarbonyl, (C₁-C₄)-alkylcarbonyl, hydroxycarbonyl, -(C=O)NR⁷R⁸, (C₁-C₄)-alkylsulphonyl, (C₃-C₆)-cycloalkylsulphonyl, (C₁-C₄)-alkoxy, trifluoromethoxy, difluoromethoxy, hydroxy, amino, morpholinyl, piperidinyl, pyrrolidinyl, piperazinyl and cyclopropyl,
in which amino may be substituted by 1 or 2 substituents independently of one another selected from (C₁-C₆)-alkyl, (C₁-C₄)-alkylcarbonyl and (C₁-C₄)-alkylsulphonyl,
in which (C₁-C₆)-alkyl may be substituted by 1 or 2 substituents selected from the group consisting of fluorine, trifluoromethoxy, -(C=O)NR⁷R⁸, (C₁-C₄)-alkoxy, (C₃-C₆)-cycloalkyl, morpholinyl, piperidinyl, pyrrolidinyl, piperazinyl, hydroxy and amino,
in which amino may be substituted by 1 or 2 substituents independently of one another selected from (C₁-C₆)-alkyl, (C₁-C₄)-alkylcarbonyl and (C₁-C₄)-alkylsulphonyl,
and in which
R⁷ and R⁸ each independently of one another represent hydrogen, (C₁-C₄)-alkyl or cyclopropyl,
where 5- or 6-membered heteroaryl may be substituted by 1 to 3 substituents independently of one another selected from the group consisting of fluorine, chlorine, cyano, (C₁-C₆)-alkyl, (C₁-C₄)-alkoxy, amino, (C₁-C₄)-alkoxycarbonyl, hydroxycarbonyl,-(C=O)NR⁷R⁸, phenyl, pyridyl, pyrimidyl, pyrrolidinyl, piperidinyl, morpholinyl, piperazinyl and cyclopropyl,
in which (C₁-C₆)-alkyl may be substituted by 1 to 3 substituents selected from the group consisting of fluorine, cyano, hydroxy, amino, trifluoromethyl, difluoromethyl, (C₁-C₄)-alkylsulphonyl, (C₁-C₄)-alkoxycarbonyl, hydroxycarbonyl, -(C=O)NR⁷R⁸, (C₁-C₄)-alkoxy, phenyl, pyridyl, pyrimidyl, 5-membered heteroaryl, (C₃-C₇)-cycloalkyl, morpholinyl, piperidinyl, pyrrolidinyl, piperazinyl, 1,1-dioxidothiomorpholin-4-yl and azetidine,
in which 5-membered heteroaryl may be substituted by 1 to 3 substituents selected from the group consisting of fluorine, chlorine, methyl, ethyl and methoxy,
in which
R⁷ and R⁸ each independently of one another represent hydrogen, methyl, ethyl or cyclopropyl,
in which piperidinyl may be substituted by 1 to 4 fluorine substituents,
in which phenyl may be substituted by 1 to 3 substituents selected from the group consisting of fluorine, methyl, ethyl and methoxy,
in which amino may be substituted by 1 or 2 substituents independently of one another selected from (C₁-C₆)-alkyl and (C₁-C₄)-alkylcarbonyl,
in which (C₁-C₆)-alkyl may be substituted by amino,
in which phenyl, pyridyl and pyrimidyl may be substituted by 1 or 2 substituents selected from the group consisting of methyl, ethyl and fluorine,
and in which
R⁷ and R⁸ each independently of one another represent hydrogen, methyl, ethyl or cyclopropyl,
or
R³ represents a group of the formula 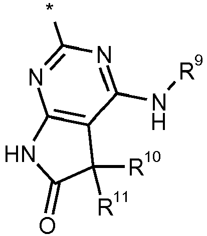 where
* represents the point of attachment to the pyrazolopyridine or the imidazopyrazine,
R⁹ represents hydrogen or (C₁-C₆)-alkyl, where (C₁-C₆)-alkyl may be substituted by amino,
R¹⁰ represents methyl or ethyl,
R¹¹ represents methyl, ethyl, trifluoromethyl or cyclopropyl,
R⁴ represents hydrogen,
R⁵ represents hydrogen, fluorine, chlorine, cyano, difluoromethyl, trifluoromethyl, (C₁-C₄)-alkyl or (C₃-C₅)-cycloalkyl,
R⁶ represents hydrogen,
and the *N*-oxides, salts, solvates, salts of the *N-*oxides and solvates of the *N*-oxides and salts thereof.

3. Compound of the formula (I-A) according to Claim 1 or 2 in which
A represents CH₂ or CD₂,
R¹ represents cyclohexyl, pyridyl or phenyl,
where cyclohexyl may be substituted up to four times by fluorine,
where pyridyl is substituted by 1 or 2 fluorine substituents,
and
where phenyl may be substituted by 1 to 4 substituents independently of one another selected from the group consisting of fluorine, chlorine, cyano, methyl and methoxy,
R² represents hydrogen, (C₁-C₄)-alkyl, cyclopropyl, difluoromethyl or trifluoromethyl,
R³ represents phenyl or 5- or 6-membered heteroaryl,
where phenyl may be substituted by 1 to 3 substituents independently of one another selected from the group consisting of fluorine, chlorine, cyano, trifluoromethyl, difluoromethyl, (C₁-C₆)-alkyl, (C₁-C₄)-alkoxycarbonyl, (C₁-C₄)-alkylcarbonyl, hydroxycarbonyl, -(C=O)NR⁷R⁸, (C₁-C₄)-alkylsulphonyl, (C₃-C₆)-cycloalkylsulphonyl, (C₁-C₄)-alkoxy, trifluoromethoxy, difluoromethoxy, hydroxy, amino, morpholinyl, piperidinyl, pyrrolidinyl, piperazinyl and cyclopropyl,
in which amino may be substituted by 1 or 2 substituents independently of one another selected from (C₁-C₆)-alkyl, (C₁-C₄)-alkylcarbonyl and (C₁-C₄)-alkylsulphonyl,
in which (C₁-C₆)-alkyl may be substituted by 1 or 2 substituents selected from the group consisting of fluorine, trifluoromethoxy, -(C=O)NR⁷R⁸, (C₁-C₄)-alkoxy, (C₃-C₆)-cycloalkyl, morpholinyl, piperidinyl, pyrrolidinyl, piperazinyl, hydroxy and amino,
in which amino may be substituted by 1 or 2 substituents independently of one another selected from (C₁-C₆)-alkyl, (C₁-C₄)-alkylcarbonyl and (C₁-C₄)-alkylsulphonyl,
and in which
R⁷ and R⁸ each independently of one another represent hydrogen, (C₁-C₄)-alkyl or cyclopropyl,
where 5- or 6-membered heteroaryl may be substituted by 1 to 3 substituents independently of one another selected from the group consisting of fluorine, chlorine, cyano, (C₁-C₆)-alkyl, (C₁-C₄)-alkoxy, amino, (C₁-C₄)-alkoxycarbonyl, hydroxycarbonyl,-(C=O)NR⁷R⁸, phenyl, pyridyl, pyrimidyl, pyrrolidinyl, piperidinyl, morpholinyl, piperazinyl and cyclopropyl,
in which (C₁-C₆)-alkyl may be substituted by 1 to 3 substituents selected from the group consisting of fluorine, cyano, hydroxy, amino, trifluoromethyl, difluoromethyl, (C₁-C₄)-alkylsulphonyl, (C₁-C₄)-alkoxycarbonyl, hydroxycarbonyl, -(C=O)NR⁷R⁸, (C₁-C₄)-alkoxy, phenyl, pyridyl, pyrimidyl, 5-membered heteroaryl, (C₃-C₇)-cycloalkyl, morpholinyl, piperidinyl, pyrrolidinyl, piperazinyl, 1,1-dioxidothiomorpholin-4-yl and azetidine,
in which 5-membered heteroaryl may be substituted by 1 to 3 substituents selected from the group consisting of fluorine, chlorine, methyl, ethyl and methoxy,
in which
R⁷ and R⁸ each independently of one another represent hydrogen, methyl, ethyl or cyclopropyl,
in which piperidinyl may be substituted by 1 to 4 fluorine substituents,
in which phenyl may be substituted by 1 to 3 substituents selected from the group consisting of fluorine, methyl, ethyl and methoxy,
in which amino may be substituted by 1 or 2 substituents independently of one another selected from (C₁-C₆)-alkyl and (C₁-C₄)-alkylcarbonyl,
in which (C₁-C₆)-alkyl may be substituted by amino,
in which phenyl, pyridyl and pyrimidyl may be substituted by 1 or 2 substituents selected from the group consisting of methyl, ethyl and fluorine,
and in which
R⁷ and R⁸ each independently of one another represent hydrogen, methyl, ethyl or cyclopropyl,
R⁴ represents hydrogen,
R⁵ represents hydrogen, fluorine, chlorine, cyano, difluoromethyl, trifluoromethyl, (C₁-C₄)-alkyl or (C₃-C₅)-cycloalkyl,
R⁶ represents hydrogen,
and the *N*-oxides, salts, solvates, salts of the *N-*oxides and solvates of the *N*-oxides and salts thereof.

4. Compound of the formula (I-A) according to Claim 1, 2, or 3 in which
A represents CH₂,
R¹ represents phenyl,
where phenyl is substituted up to three times by fluorine,
R² represents methyl,
R³ represents phenyl, pyridyl, pyrimidyl or 4-pyrazolyl,
where phenyl may be substituted by 1 to 3 substituents independently of one another selected from the group consisting of fluorine, (C₁-C₆)-alkyl, hydroxycarbonyl,-(C=O)NR⁷R⁸, methylsulphonyl, ethylsulphonyl, amino, morpholinyl, piperidinyl, pyrrolidinyl and piperazinyl,
in which amino may be substituted by 1 or 2 substituents independently of one another selected from (C₁-C₆)-alkyl, methylcarbonyl, ethylcarbonyl, methylsulphonyl and ethylsulphonyl,
in which (C₁-C₆)-alkyl may be substituted by 1 or 2 substituents selected from the group consisting of fluorine, morpholinyl, piperidinyl, pyrrolidinyl, piperazinyl and amino,
in which amino may be substituted by 1 or 2 substituents independently of one another selected from (C₁-C₄)-alkyl, methylcarbonyl, ethylcarbonyl, methylsulphonyl and ethylsulphonyl,
and in which
R⁷ and R⁸ each independently of one another represent hydrogen or cyclopropyl,
where pyridyl and 4-pyrazolyl may be substituted by 1 or 2 substituents independently of one another selected from the group consisting of fluorine, chlorine, (C₁-C₆)-alkyl, methoxy, amino, hydroxycarbonyl, -(C=O)NR⁷R⁸, phenyl, pyridyl, pyrrolidinyl, piperidinyl, morpholinyl and piperazinyl,
in which (C₁-C₆)-alkyl may be substituted by 1 to 3 substituents selected from the group consisting of fluorine, hydroxy, amino, hydroxycarbonyl, -(C=O)NR⁷R⁸, methoxy, phenyl, pyridyl, pyrazolyl, (C₃-C₇)-cycloalkyl, morpholinyl, piperidinyl, pyrrolidinyl and piperazinyl,
in which pyrazolyl may be substituted by 1 or 2 methyl substituents,
and in which
R⁷ and R⁸ each independently of one another represent hydrogen, methyl, ethyl or cyclopropyl,
in which amino may be substituted by 1 or 2 (C₁-C₆)-alkyl substituents,
in which (C₁-C₆)-alkyl may be substituted by amino,
in which phenyl, pyridyl and pyrimidyl may be substituted by 1 or 2 substituents selected from the group consisting of methyl, ethyl and fluorine,
and in which
R⁷ and R⁸ each independently of one another represent hydrogen, methyl or cyclopropyl,
R⁴ represents hydrogen,
R⁵ represents hydrogen or methyl,
R⁶ represents hydrogen,
and the *N*-oxides, salts, solvates, salts of the *N-*oxides and solvates of the *N*-oxides and salts thereof.

5. Compound of the formula (I-A) according to Claim 1, 2, 3 or 4, in which
A represents CH₂,
R¹ represents a phenyl group of the formula 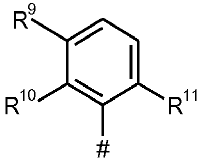 where
# represents the point of attachment to A,
and
R⁹ represents hydrogen or fluorine,
R¹⁰ represents fluorine,
R¹¹ represents fluorine,
R² represents methyl,
R³ represents phenyl, 3-pyridyl, 4-pyridyl or 4-pyrazolyl,
where phenyl in the 3-position may be substituted by fluorine, (C₁-C₆)-alkyl, hydroxycarbonyl, -(C=O)NR⁷R⁸, methylsulphonyl, ethylsulphonyl, amino, morpholinyl, piperidinyl, pyrrolidinyl and piperazinyl,
in which amino may be substituted by 1 or 2 substituents independently of one another selected from (C₁-C₆) -alkyl, methylcarbonyl, ethylcarbonyl, methylsulphonyl and ethylsulphonyl,
in which (C₁-C₆)-alkyl may be substituted by 1 or 2 substituents selected from the group consisting of fluorine, morpholinyl, piperidinyl, pyrrolidinyl, piperazinyl and amino,
in which amino may be substituted by 1 or 2 substituents independently of one another selected from (C₁-C₄)-alkyl, methylcarbonyl, ethylcarbonyl, methylsulphonyl and ethylsulphonyl,
and in which
R⁷ and R⁸ each independently of one another represent hydrogen or cyclopropyl,
where 3-pyridyl and 4-pyridyl may each be substituted by 1 or 2 substituents independently of one another selected from the group consisting of fluorine, chlorine, (C₁-C₆)-alkyl, methoxy, amino, hydroxycarbonyl, -(C=O)NR⁷R⁸, phenyl, pyridyl, pyrrolidinyl, piperidinyl, morpholinyl and piperazinyl,
in which amino may be substituted by 1 or 2 (C₁-C₆)-alkyl substituents,
in which (C₁-C₆)-alkyl may be substituted by amino,
in which phenyl, pyridyl and pyrimidyl may be substituted by 1 or 2 substituents selected from the group consisting of methyl, ethyl and fluorine,
and in which
R⁷ and R⁸ each independently of one another represent hydrogen, methyl or cyclopropyl,
where 4-pyrazolyl may be substituted at the 1-position by (C₁-C₆)-alkyl, phenyl or pyridyl,
in which (C₁-C₆)-alkyl may be substituted by 1 to 3 substituents selected from the group consisting of fluorine, hydroxy, amino, methoxycarbonyl, ethoxycarbonyl, hydroxycarbonyl, -(C=O)NR⁷R⁸, methoxy, phenyl, pyridyl, pyrazolyl, (C₃-C₇)-cycloalkyl, morpholinyl, piperidinyl, pyrrolidinyl and piperazinyl,
in which pyrazolyl may be substituted by 1 to 3 methyl substituents,
in which
R⁷ and R⁸ each independently of one another represent hydrogen, methyl, ethyl or cyclopropyl,
in which phenyl and pyridyl may be substituted by 1 or 2 substituents selected from the group consisting of methyl, ethyl and fluorine,
R⁴ represents hydrogen,
R⁵ represents hydrogen or methyl,
R⁶ represents hydrogen,
and the *N*-oxides, salts, solvates, salts of the *N-*oxides and solvates of the *N*-oxides and salts thereof.

6. Compound of the formula (I-A) according to Claim 1, 2, 3, 4 or 5 in which
A represents CH₂,
R¹ represents a phenyl group of the formula 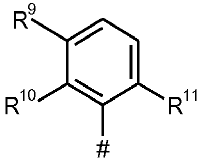 where
# represents the point of attachment to A,
and
R⁹ represents hydrogen or fluorine,
R¹⁰ represents fluorine,
R¹¹ represents fluorine,
R² represents methyl,
R³ represents phenyl, 3-pyridyl, 4-pyridyl or 4-pyrazolyl,
where phenyl in the 3-position may be substituted by (C₁-C₆)-alkyl, hydroxycarbonyl, -(C=O)NR⁷R⁸, methylsulphonyl, ethylsulphonyl, amino and pyrrolidinyl,
in which amino may be substituted by 1 or 2 substituents independently of one another selected from (C₁-C₆)-alkyl, methylcarbonyl and methylsulphonyl, in which (C₁-C₆)-alkyl may be substituted by 1 or 2 substituents selected from the group consisting of fluorine, pyrrolidinyl and amino,
in which amino may be substituted by 1 or 2 substituents independently of one another selected from (C₁-C₄)-alkyl, methylcarbonyl and methylsulphonyl,
and in which
R⁷ and R⁸ each independently of one another represent hydrogen or cyclopropyl,
where 3-pyridyl and 4-pyridyl may each be substituted by 1 or 2 substituents independently of one another selected from the group consisting of fluorine, chlorine, (C₁-C₆)-alkyl, amino, hydroxycarbonyl,-(C=O)NR⁷R⁸, phenyl and piperazinyl,
in which amino may be substituted by 1 or 2 (C₁-C₆)-alkyl substituents,
in which (C₁-C₆)-alkyl may be substituted by amino,
in which phenyl may be substituted by 1 or 2 substituents selected from the group consisting of methyl, ethyl and fluorine,
and in which
R⁷ and R⁸ each represent hydrogen,
where 4-pyrazolyl may be substituted at the 1-position by (C₁-C₆)-alkyl or phenyl,
in which (C₁-C₆)-alkyl may be substituted by 1 to 3 substituents selected from the group consisting of hydroxy, amino, methoxycarbonyl, hydroxycarbonyl,-(C=O)NR⁷R⁸, phenyl, pyrazolyl, (C₃-C₇)-cycloalkyl and morpholinyl,
in which pyrazolyl may be substituted by 1 or 3 methyl substituents,
in which
R⁷ represents hydrogen,
R⁸ represents cyclopropyl,
in which phenyl and pyridyl may be substituted by 1 or 2 fluorine substituents,
R⁴ represents hydrogen,
R⁵ represents hydrogen or methyl,
R⁶ represents hydrogen,
and the *N*-oxides, salts, solvates, salts of the *N-*oxides and solvates of the *N*-oxides and salts thereof.

7. Compound of the formula (I-B) according to Claim 1 or 2 in which
A represents CH₂ or CD₂,
R¹ represents or phenyl,
where phenyl is substituted by 1 to 3 fluorine substituents,
R² represents methyl,
R³ represents 5- or 6-membered heteroaryl,
where 5- or 6-membered heteroaryl may be substituted by 1 to 3 substituents independently of one another selected from the group consisting of (C₁-C₄)-alkyl and amino,
in which (C₁-C₄)-alkyl may be substituted by 1 to 3 substituents selected from the group consisting of hydroxy, amino, (C₁-C₄)-alkoxycarbonyl, hydroxycarbonyl, phenyl, 5-membered heteroaryl, (C₃-C₇)-cycloalkyl, morpholinyl and 1,1-dioxidothiomorpholin-4-yl,
in which amino may be substituted by (C₁-C₆)-alkyl,
in which (C₁-C₆)-alkyl may be substituted by amino,
or
R³ represents a group of the formula 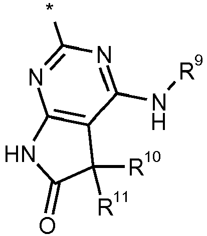 where
* represents the point of attachment to the imidazopyrazine,
R⁹ represents hydrogen or (C₁-C₆)-alkyl, where (C₁-C₆)-alkyl may be substituted by amino,
R¹⁰ represents methyl or ethyl,
R¹¹ represents methyl, ethyl or trifluoromethyl,
R⁴ represents hydrogen,
R⁵ represents hydrogen or methyl,
R⁶ represents hydrogen,
and the *N*-oxides, salts, solvates, salts of the *N-*oxides and solvates of the *N*-oxides and salts thereof.

8. Process for preparing compounds of the formula (I-A) or (I-B) as defined in Claims 1 to 7, **characterized in that**
[A] a compound of the formula (II) 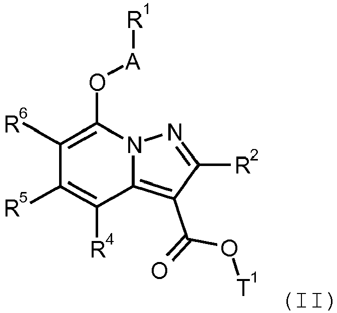
in which A, R¹, R², R⁴, R⁵ and R⁶ each have the meanings given above and
T¹ represents (C₁-C₄)-alkyl or benzyl,
is reacted in an inert solvent in the presence of a suitable base or acid to give a carboxylic acid of the formula (III) 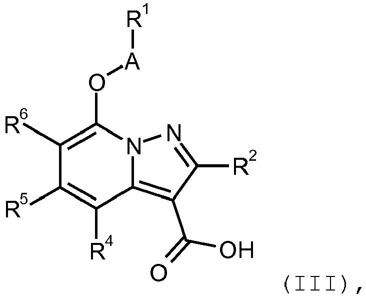 in which A, R¹, R², R⁴, R⁵ and R⁶ each have the meanings given above,
and this is then converted with a halogen equivalent in the presence of a suitable base into a compound of the formula (IV) 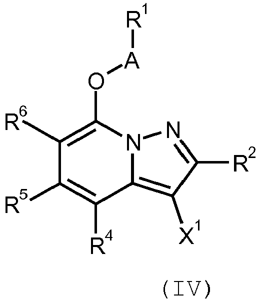
in which A, R¹, R², R⁴, R⁵ and R⁶ each have the meanings given above and
X¹ represents chlorine, bromine or iodine,
and this is subsequently reacted in an inert solvent, in the presence of a suitable transition metal catalyst, with a compound of the formula (V) 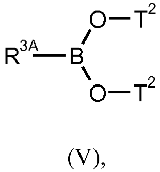 in which
R^{3A} has the meanings given above for R³ and
T² represents hydrogen or (C₁-C₄)-alkyl, or the two T² radicals together form a -C(CH₃)₂-C(CH₃)₂- bridge,
to give a compound of the formula (I-A1) 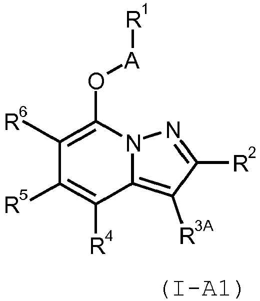 in which A, R¹, R², R⁴, R⁵ and R⁶ each have the meanings given above,
and these compounds are subsequently, if R^{3A} represents 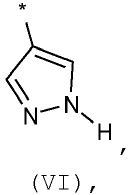 reacted in an inert solvent in the presence of a suitable base with a compound of the formula (VII) 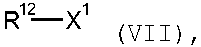 in which
X¹ represents a suitable leaving group, in particular chlorine, bromine, iodine, mesylate, triflate or tosylate,
and
R¹² represents (C₁-C₆)-alkyl,
in which (C₁-C₆)-alkyl may be substituted by 1 to 3 substituents selected from the group consisting of fluorine, cyano, hydroxy, amino, trifluoromethyl, difluoromethyl, (C₁-C₄)-alkylsulphonyl, (C₁-C₄)-alkylcarbonyl, (C₁-C₄)-alkoxycarbonyl, hydroxycarbonyl,-(C=O)NR⁷R⁸ (C₁-C₄)-alkoxy, phenoxy, phenyl, pyridyl, pyrimidyl, 5-membered heteroaryl, (C₃-C₇)-cycloalkyl, morpholinyl, piperidinyl, pyrrolidinyl, piperazinyl, 1,1-dioxidothiomorpholin-4-yl and azetidine,
in which 5-membered heteroaryl may be substituted by 1 to 3 substituents selected from the group consisting of halogen, (C₁-C₄)-alkyl and (C₁-C₄)-alkoxy,
in which
R⁷ and R⁸ each independently of one another represent hydrogen, (C₁-C₄)-alkyl or (C₃-C₇)-cycloalkyl,
in which piperidinyl may be substituted by 1 to 4 fluorine substituents,
in which phenyl may be substituted by 1 to 3 substituents selected from the group consisting of halogen, (C₁-C₄)-alkyl and (C₁-C₄)-alkoxy,
in which azetidine may be substituted by hydroxy,
in which amino may be substituted by 1 or 2 (C₁-C₄)-alkyl substituents, and
in which piperazinyl may be substituted by 1 to 3 substituents independently of one another selected from the group consisting of (C₁-C₄)-alkyl, (C₃-C₇)-cycloalkyl and trifluoromethyl,
to give a compound of the formula (I-A2) 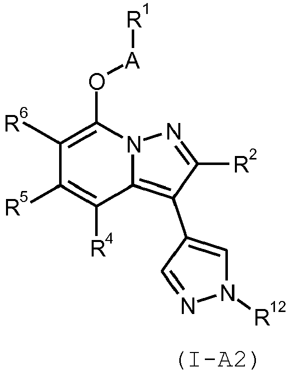 in which A, R¹, R², R⁴, R⁵, R⁶ and R¹² each have the meanings given above, and any protective groups present are subsequently detached, and the resulting compounds of the formula (I-A) are optionally converted with the appropriate (i) solvents and/or (ii) acids or bases to the solvates, salts and/or solvates of the salts thereof.
or
[B] a compound of the formula (VIII) 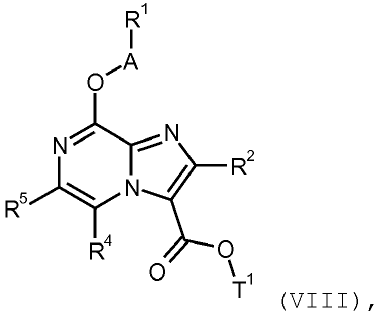
in which A, R¹, R², R⁴ and R⁵ each have the meanings given above and
T¹ represents (C₁-C₄)-alkyl or benzyl,
is reacted in an inert solvent in the presence of a suitable base or acid to give a carboxylic acid of the formula (IX) 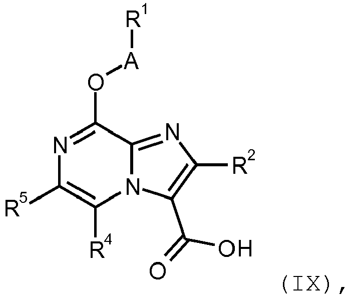 in which A, R¹, R², R⁴ and R⁵ each have the meanings given above,
and this is then converted with a halogen equivalent in the presence of a base into a compound of the formula (X) 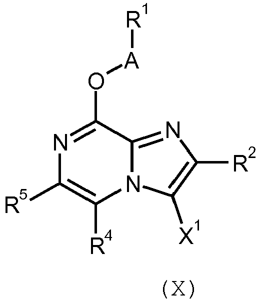
in which A, R¹, R², R⁴ and R⁵ each have the meanings given above and
X¹ represents chlorine, bromine or iodine,
and this is subsequently reacted in an inert solvent, in the presence of a suitable transition metal catalyst, with a compound of the formula (V) 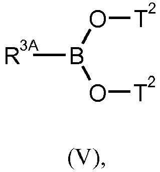 in which
R^{3A} has the meanings given above for R³ and
T² represents hydrogen or (C₁-C₄)-alkyl, or the two T² radicals together form a -C(CH₃)₂-C(CH₃)₂-bridge,
then any protective groups present are detached, and the resulting compounds of the formula (I-B) are optionally converted with the appropriate (i) solvents and/or (ii) acids or bases to the solvates, salts and/or solvates of the salts thereof.

9. Compound of the formula (I-A) or (I-B) as defined in any of Claims 1 to 7 for the treatment and/or prophylaxis of diseases.

10. Use of a compound of the formula (I-A) or (I-B) as defined in any of Claims 1 to 7 for production of a medicament for the treatment and/or prophylaxis of heart failure, angina pectoris, hypertension, pulmonary hypertension, ischaemias, vascular disorders, renal insufficiency, thromboembolic disorders and arteriosclerosis.

11. Medicament comprising a compound of the formula (I-A) or (I-B) as defined in any of Claims 1 to 7 in combination with an inert, non-toxic, pharmaceutically suitable excipient.

12. Medicament comprising a compound of the formula (I-A) or (I-B) as defined in any of Claims 1 to 7 in combination with a further active compound selected from the group consisting of organic nitrates, NO donors, cGMP-PDE inhibitors, antithrombotic agents, hypotensive agents and lipid metabolism modifiers.

13. Medicament according to Claim 11 or 12 for the treatment and/or prophylaxis of heart failure, angina pectoris, hypertension, pulmonary hypertension, ischaemias, vascular disorders, kidney failure, thromboembolic disorders and arteriosclerosis.

## Revendications

1. Composé de formule (I-A) ou (I-B), dans lequel 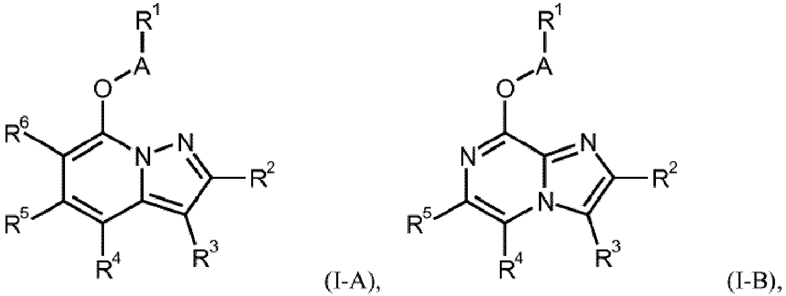
A représente CH₂, CD₂ ou CH(CH₃),
R¹ représente alkyle en (C₄-C₆), cycloalkyle en (C₃-C₇), pyridyle ou phényle,
l'alkyle en (C₄-C₆) pouvant être substitué jusqu'à six fois avec fluor,
le cycloalkyle en (C₃-C₇) pouvant être substitué avec 1 à 4 substituants choisis indépendamment les uns des autres dans le groupe constitué par fluor, trifluorométhyle et alkyle en (C₁-C₄),
le pyridyle étant substitué avec 1 ou 2 substituants choisis indépendamment l'un de l'autre dans le groupe constitué par halogène, cyano et alkyle en (C₁-C₄),
et
le phényle pouvant être substitué avec 1 à 4 substituants choisis indépendamment les uns des autres dans le groupe constitué par halogène, cyano, monofluorométhyle, difluorométhyle, trifluorométhyle, alkyle en (C₁-C₄) et alcoxy en (C₁-C₄),
R² représente hydrogène, alkyle en (C₁-C₄), alcoxyméthyle en (C₁-C₄), cyclopropyle, cyclobutyle, monofluorométhyle, difluorométhyle ou trifluorométhyle,
R³ représente phényle ou hétéroaryle de 5 à 10 chaînons,
le phényle pouvant être substitué avec 1 à 3 substituants choisis indépendamment les uns des autres dans le groupe constitué par halogène, cyano, trifluorométhyle, difluorométhyle, alkyle en (C₁-C₆), alcoxycarbonyle en (C₁-C₄), alkylcarbonyle en (C₁-C₄), hydroxycarbonyle, -(C=O)NR⁷R⁸, alkylsulfonyle en (C₁-C₄), cycloalkylsulfonyle en (C₃-C₆), alkylthio en (C₁-C₄), alcoxy en (C₁-C₄), trifluorométhoxy, difluorométhoxy, phénoxy, hydroxy, amino, morpholinyle, pipéridinyle, pyrrolidinyle, pipérazinyle et cycloalkyle en (C₃-C₆),
l'amino pouvant être substitué avec 1 ou 2 substituants choisis indépendamment l'un de l'autre parmi alkyle en (C₁-C₆), alkylcarbonyle en (C₁-C₄), cycloalkylsulfonyle en (C₃-C₆), alkylsulfonyle en (C₁-C₄) et méthoxy-alkyle en (C₁-C₄),
l'alkyle en (C₁-C₆) pouvant être substitué avec amino,
l'alkyle en (C₁-C₆) pouvant être substitué avec 1 ou 2 substituants choisis dans le groupe constitué par fluor, trifluorométhoxy, -(C=O)NR⁷R⁸, alcoxy en (C₁-C₄), cycloalkyle en (C₃-C₆), morpholinyle, pipéridinyle, pyrrolidinyle, pipérazinyle, hydroxy et amino,
l'amino pouvant être substitué avec 1 ou 2 substituants choisis indépendamment l'un de l'autre parmi alkyle en (C₁-C₆), alkylcarbonyle en (C₁-C₄), alcoxycarbonyle en (C₁-C₄), cycloalkylsulfonyle en (C₃-C₆), alkylsulfonyle en (C₁-C₄) et méthoxy-alkyle en (C₁-C₄),
et
R⁷ et R⁸ représentant chacun indépendamment l'un de l'autre hydrogène, alkyle en (C₁-C₄) ou cycloalkyle en (C₃-C₇),
l'hétéroaryle de 5 à 10 chaînons pouvant être substitué avec 1 à 3 substituants choisis indépendamment les uns des autres dans le groupe constitué par fluor, chlore, cyano, alkyle en (C₁-C₆), alcoxy en (C₁-C₄), amino, alcoxycarbonyle en (C₁-C₄), hydroxycarbonyle, -(C=O)NR⁷R⁸, phényle, pyridyle, pyrimidyle, 1,3-thiazol-5-yle, pyrrolidinyle, pipéridinyle, morpholinyle, pipérazinyle et cycloalkyle en (C₃-C₇),
l'alkyle en (C₁-C₆) pouvant être substitué avec 1 à 3 substituants choisis dans le groupe constitué par fluor, cyano, hydroxy, amino, trifluorométhyle, difluorométhyle, alkylsulfonyle en (C₁-C₄), alkylcarbonyle en (C₁-C₄), alcoxycarbonyle en (C₁-C₄), hydroxycarbonyle, -(C=O)NR⁷R⁸, alcoxy en (C₁-C₄), phénoxy, phényle, pyridyle, pyrimidyle, hétéroaryle à 5 chaînons, cycloalkyle en (C₃-C₇), morpholinyle, pipéridinyle, pyrrolidinyle, pipérazinyle, 1,1-dioxydothiomorpholin-4-yle et azétidine,
l'hétéroaryle à 5 chaînons pouvant être substitué avec 1 à 3 substituants choisis dans le groupe constitué par halogène, alkyle en (C₁-C₄) et alcoxy en (C₁-C₄),
R⁷ et R⁸ représentant chacun indépendamment l'un de l'autre hydrogène, alkyle en (C₁-C₄) ou cycloalkyle en (C₃-C₇),
le pipéridinyle pouvant être substitué avec 1 à 4 substituants fluor,
le phényle pouvant être substitué avec 1 à 3 substituants choisis dans le groupe constitué par halogène, alkyle en (C₁-C₄) et alcoxy en (C₁-C₄),
l'azétidine pouvant être substituée avec hydroxy,
l'amino pouvant être substitué avec 1 ou 2 substituants alkyle en (C₁-C₄),
et
le pipérazinyle pouvant être substitué avec 1 à 3 substituants choisis indépendamment les uns des autres dans le groupe constitué par alkyle en (C₁-C₄), cycloalkyle en (C₃-C₇) et trifluorométhyle,
le cycloalkyle en (C₃-C₇) pouvant être substitué avec 1 ou 2 substituants choisis indépendamment l'un de l'autre dans le groupe constitué par halogène, alkyle en (C₁-C₆), alcoxycarbonyle en (C₁-C₄) et hydroxycarbonyle,
l'alcoxy en (C₁-C₄) pouvant être substitué avec amino,
l'amino pouvant être substitué avec 1 ou 2 substituants choisis indépendamment l'un de l'autre parmi alkyle en (C₁-C₆), alkylcarbonyle en (C₁-C₄), cycloalkylsulfonyle en (C₃-C₆) et alkylsulfonyle en (C₁-C₄),
l'alkyle en (C₁-C₆) pouvant être substitué avec amino,
et
l'alkyle en (C₁-C₆) pouvant être substitué jusqu'à cinq fois avec fluor,
le phényle, le pyridyle et le pyrimidyle pouvant être substitués avec 1 ou 2 substituants choisis dans le groupe constitué par méthyle, éthyle et fluor,
R⁷ et R⁸ représentant chacun indépendamment l'un de l'autre hydrogène, alkyle en (C₁-C₄) ou cycloalkyle en (C₃-C₇),
ou
R³ représente un groupe de formule 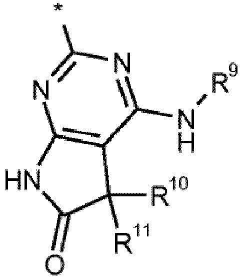 dans laquelle
* représente l'emplacement de liaison à la pyrazolopyridine ou à l'imidazopyrazine,
R⁹ représente hydrogène ou alkyle en (C₁-C₆),
l'alkyle en (C₁-C₆) pouvant être substitué avec amino,
et
l'alkyle en (C₁-C₆) pouvant être substitué jusqu'à cinq fois avec fluor,
R¹⁰ représente hydrogène, méthyle ou éthyle,
R¹¹ représente hydrogène, méthyle, éthyle, trifluorométhyle ou cyclopropyle,
ou
R¹⁰ et R¹¹ forment ensemble avec l'atome de carbone auquel ils sont reliés un carbocycle de 3 à 6 chaînons,
R⁴ représente hydrogène,
R⁵ représente hydrogène, halogène, cyano, difluorométhyle, trifluorométhyle, alkyle en (C₁-C₄), cycloalkyle en (C₃-C₇), alcynyle en (C₂-C₄), alkylamino en (C₁-C₄), difluorométhoxy, trifluorométhoxy, alcoxy en (C₁-C₄), amino, hétérocyclyle de 4 à 7 chaînons ou hétéroaryle à 5 ou 6 chaînons,
R⁶ représente hydrogène, cyano ou halogène,
ainsi que ses *N*-oxydes, sels, solvates, sels des *N-*oxydes et solvates des *N*-oxydes et des sels.

2. Composé de formule (I-A) ou (I-B) selon la revendication 1, dans lequel
A représente CH₂ ou CD₂,
R¹ représente cyclohexyle, pyridyle ou phényle,
le cyclohexyle pouvant être substitué jusqu'à quatre fois avec fluor,
le pyridyle étant substitué avec 1 ou 2 substituants fluor,
et
le phényle pouvant être substitué avec 1 à 4 substituants choisis indépendamment les uns des autres dans le groupe constitué par fluor, chlore, cyano, méthyle et méthoxy,
R² représente hydrogène, alkyle en (C₁-C₄), cyclopropyle, difluorométhyle ou trifluorométhyle,
R³ représente phényle ou hétéroaryle à 5 ou 6 chaînons,
le phényle pouvant être substitué avec 1 à 3 substituants choisis indépendamment les uns des autres dans le groupe constitué par fluor, chlore, cyano, trifluorométhyle, difluorométhyle, alkyle en (C₁-C₆), alcoxycarbonyle en (C₁-C₄), alkylcarbonyle en (C₁-C₄), hydroxycarbonyle, -(C=O)NR⁷R⁸, alkylsulfonyle en (C₁-C₄), cycloalkylsulfonyle en (C₃-C₆), alcoxy en (C₁-C₄), trifluorométhoxy, difluorométhoxy, hydroxy, amino, morpholinyle, pipéridinyle, pyrrolidinyle, pipérazinyle et cyclopropyle,
l'amino pouvant être substitué avec 1 ou 2 substituants choisis indépendamment l'un de l'autre parmi alkyle en (C₁-C₆), alkylcarbonyle en (C₁-C₄) et alkylsulfonyle en (C₁-C₄),
l'alkyle en (C₁-C₆) pouvant être substitué avec 1 ou 2 substituants choisis dans le groupe constitué par fluor, trifluorométhoxy, -(C=O)NR⁷R⁸, alcoxy en (C₁-C₄), cycloalkyle en (C₃-C₆), morpholinyle, pipéridinyle, pyrrolidinyle, pipérazinyle, hydroxy et amino,
l'amino pouvant être substitué avec 1 ou 2 substituants choisis indépendamment l'un de l'autre parmi alkyle en (C₁-C₆), alkylcarbonyle en (C₁-C₄) et alkylsulfonyle en (C₁-C₄),
et
R⁷ et R⁸ représentant chacun indépendamment l'un de l'autre hydrogène, alkyle en (C₁-C₄) ou cyclopropyle,
l'hétéroaryle à 5 ou 6 chaînons pouvant être substitué avec 1 à 3 substituants choisis indépendamment les uns des autres dans le groupe constitué par fluor, chlore, cyano, alkyle en (C₁-C₆), alcoxy en (C₁-C₄), amino, alcoxycarbonyle en (C₁-C₄), hydroxycarbonyle, -(C=O)NR⁷R⁸, phényle, pyridyle, pyrimidyle, pyrrolidinyle, pipéridinyle, morpholinyle, pipérazinyle et cyclopropyle,
l'alkyle en (C₁-C₆) pouvant être substitué avec 1 à 3 substituants choisis dans le groupe constitué par fluor, cyano, hydroxy, amino, trifluorométhyle, difluorométhyle, alkylsulfonyle en (C₁-C₄), alcoxycarbonyle en (C₁-C₄), hydroxycarbonyle,-(C=O)NR⁷R⁸, alcoxy en (C₁-C₄), phényle, pyridyle, pyrimidyle, hétéroaryle à 5 chaînons, cycloalkyle en (C₃-C₇), morpholinyle, pipéridinyle, pyrrolidinyle, pipérazinyle, 1,1-dioxydothiomorpholin-4-yle et azétidine,
l'hétéroaryle à 5 chaînons pouvant être substitué avec 1 à 3 substituants choisis dans le groupe constitué par fluor, chlore, méthyle, éthyle et méthoxy,
R⁷ et R⁸ représentant chacun indépendamment l'un de l'autre hydrogène, méthyle, éthyle ou cyclopropyle,
le pipéridinyle pouvant être substitué avec 1 à 4 substituants fluor,
le phényle pouvant être substitué avec 1 à 3 substituants choisis dans le groupe constitué par fluor, méthyle, éthyle et méthoxy,
l'amino pouvant être substitué avec 1 ou 2 substituants choisis indépendamment l'un de l'autre parmi alkyle en (C₁-C₆) ou alkylcarbonyle en (C₁-C₄),
l'alkyle en (C₁-C₆) pouvant être substitué avec amino,
le phényle, le pyridyle et le pyrimidyle pouvant être substitués avec 1 ou 2 substituants choisis dans le groupe constitué par méthyle, éthyle et fluor,
et
R⁷ et R⁸ représentant chacun indépendamment l'un de l'autre hydrogène, méthyle, éthyle ou cyclopropyle,
ou
R³ représente un groupe de formule 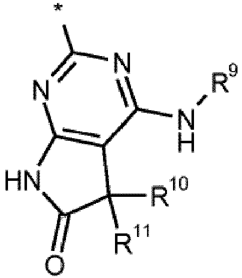 dans laquelle
* représente l'emplacement de liaison à la pyrazolopyridine ou à l'imidazopyrazine,
R⁹ représente hydrogène ou alkyle en (C₁-C₆),
l'alkyle en (C₁-C₆) pouvant être substitué avec amino,
R¹⁰ représente méthyle ou éthyle,
R¹¹ représente méthyle, éthyle, trifluorométhyle ou cyclopropyle,
R⁴ représente hydrogène,
R⁵ représente hydrogène, fluor, chlore, cyano, difluorométhyle, trifluorométhyle, alkyle en (C₁-C₄), cycloalkyle en (C₃-C₅),
R⁶ représente hydrogène,
ainsi que ses *N*-oxydes, sels, solvates, sels des *N-*oxydes et solvates des *N*-oxydes et des sels.

3. Composé de formule (I-A) selon la revendication 1 ou 2, dans lequel
A représente CH₂ ou CD₂,
R¹ représente cyclohexyle, pyridyle ou phényle,
le cyclohexyle pouvant être substitué jusqu'à quatre fois avec fluor,
le pyridyle étant substitué avec 1 ou 2 substituants fluor,
et
le phényle pouvant être substitué avec 1 à 4 substituants choisis indépendamment les uns des autres dans le groupe constitué par fluor, chlore, cyano, méthyle et méthoxy,
R² représente hydrogène, alkyle en (C₁-C₄), cyclopropyle, difluorométhyle ou trifluorométhyle,
R³ représente phényle ou hétéroaryle à 5 ou 6 chaînons,
le phényle pouvant être substitué avec 1 à 3 substituants choisis indépendamment les uns des autres dans le groupe constitué par fluor, chlore, cyano, trifluorométhyle, difluorométhyle, alkyle en (C₁-C₆), alcoxycarbonyle en (C₁-C₄), alkylcarbonyle en (C₁-C₄), hydroxycarbonyle, -(C=O)NR⁷R⁸, alkylsulfonyle en (C₁-C₄), cycloalkylsulfonyle en (C₃-C₆), alcoxy en (C₁-C₄), trifluorométhoxy, difluorométhoxy, hydroxy, amino, morpholinyle, pipéridinyle, pyrrolidinyle, pipérazinyle et cyclopropyle,
l'amino pouvant être substitué avec 1 ou 2 substituants choisis indépendamment l'un de l'autre parmi alkyle en (C₁-C₆), alkylcarbonyle en (C₁-C₄) et alkylsulfonyle en (C₁-C₄),
l'alkyle en (C₁-C₆) pouvant être substitué avec 1 ou 2 substituants choisis dans le groupe constitué par fluor, trifluorométhoxy, -(C=O)NR⁷R⁸, alcoxy en (C₁-C₄), cycloalkyle en (C₃-C₆), morpholinyle, pipéridinyle, pyrrolidinyle, pipérazinyle, hydroxy et amino,
l'amino pouvant être substitué avec 1 ou 2 substituants choisis indépendamment l'un de l'autre parmi alkyle en (C₁-C₆), alkylcarbonyle en (C₁-C₄) et alkylsulfonyle en (C₁-C₄),
et
R⁷ et R⁸ représentant chacun indépendamment l'un de l'autre hydrogène, alkyle en (C₁-C₄) ou cyclopropyle,
l'hétéroaryle à 5 ou 6 chaînons pouvant être substitué avec 1 à 3 substituants choisis indépendamment les uns des autres dans le groupe constitué par fluor, chlore, cyano, alkyle en (C₁-C₆), alcoxy en (C₁-C₄), amino, alcoxycarbonyle en (C₁-C₄), hydroxycarbonyle, -(C=O)NR⁷R⁸, phényle, pyridyle, pyrimidyle, pyrrolidinyle, pipéridinyle, morpholinyle, pipérazinyle et cyclopropyle,
l'alkyle en (C₁-C₆) pouvant être substitué avec 1 à 3 substituants choisis dans le groupe constitué par fluor, cyano, hydroxy, amino, trifluorométhyle, difluorométhyle, alkylsulfonyle en (C₁-C₄), alcoxycarbonyle en (C₁-C₄), hydroxycarbonyle,-(C=O)NR⁷R⁸, alcoxy en (C₁-C₄), phényle, pyridyle, pyrimidyle, hétéroaryle à 5 chaînons, cycloalkyle en (C₃-C₇), morpholinyle, pipéridinyle, pyrrolidinyle, pipérazinyle, 1,1-dioxydothiomorpholin-4-yle et azétidine,
l'hétéroaryle à 5 chaînons pouvant être substitué avec 1 à 3 substituants choisis dans le groupe constitué par fluor, chlore, méthyle, éthyle et méthoxy,
R⁷ et R⁸ représentant chacun indépendamment l'un de l'autre hydrogène, méthyle, éthyle ou cyclopropyle,
le pipéridinyle pouvant être substitué avec 1 à 4 substituants fluor,
le phényle pouvant être substitué avec 1 à 3 substituants choisis dans le groupe constitué par fluor, méthyle, éthyle et méthoxy,
l'amino pouvant être substitué avec 1 ou 2 substituants choisis indépendamment l'un de l'autre parmi alkyle en (C₁-C₆) ou alkylcarbonyle en (C₁-C₄),
l'alkyle en (C₁-C₆) pouvant être substitué avec amino,
le phényle, le pyridyle et le pyrimidyle pouvant être substitués avec 1 ou 2 substituants choisis dans le groupe constitué par méthyle, éthyle et fluor,
et
R⁷ et R⁸ représentant chacun indépendamment l'un de l'autre hydrogène, méthyle, éthyle ou cyclopropyle,
R⁴ représente hydrogène,
R⁵ représente hydrogène, fluor, chlore, cyano, difluorométhyle, trifluorométhyle, alkyle en (C₁-C₄), cycloalkyle en (C₃-C₅),
R⁶ représente hydrogène,
ainsi que ses N-oxydes, sels, solvates, sels des N-oxydes et solvates des N-oxydes et des sels.

4. Composé de formule (I-A) selon la revendication 1, 2 ou 3, dans lequel
A représente CH₂,
R¹ représente phényle,
le phényle pouvant être substitué jusqu'à trois fois avec fluor,
R² représente méthyle,
R³ représente phényle, pyridyle, pyrimidyle ou 4-pyrazolyle,
le phényle pouvant être substitué avec 1 à 3 substituants choisis indépendamment les uns des autres dans le groupe constitué par fluor, alkyle en (C₁-C₆), hydroxycarbonyle, -(C=O)NR⁷R⁸, méthylsulfonyle, éthylsulfonyle, amino, morpholinyle, pipéridinyle, pyrrolidinyle et pipérazinyle,
l'amino pouvant être substitué avec 1 ou 2 substituants choisis indépendamment l'un de l'autre parmi alkyle en (C₁-C₆), méthylcarbonyle, éthylcarbonyle, méthylsulfonyle et éthylsulfonyle,
l'alkyle en (C₁-C₆) pouvant être substitué avec 1 ou 2 substituants choisis dans le groupe constitué par fluor, morpholinyle, pipéridinyle, pyrrolidinyle, pipérazinyle et amino,
l'amino pouvant être substitué avec 1 ou 2 substituants choisis indépendamment l'un de l'autre parmi alkyle en (C₁-C₄), méthylcarbonyle, éthylcarbonyle, méthylsulfonyle et éthylsulfonyle,
et
R⁷ et R⁸ représentant chacun indépendamment l'un de l'autre hydrogène ou cyclopropyle,
le pyridyle et le 4-pyrazolyle pouvant être substitués avec 1 ou 2 substituants choisis indépendamment l'un de l'autre dans le groupe constitué par fluor, chlore, alkyle en (C₁-C₆), méthoxy, amino, hydroxycarbonyle, -(C=O)NR⁷R⁸, phényle, pyridyle, pyrrolidinyle, pipéridinyle, morpholinyle et pipérazinyle,
l'alkyle en (C₁-C₆) pouvant être substitué avec 1 à 3 substituants choisis dans le groupe constitué par fluor, hydroxy, amino, hydroxycarbonyle, -(C=O)NR⁷R⁸, méthoxy, phényle, pyridyle, pyrazolyle, cycloalkyle en (C₃-C₇), morpholinyle, pipéridinyle, pyrrolidinyle et pipérazinyle,
le pyrazolyle pouvant être substitué avec 1 ou 2 substituants méthyle,
et
R⁷ et R⁸ représentant chacun indépendamment l'un de l'autre hydrogène, méthyle, éthyle ou cyclopropyle,
l'amino pouvant être substitué avec 1 ou 2 substituants alkyle en (C₁-C₆),
l'alkyle en (C₁-C₆) pouvant être substitué avec amino,
le phényle, le pyridyle et le pyrimidyle pouvant être substitués avec 1 ou 2 substituants choisis dans le groupe constitué par méthyle, éthyle et fluor,
et
R⁷ et R⁸ représentant chacun indépendamment l'un de l'autre hydrogène, méthyle ou cyclopropyle,
R⁴ représente hydrogène,
R⁵ représente hydrogène ou méthyle,
R⁶ représente hydrogène,
ainsi que ses *N*-oxydes, sels, solvates, sels des *N-*oxydes et solvates des *N*-oxydes et des sels.

5. Composé de formule (I-A) selon la revendication 1, 2, 3 ou 4, dans lequel
A représente CH₂,
R¹ représente un groupe phényle de formule 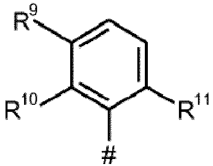 dans laquelle
# représente l'emplacement de liaison à A,
et
R⁹ représente hydrogène ou fluor,
R¹⁰ représente fluor,
R¹¹ représente fluor,
R² représente méthyle,
R³ représente phényle, 3-pyridyle, 4-pyridyle ou 4-pyrazolyle,
le phényle pouvant être substitué en position 3 avec fluor, alkyle en (C₁-C₆), hydroxycarbonyle,-(C=O)NR⁷R⁸, méthylsulfonyle, éthylsulfonyle, amino, morpholinyle, pipéridinyle, pyrrolidinyle et pipérazinyle
l'amino pouvant être substitué avec 1 ou 2 substituants choisis indépendamment l'un de l'autre parmi alkyle en (C₁-C₆), méthylcarbonyle, éthylcarbonyle, méthylsulfonyle et éthylsulfonyle,
l'alkyle en (C₁-C₆) pouvant être substitué avec 1 ou 2 substituants choisis dans le groupe constitué par fluor, morpholinyle, pipéridinyle, pyrrolidinyle, pipérazinyle et amino,
l'amino pouvant être substitué avec 1 ou 2 substituants choisis indépendamment l'un de l'autre parmi alkyle en (C₁-C₄), méthylcarbonyle, éthylcarbonyle, méthylsulfonyle et éthylsulfonyle,
et
R⁷ et R⁸ représentant chacun indépendamment l'un de l'autre hydrogène ou cyclopropyle,
le 3-pyridyle et le 4-pyridyle pouvant chacun être substitués avec 1 ou 2 substituants choisis indépendamment l'un de l'autre dans le groupe constitué par fluor, chlore, alkyle en (C₁-C₆), méthoxy, amino, hydroxycarbonyle, -(C=O)NR⁷R⁸, phényle, pyridyle, pyrrolidinyle, pipéridinyle, morpholinyle et pipérazinyle,
l'amino pouvant être substitué avec 1 ou 2 substituants alkyle en (C₁-C₆),
l'alkyle en (C₁-C₆) pouvant être substitué avec amino,
le phényle, le pyridyle et le pyrimidyle pouvant être substitués avec 1 ou 2 substituants choisis dans le groupe constitué par méthyle, éthyle et fluor,
et
R⁷ et R⁸ représentant chacun indépendamment l'un de l'autre hydrogène, méthyle ou cyclopropyle,
le 4-pyrazolyle pouvant être substitué en position 1 avec alkyle en (C₁-C₆), phényle ou pyridyle,
l'alkyle en (C₁-C₆) pouvant être substitué avec 1 à 3 substituants choisis dans le groupe constitué par fluor, hydroxy, amino, méthoxycarbonyle, éthoxycarbonyle, hydroxycarbonyle, -(C=O)NR⁷R⁸, méthoxy, phényle, pyridyle, pyrazolyle, cycloalkyle en (C₃-C₇), morpholinyle, pipéridinyle, pyrrolidinyle et pipérazinyle,
le pyrazolyle pouvant être substitué avec 1 à 3 substituants méthyle,
R⁷ et R⁸ représentant chacun indépendamment l'un de l'autre hydrogène, méthyle, éthyle ou cyclopropyle,
le phényle et le pyridyle pouvant être substitués avec 1 ou 2 substituants choisis dans le groupe constitué par méthyle, éthyle et fluor,
R⁴ représente hydrogène,
R⁵ représente hydrogène ou méthyle,
R⁶ représente hydrogène,
ainsi que ses *N*-oxydes, sels, solvates, sels des *N-*oxydes et solvates des *N*-oxydes et des sels.

6. Composé de formule (I-A) selon la revendication 1, 2, 3, 4 ou 5, dans lequel
A représente CH₂,
R¹ représente un groupe phényle de formule 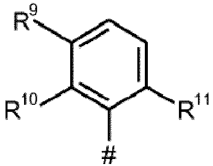 dans laquelle
# représente l'emplacement de liaison à A,
et
R⁹ représente hydrogène ou fluor,
R¹⁰ représente fluor,
R¹¹ représente fluor,
R² représente méthyle,
R³ représente phényle, 3-pyridyle, 4-pyridyle ou 4-pyrazolyle,
le phényle pouvant être substitué en position 3 avec alkyle en (C₁-C₆), hydroxycarbonyle, -(C=O)NR⁷R⁸, méthylsulfonyle, éthylsulfonyle, amino et pyrrolidinyle,
l'amino pouvant être substitué avec 1 ou 2 substituants choisis indépendamment l'un de l'autre parmi alkyle en (C₁-C₆), méthylcarbonyle et méthylsulfonyle,
l'alkyle en (C₁-C₆) pouvant être substitué avec 1 ou 2 substituants choisis dans le groupe constitué par fluor, pyrrolidinyle et amino,
l'amino pouvant être substitué avec 1 ou 2 substituants choisis indépendamment l'un de l'autre parmi alkyle en (C₁-C₄), méthylcarbonyle et méthylsulfonyle,
et
R⁷ et R⁸ représentant chacun indépendamment l'un de l'autre hydrogène ou cyclopropyle,
le 3-pyridyle et le 4-pyridyle pouvant chacun être substitués avec 1 ou 2 substituants choisis indépendamment l'un de l'autre dans le groupe constitué par fluor, chlore, alkyle en (C₁-C₆), amino, hydroxycarbonyle, -(C=O)NR⁷R⁸, phényle et pipérazinyle,
l'amino pouvant être substitué avec 1 ou 2 substituants alkyle en (C₁-C₆),
l'alkyle en (C₁-C₆) pouvant être substitué avec amino,
le phényle pouvant être substitué avec 1 ou 2 substituants choisis dans le groupe constitué par méthyle, éthyle et fluor,
et
R⁷ et R⁸ représentant chacun indépendamment l'un de l'autre hydrogène,
le 4-pyrazolyle pouvant être substitué en position 1 avec alkyle en (C₁-C₆) ou phényle,
l'alkyle en (C₁-C₆) pouvant être substitué avec 1 à 3 substituants choisis dans le groupe constitué par hydroxy, amino, méthoxycarbonyle, hydroxycarbonyle,-(C=O)NR⁷R⁸, phényle, pyrazolyle, cycloalkyle en (C₃-C₇) et morpholinyle,
le pyrazolyle pouvant être substitué avec 1 à 3 substituants méthyle,
R⁷ représentant hydrogène,
R⁸ représentant cyclopropyle,
le phényle et le pyridyle pouvant être substitués avec 1 ou 2 substituants fluor,
R⁴ représente hydrogène,
R⁵ représente hydrogène ou méthyle,
R⁶ représente hydrogène,
ainsi que ses *N*-oxydes, sels, solvates, sels des *N-*oxydes et solvates des *N*-oxydes et des sels.

7. Composé de formule (I-B) selon la revendication 1 ou 2, dans lequel
A représente CH₂ ou CD₂,
R¹ représente ou phényle,
le phényle pouvant être substitué avec 1 à 3 substituants fluor,
R² représente méthyle,
R³ représente hétéroaryle à 5 ou 6 chaînons,
l'hétéroaryle à 5 ou 6 chaînons pouvant être substitué avec 1 à 3 substituants choisis indépendamment les uns des autres dans le groupe constitué par alkyle en (C₁-C₄) et amino,
l'alkyle en (C₁-C₄) pouvant être substitué avec 1 à 3 substituants choisis dans le groupe constitué par hydroxy, amino, alcoxycarbonyle en (C₁-C₄), hydroxycarbonyle, phényle, hétéroaryle à 5 chaînons, cycloalkyle en (C₃-C₇), morpholinyle et 1,1-dioxydothiomorpholin-4-yle,
l'amino pouvant être substitué alkyle en (C₁-C₆),
l'alkyle en (C₁-C₆) pouvant être substitué avec amino,
ou
R³ représente un groupe de formule 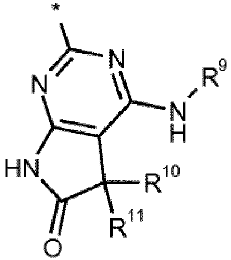 dans laquelle
* représente l'emplacement de liaison à l'imidazopyrazine,
R⁹ représente hydrogène ou alkyle en (C₁-C₆),
l'alkyle en (C₁-C₆) pouvant être substitué avec amino,
R¹⁰ représente méthyle ou éthyle,
R¹¹ représente méthyle, éthyle ou trifluorométhyle,
R⁴ représente hydrogène,
R⁵ représente hydrogène ou méthyle,
R⁶ représente hydrogène,
ainsi que ses *N*-oxydes, sels, solvates, sels des *N-*oxydes et solvates des *N*-oxydes et des sels.

8. Procédé de fabrication de composés de formule (I-A) ou (I-B), tels que définis dans les revendications 1 à 7, **caractérisé en ce que**
[A] un composé de formule (II) 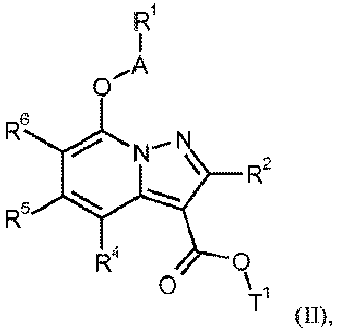
dans laquelle A, R¹, R², R⁴, R⁵ et R⁶ ont chacun les significations indiquées précédemment, et
T¹ représente alkyle en (C₁-C₄) ou benzyle,
est mis en réaction dans un solvant inerte en présence d'une base ou d'un acide approprié pour former un acide carboxylique de formule (III) 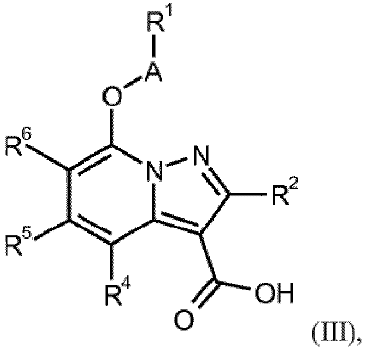 dans laquelle A, R¹, R², R⁴, R⁵ et R⁶ ont chacun les significations indiquées précédemment,
puis celui-ci est transformé avec un équivalent d'halogène en présence d'une base appropriée en un composé de formule (IV) 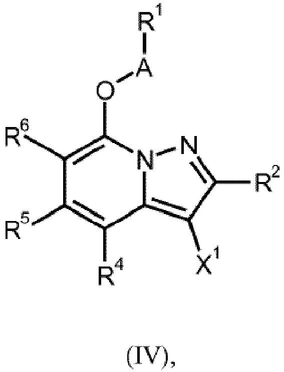
dans laquelle A, R¹, R², R⁴, R⁵ et R⁶ ont chacun les significations indiquées précédemment,
X¹ représente chlore, brome ou iode,
puis celui-ci est mis en réaction dans un solvant inerte en présence d'un catalyseur de métal de transition approprié avec un composé de formule (V) 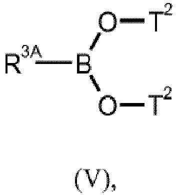 dans laquelle
R^{3A} a les significations indiquées précédemment pour R³, et
T² représente hydrogène ou alkyle en (C₁-C₄), ou les deux radicaux T² forment ensemble un pont -C(CH₃)₂-C(CH₃)₂-,
pour former un composé de formule (I-A1) 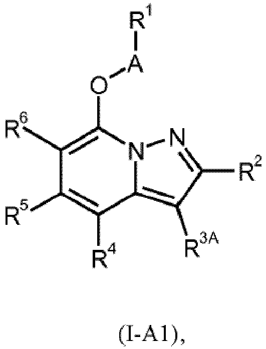 dans laquelle A, R¹, R², R⁴, R⁵ et R⁶ ont chacun les significations indiquées précédemment,
puis, lorsque R^{3A} représente 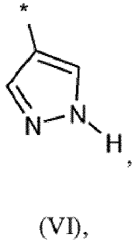 ces composés sont mis en réaction dans un solvant inerte en présence d'une base appropriée avec un composé de formule (VII)
R¹²-X¹ (VII),
dans laquelle
X¹ représente un groupe partant approprié, notamment chlore, brome, iode, mésylate, triflate ou tosylate,
et
R¹² représente alkyle en (C₁-C₆),
l'alkyle en (C₁-C₆) pouvant être substitué avec 1 à 3 substituants choisis dans le groupe constitué par fluor, cyano, hydroxy, amino, trifluorométhyle, difluorométhyle, alkylsulfonyle en (C₁-C₄), alkylcarbonyle en (C₁-C₄), alcoxycarbonyle en (C₁-C₄), hydroxycarbonyle, -(C=O)NR⁷R⁸, alcoxy en (C₁-C₄), phénoxy, phényle, pyridyle, pyrimidyle, hétéroaryle à 5 chaînons, cycloalkyle en (C₃-C₇), morpholinyle, pipéridinyle, pyrrolidinyle, pipérazinyle, 1,1-dioxydothiomorpholin-4-yle et azétidine,
l'hétéroaryle à 5 chaînons pouvant être substitué avec 1 à 3 substituants choisis dans le groupe constitué par halogène, alkyle en (C₁-C₄) et alcoxy en (C₁-C₄),
R⁷ et R⁸ représentant chacun indépendamment l'un de l'autre hydrogène, alkyle en (C₁-C₄) ou cycloalkyle en (C₃-C₇),
le pipéridinyle pouvant être substitué avec 1 à 4 substituants fluor,
le phényle pouvant être substitué avec 1 à 3 substituants choisis dans le groupe constitué par halogène, alkyle en (C₁-C₄) et alcoxy en (C₁-C₄),
l'azétidine pouvant être substituée avec hydroxy,
l'amino pouvant être substitué avec 1 ou 2 substituants alkyle en (C₁-C₄),
et
le pipérazinyle pouvant être substitué avec 1 à 3 substituants choisis indépendamment les uns des autres dans le groupe constitué par alkyle en (C₁-C₄), cycloalkyle en (C₃-C₇) et trifluorométhyle,
pour former un composé de formule (I-A2) 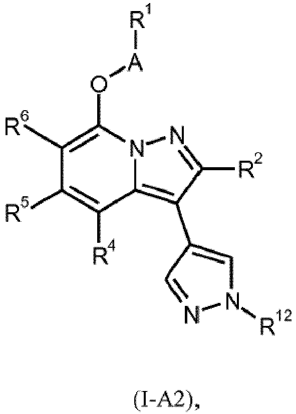 dans laquelle A, R¹, R², R⁴, R⁵, R⁶ et R¹² ont chacun les significations indiquées précédemment, puis des groupes protecteurs éventuellement présents sont clivés, et les composés de formule (I-A) résultants sont éventuellement transformés avec (i) les solvants et/ou (ii) les acides ou les bases appropriés en leurs solvates, sels et/ou solvates des sels,
ou
[B] un composé de formule (VIII) 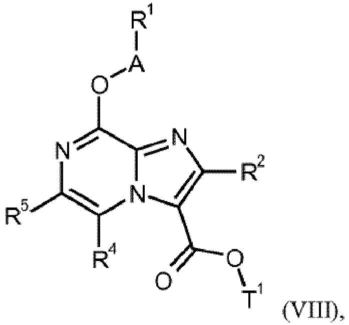
dans laquelle A, R¹, R², R⁴ et R⁵ ont chacun les significations indiquées précédemment, et
T¹ représente alkyle en (C₁-C₄) ou benzyle,
est mis en réaction dans un solvant inerte en présence d'une base ou d'un acide approprié pour former un acide carboxylique de formule (IX) 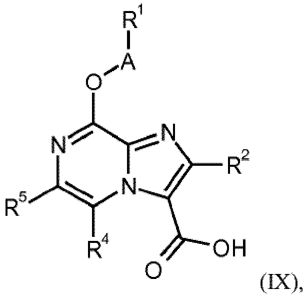 dans laquelle A, R¹, R², R⁴ et R⁵ ont chacun les significations indiquées précédemment,
puis celui-ci est transformé avec un équivalent d'halogène en présence d'une base en un composé de formule (X) 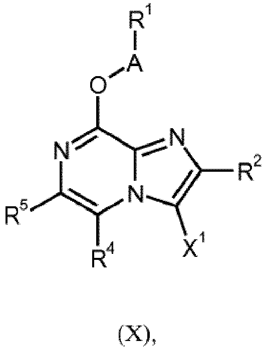
dans laquelle A, R¹, R², R⁴ et R⁵ ont chacun les significations indiquées précédemment, et
X¹ représente chlore, brome ou iode,
puis celui-ci est mis en réaction dans un solvant inerte en présence d'un catalyseur de métal de transition approprié avec un composé de formule (V) 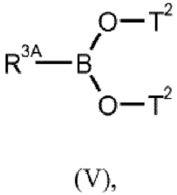 dans laquelle
R^{3A} a les significations indiquées précédemment pour R³ et
T² représente hydrogène ou alkyle en (C₁-C₄), ou les deux radicaux T² forment ensemble un pont -C(CH₃)₂-C(CH₃)₂-,
des groupes protecteurs éventuellement présents sont clivés, et les composés de formule (I-B) résultants sont éventuellement transformés avec (i) les solvants et/ou (ii) les acides ou les bases appropriés en leurs solvates, sels et/ou solvates des sels.

9. Composé de formule (I-A) ou (I-B), tel que défini dans l'une quelconque des revendications 1 à 7, pour le traitement et/ou la prophylaxie de maladies.

10. Utilisation d'un composé de formule (I-A) ou (I-B), tel que défini dans l'une quelconque des revendications 1 à 7, pour la fabrication d'un médicament pour le traitement et/ou la prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques et de l'artériosclérose.

11. Médicament contenant un composé de formule (I-A) ou (I-B), tel que défini dans l'une quelconque des revendications 1 à 7, en combinaison avec un adjuvant inerte, non toxique, pharmaceutiquement approprié.

12. Médicament contenant un composé de formule (I-A) ou (I-B), tel que défini dans l'une quelconque des revendications 1 à 7, en combinaison avec un autre agent actif choisi dans le groupe constitué par les nitrates organiques, les donneurs de NO, les inhibiteurs de cGMP-PDE, les agents antithrombotiques, les agents abaissant la tension artérielle et les agents modifiant le métabolisme lipidique.

13. Médicament selon la revendication 11 ou 12 pour le traitement et/ou la prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques et de l'artériosclérose.
